# EUROPÄISCHE PATENTSCHRIFT

(11) **EP 0 877 019 B1**
(45) Veröffentlichungstag und Bekanntmachung des Hinweises auf die Patenterteilung: **12.12.2001**
(21) Anmeldenummer: 98108040.1
(22) Anmeldetag: 02.05.1998
(51) Int. Cl.: C07C 311/19, C07C 311/29, C07D 261/18, C07D 209/48, C07D 209/46, C07D 241/24, C07D 213/81, C07D 307/24, C07D 233/54, C07D 235/24, C07D 513/04, C07D 233/74, C07D 207/16, C07D 277/06, C07D 209/42, C07D 307/68, C07D 333/38, C07D 333/24, C07D 275/04, C07D 249/18, C07D 215/48, C07D 295/20, C07D 207/34, C07D 207/38, C07D 239/42, A61K 31/18, A61K 31/63

(54) **Substituierte Diaminocarbonsäuren**
Substituted diaminocarboxylic acid
Acides diaminocarboxyliques substitués

(30) Priorität: 09.05.1997 DE 19719585; 12.05.1997 DE 19719428
(43) Veröffentlichungstag der Anmeldung: 11.11.1998
(73) Patentinhaber: HOECHST AKTIENGESELLSCHAFT, 65929 Frankfurt am Main (DE)
(72) Erfinder: Thorwart, Werner, Dr., 65239 Hochheim (DE); Schwab, Wilfried, Dr., 65193 Wiesbaden (DE); Schudok, Manfred, Dr., 65817 Eppstein/Ts. (DE); Haase, Burkhard, Dr., 65719 Hofheim (DE); Neises, Bernhard, Dr., 77652 Offenburg (DE); Billen, Günter, Dr., 65527 Niedernhausen (DE)

(56) Entgegenhaltungen:
- EP-A- 0 606 046
- EP-A- 0 757 037
- WO-A-95/35276
- WO-A-96/00214
- WO-A-96/27583
- WO-A-97/19068
- R.P. Beckett et al., DDT, Vol.1, No.1, 1996, pp.16-26
- J. Med. Chem., 1998, 41, 640-649

## Beschreibung

Die Erfindung betrifft neue substituierte Diaminocarbonsäuren, Verfahren zu ihrer Herstellung und Verwendung derselben als Arzneimittel.

In den Anmeldungen EP 0 606 046, WO 95/35276 und WO 96/27583 werden Arylsulfonaminohydroxamsäuren und deren Wirkung als Matrix-Metallproteinase-Inhibitoren beschrieben. Spezielle Arylsulfonaminocarbonsäuren, dienen als Zwischenprodukte zur Darstellung von Thrombin-Inhibitoren (EP 0 468 231) und Aldose-Reduktase-Inhibitoren (EP 0 305 947). In der Anmeldung EP 0 757 037 wird auch die Wirkung von Sulfonylaminocarbonsäure-Derivate als Metalloproteinase-Inhibitoren beschrieben.

Ferner hat sich die Arylsulfonylgruppe als eine effektive Schutzgruppe der Aminofunktion von α-Aminocarbonsäuren bewährt (R. Roemmele, H. Rapoport, J. Org. Chem. 53 (1988) 2367-2371).

In dem Bestreben wirksame Verbindungen zur Behandlung von Bindegewebserkrankungen zu finden, wurde nun gefunden, daß die erfindungsgemäßen Diaminocarbonsäuren starke Inhibitoren der Matrix-Metalloproteinasen sind. Dabei wird auf die Hemmung von Stromelysin (Matrix Metalloproteinase 3) und der Neutrophilen Kollagenase (MMP-8) besonderer Wert gelegt, da beide Enzyme insbesondere beim Abbau der Proteoglykane, als wichtige Bestandteile des Knorpelgewebes, maßgeblich beteiligt sind (A. J. Fosang et al. J. Clin. Invest. 98 (1996) 2292-2299).

Die Erfindung betrifft daher die Verbindung der Formel I 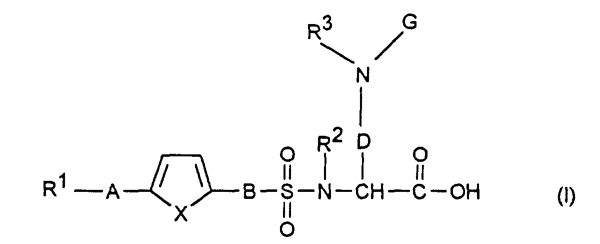 und/oder eine stereoisomere Form der Verbindung der Formel I und/oder ein physiologisch verträgliches Salz der Verbindung der Formel I, wobei
R¹ für
   1. Phenyl,
   2. Phenyl, welches ein- oder zweifach substituiert ist durch
      2.1. (C₁-C₇)-Alkyl, gerade, cyclisch oder verzweigt,
      2.2. -OH,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃,
      2.8. -CN,
      2.9. -NO₂,
      2.10. HO-C(O)-,
      2.11. (C₁-C₆)-Alkyl-O-C(O)-,
      2.12. Methylendioxo,
      2.13. R⁴-(R⁵)N-C(O)-,
      2.14. R⁴-(R⁵)N-, oder
   3. einen Heteroaromaten aus der nachfolgenden Gruppe 3.1. bis 3.16., der unsubstituiert oder wie unter 2.1 bis 2.14 beschrieben substituiert ist,
      3.1. Pyrrol,
      3.2. Pyrazol,
      3.3. Imidazol,
      3.4. Triazol,
      3.5. Thiophen,
      3.6. Thiazol,
      3.7. Oxazol,
      3.8. Isoxazol,
      3.9. Pyridin,
      3.10. Pyrimidin,
      3.11. Indol,
      3.12 Benzothiophen,
      3.13. Benzimidazol,
      3.14. Benzoxazol,
      3.15. Benzothiazol oder
      3.16 Benzotriazol steht,
R², R⁴ und R⁵ gleich oder verschieden sind und für
   1. Wasserstoffatom,
   2. (C₁-C₆)-Alkyl-,
   3. HO-C(O)-(C₁-C₆)-Alkyl-,
   4. Phenyl-(CH₂)ₒ-, worin Phenyl unsubstituiert oder ein- oder zweifach wie unter 2.1. bis 2.14. beschrieben substituiert ist und o die ganze Zahl Null, 1 oder 2 darstellt, oder
   5. Picolyl stehen oder
   6. R⁴ und R⁵ zusammen mit der ringständigen Aminogruppe einen 4- bis 7-gliedrigen Ring bilden, worin gegebenenfalls eines der Kohlenstoffatome durch -O-, -S- oder -NH- ersetzt ist,
R³ und G gleich oder verschieden sind und für
   1. Wasserstoffatom,
   2. (C₁ -C₆)-Alkyl-, worin Alkyl gerade, verzweigt oder cyclisch ist,
   3. (C₂-C₆)-Alkenyl-,
   4. Phenyl-(CH₂)ₘ-, worin Phenyl unsubstituiert oder ein- oder zweifach wie unter 2.1. bis 2.14. beschrieben substituiert ist und m die ganze Zahl Null, 1, 2 oder 3 ist,
   5. Heteroaryl-(CH₂)ₘ-, worin Heteroaryl wie unter 3.1. bis 3.16. definiert und/oder wie unter 2.1 bis 2.14 beschrieben substituiert ist und m die ganze Zahl Null, 1, 2 oder 3 darstellt,
   6. R⁶-C(O)-, worin
      R⁶ für
      6.1 (C₁-C₆)-Alkyl-, worin Alkyl unsubstituiert oder wie unter 2.1. bis 2.14. beschrieben oder durch (C₃-C₆)-Cycloalkyl substituiert ist,
      6.2 (C₃-C₆)-Cycloalkyl, worin Cycloalkyl unsubstituiert oder wie unter 2.1. bis 2.14. beschrieben substituiert ist,
      6.3 (C₂-C₆)-Alkenyl-, worin Alkenyl unsubstituiert oder ein- bis dreifach substituiert ist durch
         6.3.1 Phenyl, worin Phenyl unsubstiituiert oder ein- bis dreifach wie unter 2.1. bis 2.14. beschrieben substituiert ist
         6.3.2 Heteroaryl -, worin Heteroaryl wie unter 3.1. bis 3.16. definiert und unsubstituiuert oder ein- bis dreifach wie unter 2.1. bis 2.14. beschrieben substituiert ist oder
         6.3.3 die unter 2.1. bis 2.14. beschriebenen Reste,
      6.4 Phenyl-(CH₂)ₘ-, worin Phenyl unsubstituiert oder ein- bis dreifach wie unter 2.1. bis 2.14. beschrieben, durch -O-CF₃, -SO₂-NH₂, -NH-C(O)-CF₃ oder durch Benzyl substituiert ist und gegebenenfalls ein Wasserstoffatom des -(CH₂)-Restes durch den Rest -COOH substituiert ist und m die ganze Zahl Null, 1, 2 oder 3 bedeutet,
      6.5 Naphthyl,
      6.6 Adamantyl oder
      6.7 Heteroaryl-(CH₂)ₘ-, worin Heteroaryl wie unter 3.1. bis 3.16. definiert und/oder wie unter 2.1. bis 2.14. beschrieben substituiert ist und m die ganze Zahl Null, 1, 2 oder 3 darstellt, steht,
   7. R⁶-O-C(O)-, worin R⁶ wie oben genannt definiert ist,
   8. R⁶-CH(NH₂)-C(O)-, worin R⁶ wie oben genannt definiert ist,
   9. R⁸-N(R⁷)-C(O)-, worin
      R⁸ für
      9.1 Wasserstoffatom
      9.2 (C₁-C₆)-Alkyl-,
      9.3 Phenyl-(CH₂)ₘ, worin Phenyl unsubstituiert oder ein-oder zweifach wie unter 2.1. bis 2.14. beschrieben substituiert ist und m die ganze Zahl Null, 1,2 oder 3 darstellt, oder
      9.4 Heteroaryl-(CH₂)ₘ, worin Heteroaryl wie unter 3.1. bis 3.16. definiert und/oder wie unter 2.1 bis 2.14 beschrieben substituiert ist und m die ganze Zahl Null, 1, 2 oder 3 darstellt, steht und worin R⁷ Wasserstoffatom oder (C₁-C₆)-Alkyl bedeutet oder worin R⁷ und R⁸ zusammen mit dem Stickstoffatom an das sie gebunden sind einen 4- bis 7-gliedrigen Ring bilden und der Ring unsubstituiert ist oder ein Kohlenstoffatom im Ring durch -O-, -S- oder -NH- ersetzt ist,
   10. R⁶-SO₂-, worin R⁶ wie oben genannt definiert ist,
   11. R⁶-SO₂-N(R⁷)-C(O)-, worin R⁶ und R⁷ wie oben genannt definiert sind,
   12. R⁶-NH-C(=NR⁷)-, worin R⁶ und R⁷ wie oben genannt definiert sind oder
      12.1 (C₁-C₆)-Alkyl-C(O)-,
      12.2 -NO₂ oder
      12.3 -SO₂-(CH₂)ₘ-Phenyl, worin Phenyl unsubstituiert oder ein- oder zweifach wie unter 2.1. bis 2.14. beschrieben substituiert ist und m die ganze Zahl Null, 1,2 oder 3 ist, darstellen,
   13. 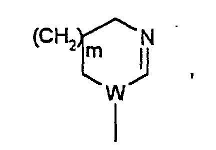
   worin m die ganze Zahl Null, 1, 2 oder 3 bedeutet und W für ein Stickstoff-, Sauerstoff- oder Schwefelatom steht, oder
R³ und G zusammen mit dem Stickstoffatom an das sie gebunden sind einen Ring der Teilformel IIa bis IIp
   bilden, 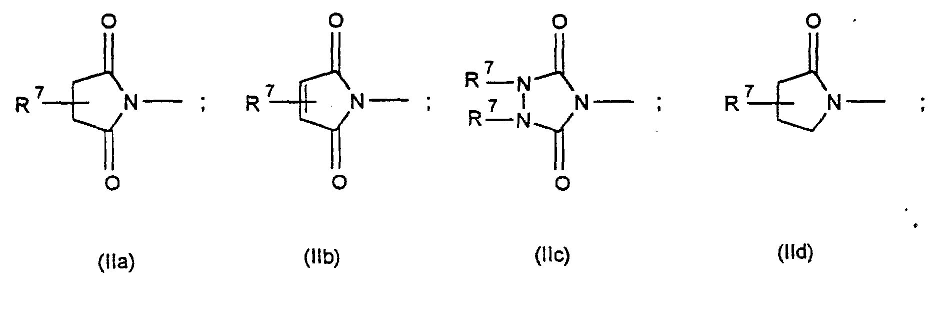 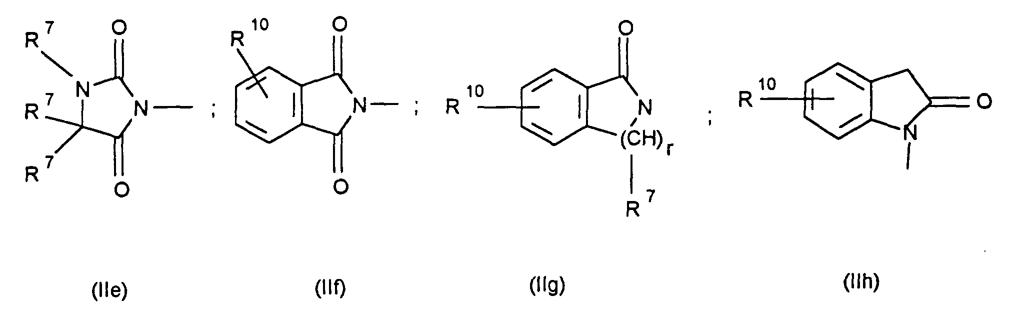 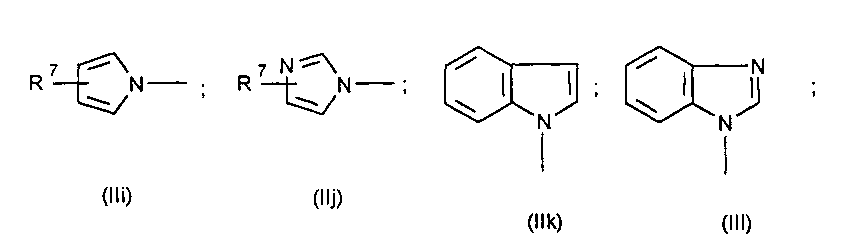 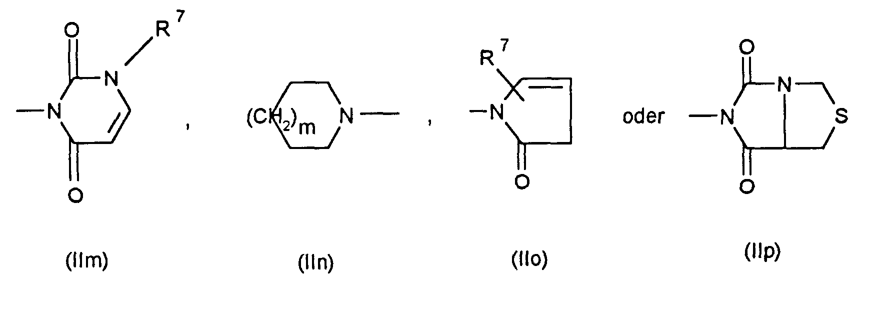 wobei r die ganze Zahl 1 oder 2 bedeutet, R¹⁰ einen Rest wie unter 2.1. bis 2.14. beschrieben bedeutet, und R⁷ und m die oben genannte Bedeutung haben und gegebenenfalls bei der Teilformel IIg ein Kohlenstoffatom im Ring durch Sauerstoff-, Schwefel-, SO₂ oder unsubstituiertes oder mit R² substituiertes Stickstoffatom ersetzt ist,
A für
   a) eine kovalente Bindung,
   b) -O-,
   c) -CH=CH- oder
   d) -C≡C- steht,
B für
   a) -(CH₂)ₘ-, worin m die obengenannte Bedeutung hat,
   b) -O-(CH₂)_{q}, worin q die ganze Zahl 1, 2, 3, 4 oder 5 bedeutet, oder
   c) -CH=CH- steht,
D - (CH₂)ₛ- bedeutet, worin s für die ganze Zahl 1, 2, 3, 4, 5 oder 6 steht und gegebenenfalls einer der Ketten-C-Atome durch ein gegebenenfalls substituiertes -NH-, -O- oder -S- Atom ersetzt ist, und
X für -CH=CH-, Sauerstoffatom oder Schwefelatom steht.

Bevorzugt ist eine Verbindung der Formel I, wobei
R¹ für
   1. Phenyl oder
   2. Phenyl, welches einfach substituiert ist durch
      1. Halogen, insbesondere Chlor oder Fluor oder
      2. R⁴-(R⁵)N-, wobei R⁴ und R⁵ gleich oder verschieden sind und für
         2.1. (C₁-C₃)-Alkyl stehen oder
         2.2. R⁴ und R⁵ zusammen mit der ringständigen Aminogruppe einen 5-6-gliedrigen Ring bilden, wobei gegebenenfalls eines der C-Atome durch -O- oder -NH- ersetzt ist, steht,
R² für Wasserstoffatom steht,
G und R³ verschieden sind, wobei
   G für Wasserstoffatom oder (C₁-C₄)-Alkyl steht und
      R³ für
         1. Phenyl-(CH₂)ₘ steht, worin Phenyl unsubstituiert ist oder ein- oder zweifach wie unter 2.1 bis 2.14 beschrieben substituiert ist und m die ganze Zahl 1 ist,
         2. für Heteroaryl-(CH₂)ₙ steht, worin Heteroaryl wie unter 3.10 definiert ist und unsubstituiert ist oder wie unter 2.1 bis 2.14 beschrieben substituiert ist und n für Null steht,
         3. für R⁶-C(O)- steht, worin
            R⁶ für
            3.1 (C₁ -C₆)-Alkyl-, worin Alkyl gerade, verzweigt oder cyclisch ist,
            3.2 Phenyl-(CH₂)ᵣ- worin Phenyl unsubstituiert oder ein- oder zweifach wie unter 2.1 bis 2.14 beschrieben substituiert ist und gegebenenfalls ein Wasserstoffatom des -(CH₂)-Restes durch den Rest -COOH ersetzt ist und r Null, 1, 2 oder 3 bedeutet, oder
            3.3 Heteroaryl-(CH₂)ₒ-, worin Heteroaryl wie unter 3.1 bis 3.15 definiert und unsubstituiert oder wie unter 2.1 bis 2.14 beschrieben substituiert ist und o Null 1, 2, oder 3 bedeutet, oder
         4. für R⁸-N(R⁷)-C(O)- steht, worin
            R⁸ und R⁷ zusammen mit dem Stickstoffatom an das sie gebunden sind einen 5- oder 6-gliedrigen Ring bilden und der Ring unsubstituiert oder ein Ring-C-atom durch ein Sauerstoffatom ersetzt ist, oder
R³ und G zusammen mit dem Stickstoffatom, an das sie gebunden sind, einen Ring der Teilformel IIg bilden, worin r 1 ist,
A für eine kovalente Bindung steht,
B für -(CH₂)ₚ- steht und p Null bedeutet,
D -(CH₂)_{q}- bedeutet und q für eine ganze Zahl 2, 3 oder 4 steht, und
X für -CH=CH- steht.

Mit dem Begriff "R⁴ und R⁵ zusammen mit der ringständigen Aminogruppe einen 4- bis 7-gliedrigen Ring bilden und/oder eines der Kohlenstoffatome durch -O-, -S- oder -NH- ersetzt ist" werden Reste verstanden, die sich beispielsweise von Pyrrolidin, Piperazin, Morpholin, Piperidin oder Thiomorpholin ableiten. Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffketten geradkettig oder verzweigt sind. Cyclische Alkylreste sind beispielsweise 3- bis 6-gliedrige Monocyclen wie Cyclopropyl, Cyclobutyl, Cyclopentyl oder Cyclohexyl. Ferner können die Alkenylreste auch mehrere Doppelbindungen enthalten. Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) eine Diaminocarbonsäure der Formel III 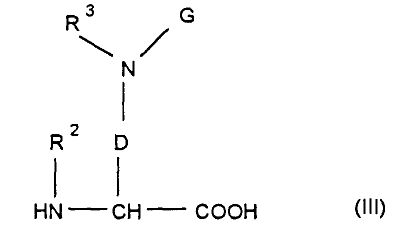 worin R², R³, D und G wie in Formel I definiert sind, mit einem Sulfonsäurederivat der Formel IV 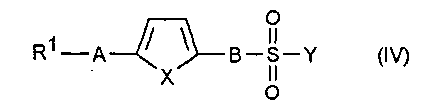 worin R¹, A und B wie in Formel I definiert sind und Y ein Halogenatom, Imidazolyl oder -OR⁹ bedeutet, worin R⁹ Wasserstoffatom, (C₁-C₆)-Alkyl, Phenyl, Succinimidyl, Benzotriazolyl oder Benzyl, gegebenenfalls substituiert darstellt, in Gegenwart einer Base oder gegebenenfalls eines wasserentziehenden Mittels zu einer Verbindung der Formel I umsetzt, oder
b) einen Diaminocarbonsäureester der Formel V 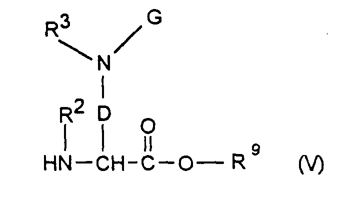 worin R², R³, D, G und R⁹ die obengenannte Bedeutung haben, mit einem Sulfonsäurederivat der Formel IV unter den obengenannten Bedingungen zu einer Verbindung der Formel VI 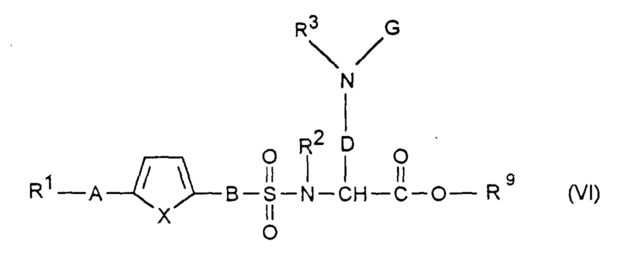 umsetzt, und die Verbindung der Formel VI unter Abspaltung des Restes R⁹, bevorzugt in Gegenwart einer Base oder Säure in eine Verbindung der Formel I umwandelt,
c) die geschützten Diaminocarbonsäuren der Formel VII, 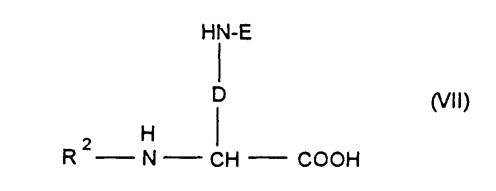 worin R² und D die oben genannte Bedeutungen haben und E für eine Schutzgruppe der Aminofunktion steht, mit einem Sulfonsäurederivat der Formel IV zu einer Verbindung der Formel VIII 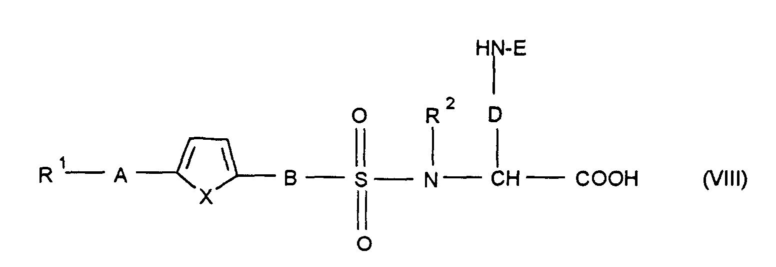 umsetzt, anschließend die Verbindung der Formel VIII unter Abspaltung der Schutzgruppe E mit Hilfe geeigneter Spaltreagenzien in eine Verbindung der Formel I überführt, 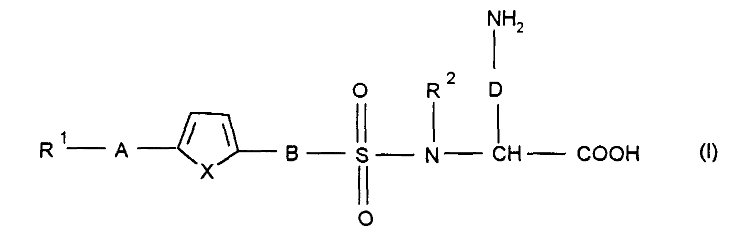 worin R¹, R², A, B, D und X die oben genannte Bedeutung haben und R³ und G ein Wasserstoffatom bedeuten, und diese Verbindung der Formel I gegebenenfalls mit Hilfe von R³-Y, in dem R³ und Y die oben angegebenen Bedeutungen haben, in Gegenwart einer Base zu einer Verbindung der Formel I, 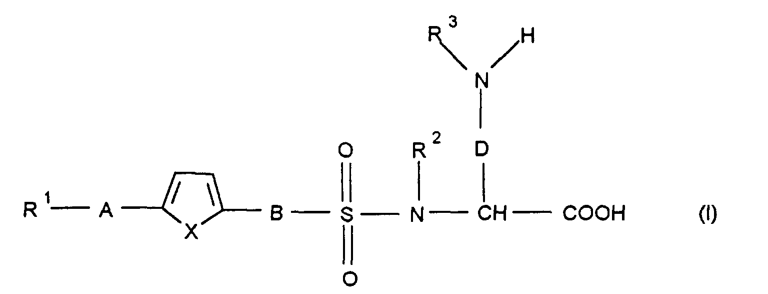 worin R¹, R², R³, A, B und X die oben genannte Bedeutungen haben und G ein Wasserstoffatom darstellt, umsetzt, oder
d) für den Fall, daß als Ausgangsverbindungen geschützte Diaminosäureester der Formel IX, 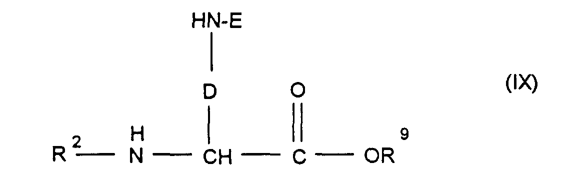 worin R², R⁹, D und E die oben genannte Bedeutung haben, in gleicher Weise wie in der Verfahrensvariante c) beschrieben in die Ester der Formel X umwandelt, 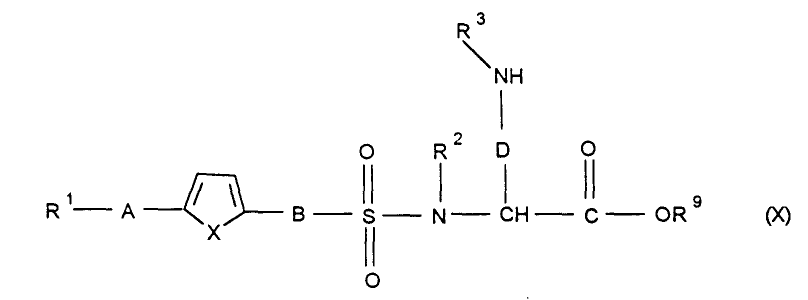 die gegebenenfalls nach Verfahrensvariante b) in die entsprechenden Verbindungen der Formel I umgewandelt werden, oder
e) eine Diaminocarbonsäure der Formel XI, 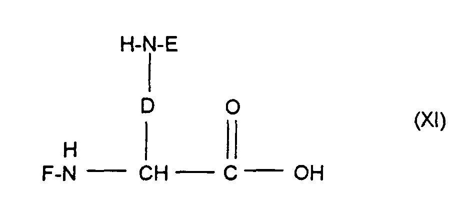
worin D wie in Formel I definiert ist und E und F untereinander verschiedene N-Aminoschutzgruppen darstellen, mit ihrer Carboxylgruppe über eine Zwischenkette L an ein polymeres Harz der allgemeinen Formel PS ankoppelt, wobei eine Verbindung der Formel XII 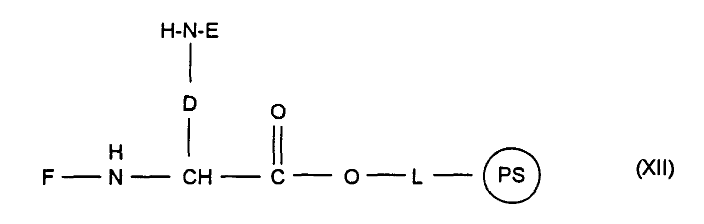 entsteht, die sich nach selektiver Abspaltung der Schutzgruppe F mit einem Sulfonsäurederivat der Formel IV 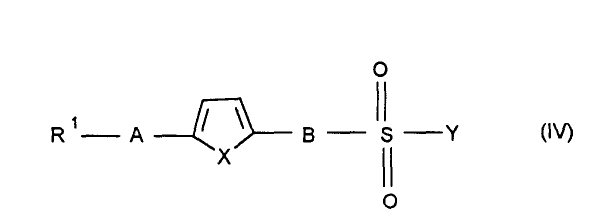 wobei R¹, A, B und Y die oben genannten Bedeutungen haben, in Gegenwart einer Base oder gegebenenfalls eines wasserentziehenden Mittels zu einer Verbindung der Formel XIII 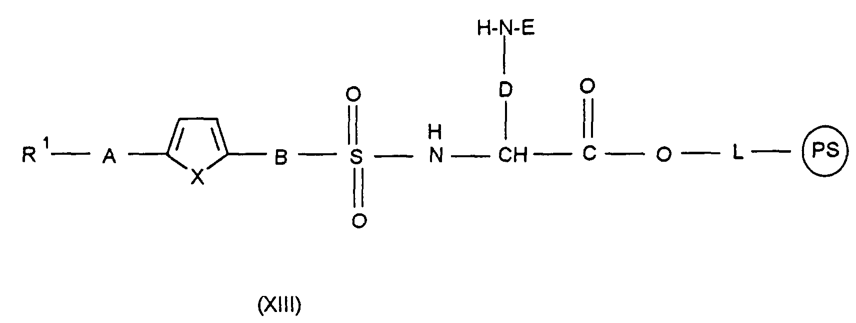 umsetzt, und die Verbindung der Formel XIII nach Abspaltung der Schutzgruppe E mit einem Carbonsäurederivat der Forme XIV

R⁶-C(O)-Y (XIV)

worin R⁶ und Y die oben genannte Bedeutung haben, in Gegenwart einer Base oder eines wasserentziehenden Mittels zu einer Verbindung der Formel XV 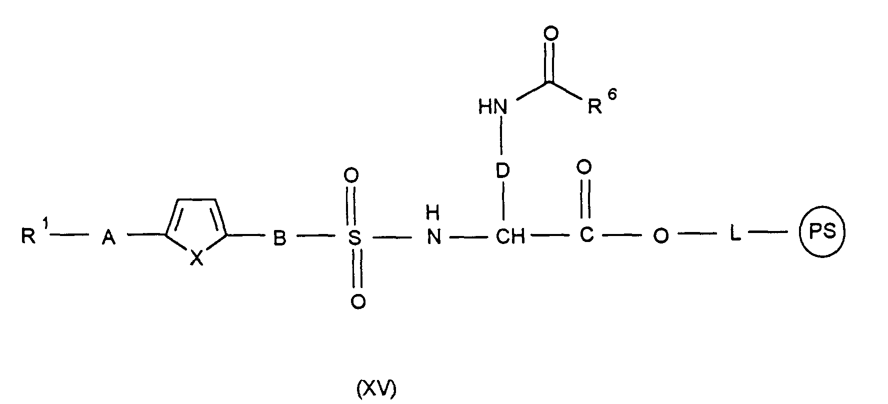 umsetzt, und diese nach Abspaltung vom Trägermaterial in eine Verbindung der Formel I, 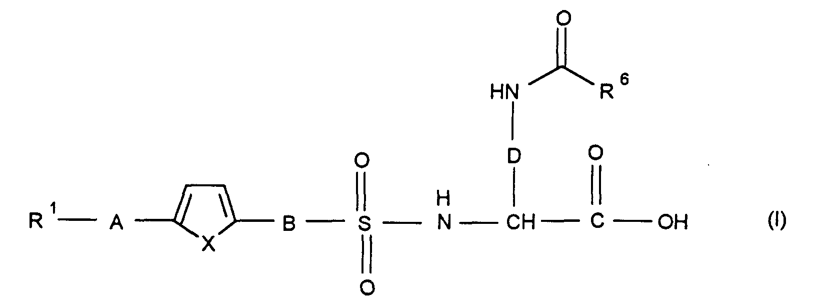 worin R¹, R⁶, A, B, D und X die oben genannte Bedeutung haben, überführt.

Als Ausgangsverbindungen der Formel III, bei denen R², R³ und G ein Wasserstoffatom bedeuten, werden vorzugsweise 2,3-Diaminopropionsäure, 2,4-Diaminobuttersäure, Omithin, Lysin und Homolysin eingesetzt. Stellen R³ und G zusammen mit der Aminofunktion einen Guanidylgruppe dar, wird vorzugsweise Arginin verwendet. Werden, wie in der Verfahrensvariante c), d) und e) die Aminofunktionen der Ausgangsverbindungen der Formeln VII, IX und XI mit einer Schutzgruppe E oder F versehen, erfolgt diese selektive Aminogruppen-Derivatisierung nach Methoden wie sie in Houben-Weyl "Methoden der Org. Chemie", Band 15/1 beschrieben sind.

Als geeignete Schutzgruppen E und F werden dafür vorzugsweise die in der Peptidchemie gebräuchlichen N-Schutzgruppen verwendet, beispielsweise Schutzgruppen vom Urethan-Typ, wie Benzyloxycarbonyl(Z), t-Butyloxycarbonyl (Boc), 9-Fluorenylmethoxycarbonyl (Fmoc) und Allyloxycarbonyl (Aloc) oder von Säureamid-Typ insbesondere Formyl, Acetyl oder Trifluoracetyl oder vom Alkyl-Typ wie Benzyl. Besonders geeignet hat sich dafür auch die (Trimethyl-silyl)ethoxycarbonyl (Teoc)Gruppe (P. Kociénski, Protecting Groups, Thieme Verlag 1994). Viele der selektiv derivatisierten Verbindungen sind auch käuflich, so daß die Darstellung der erfindungsgemäßen Verbindungen der Formel I, wie in der Verfahrensvariante c) beschrieben ist, darin besteht, daß nach der Einführung der Sulfonsäureesters in die α-Aminogruppe, die Abspaltung der Seitenketteschutzgruppe E erfolgt, an die sich gegebenenfalls eine mehrstufige Derivatisierung der freien Aminogruppe in der Seitenkette anschließen kann. Während dieser Vorgehensweise kann die Carboxylgruppe in freier Form oder in Form eines Esters mit dem Rest -OR⁹ vorliegen. Für den Fall, das es sich bei dem Rest -OR⁹ um einen geradkettigen (C₁-C₃)-Alkyl-Rest handelt, lassen sich dies Ester der allgemeinen Formel I auch in dieser Form in die Therapie einsetzen (Prodrug).
Für den Fall, daß R⁹ ein tert. Butylrest darstellt, erfolgt die Esterspaltung bevorzugt auf der letzten Synthesestufe nach bekannten Methoden mit HCI in Diethylether oder Trifluoressigsäure.

Als Ausgangsprodukte zur Darstellung der Sulfonsäurederivate der Formel IV dienen bevorzugt Sulfonsäuren oder deren Salze der Formel XVIa - XVIg, beispielsweise 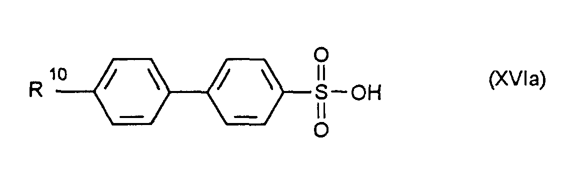 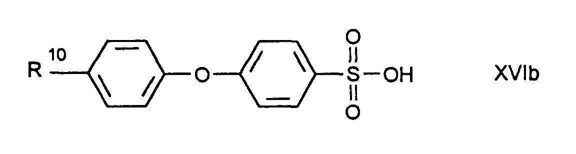 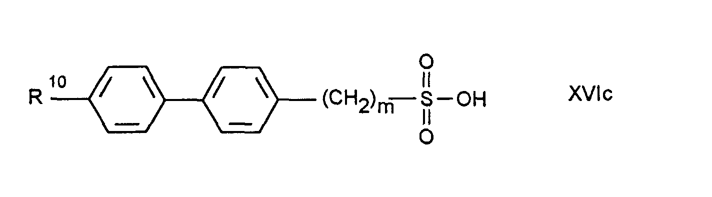 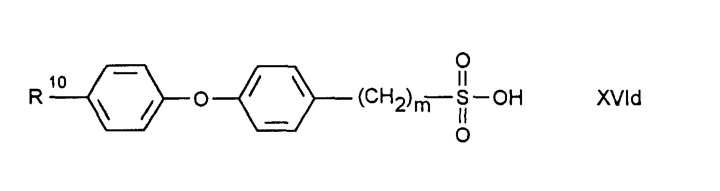 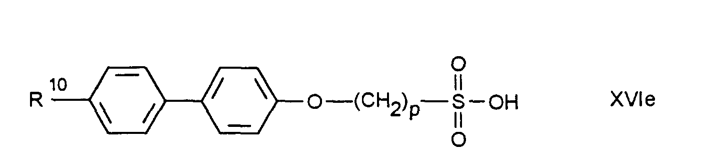 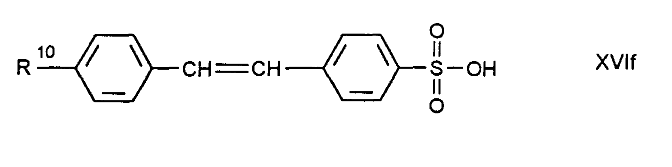 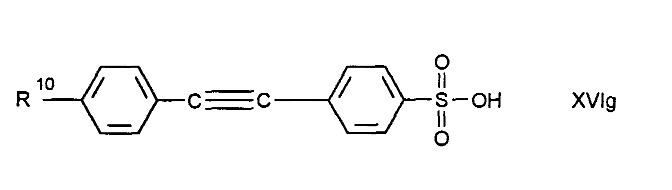 wobei R¹⁰ ein unter Phenyl 2.1. bis 2.14. beschriebener Rest bedeutet.

Zur Herstellung der Arylsulfonsäuren der Formeln XVIa und b bedient man sich vorzugsweise der im Houben/Weyl "Methoden der Organischen Chemie" Band 9, S. 450-546 beschriebenen Sulfonierungsverfahren mit konzentrierter Schwefelsäure ggf. in Gegenwart eines Katalysators, Schwefeltrioxids und seinen Additionsverbindungen oder Halogensulfonsäuren, wie Chlorsulfonsäure. Besonders im Falle der Diphenylether der Formel XVIb hat sich die Verwendung von konzentrierte Schwefelsäure und Essigsäureanhydrid als Lösemittel (vergl. C.M. Suter, J. Am. Chem. Soc. 53 (1931) 1114), oder die Umsetzung mit überschüssiger Chlorsulfonsäure (J.P. Bassin, R. Cremlyn und F. Swinbourne; Phosphorus, Sulfur and Silicon 72 (1992) 157) bewährt. Sulfonsäuren gemäß der Formeln XVIc, XVId, oder XVIe lassen sich in an sich bekannter Weise herstellen, in dem man das entsprechende Arylalkylhalogenid mit Sulfiten wie Natriumsulfit oder Ammoniumsulfit in wäßriger oder wäßrig/alkoholischer Lösung umsetzt, wobei die Umsetzung in Gegenwart von Tetraorganoammoniumsalzen wie Tetrabutylammoniumchlorid beschleunigt werden kann.

Als Sulfonsäurederivate gemäß Formel IV finden insbesondere die Sulfonsäurechloride Verwendung. Zu ihrer Herstellung werden die entsprechenden Sulfonsäuren, auch in Form ihrer Salze wie Natrium-, Ammonium- oder Pyridiniumsalze in bekannter Weise mit Phosphorpentachlorid oder Thionylchlorid ohne oder in Gegenwart eines Lösemittels wie Phosphoroxytrichlorid oder eines inerten Lösemittels wie Methylenchlorid, Cyclohexan oder Chloroform im allgemeinen bei Reaktionstemperaturen von 20°C bis zum Siedepunkt des verwendeten Reaktionsmediums umgesetzt.
Die Umsetzung der Sulfonsäurederivate der Formel IV mit den Aminosäuren der Formeln III, V, VII oder IX gemäß Verfahrensvarianten a), b), c) oder d) verläuft vorteilhaft nach Art der Schotten-Baumann-Reaktion. Als Base eignen sich dafür besonders Alkalihydroxide wie Natriumhydroxid, aber auch Alkaliacetate, -hydrogencarbonate, -carbonate und Amine. Die Umsetzung findet in Wasser oder in einem mit Wasser mischbaren oder nichtmischbaren Lösemittel wie Tetrahydrofuran (THF), Aceton, Dioxan oder Acetonitril statt, wobei die Reaktionstemperatur im allgemeinen von -10°C bis 50°C gehalten wird. Für den Fall, daß die Reaktion im wasserfreien Medium durchgeführt wird, findet vor allem Tetrahydrofuran oder Methylenchlorid, Acetonitril oder Dioxan in Gegenwart einer Base, wie Triethylamin, N-Methylmorpholin, N-Ethyl- oder Diisopropylethylamin Verwendung, eventuell in Gegenwart von N,N-Dimethylaminopyridin als Katalysator.

In einer anderen Variante kann man die Aminocarbonsäuren der Formel III, V, VII oder IX zuerst mit Hilfe eines Silylierungsmittels wie Bis-trimethylsilyltrifluoracetamid (BSTFA) in ihre silylierte Form überführen und sie dann mit Sulfonsäurederivaten zu Verbindungen der Formel I umsetzen.

Der in der Formel XII mit PS bezeichnete polymere Träger ist ein quervernetztes Polystyrolharz mit einem als Zwischenkette L bezeichneten Linker, bekannt als Wang-Harz (S.W. Wang, Journal of the American Chemical Society (1973), 1328 p-Benzyloxybenzyl-Alkohol-Polystyrol Harz). Alternativ können andere polymere Träger wie Glas, Baumwolle oder Cellulose mit verschiedenen Zwischenketten L eingesetzt werden.
Die mit L bezeichnete Zwischenkette ist kovalent an den polymeren Träger gebunden und erlaubt eine reversible, esterartige Bindung mit der Diaminosäure der Formel XI die während der weiteren Umsetzung an der gebundene Diaminocarbonsäure stabil gebunden bleibt; jedoch unter stark sauren Reaktionsbedingungen, z.B. reine Trifluoressigsäure, die am Linker befindliche Gruppe wieder freisetzt.
Die Freisetzung der gewünschten Verbindung der allgemeinen Formel I vom Linker kann an verschiedenen Stellen in der Reaktionsfolge geschehen.
1.) Für den Fall von daß eine Verbindung der allgemeinen Formel I, in der R³ und G Wasserstoff bedeuten, wird das α-Sulfonylamino-ω-carbonsäurederivat, nach Abspaltung der Schutzgruppe E, durch Behandlung des Harzes mit Trifluoressigsäure freigesetzt.
2.) Soll eine Verbindung der allgemeinen Formel I mit R³ gleich Wasserstoffatom und G gleich R⁶-C(O)- erhalten werden, so wird die Freisetzung der Verbindung von Harz nach einfacher Acylierung mit R⁶-C(O)-Y, wie in 1) vorgenommen.
3.) Für den Fall von daß eine Verbindung der allgemeinen Formel I in der R³ und G gleich R⁶-C(O)- sind, wird die Abspaltung erst nach ausgiebiger Diacylierung unter Zuhilfenahme eines Acylierungskatalysators, z.B. Dimethylaminopyridin, wie in 1) vorgenommen.
4) Weiterhin läßt diese Verfahrensweise zu, daß die in Formel I definierten Reste 2 bis 13 für R³ und G unter Verwendung geeigneter Reagenzien, z.B. Alkylhalogeniden, Alkenylhalogeniden, Chloroformiate, Isocyanate, Sulfonsäure-Derivaten oder cyclischen Anhydriden, an dieser Stelle der Reaktionsfolge an die an den festen Träger gebundenen α-Sulfonyl-amido-ω-aminocarbonsäure gekoppelt werden können. Nach Abspaltung der erhaltenen Verbindungen vom festen Träger werden somit auch beispielsweise die entsprechenden substituierten Amine, Urethane, Harnstoffe, Sulfonamide oder Amide erhalten.

### A. Allgemeine Vorgehensweise zur Kopplung von geschützten Diaminocarbonsäuren der Formel XI an den festen Träger nach Verfahrensweise e):

2 g Wang-Harz (Nova-Biochem; Beladung 0,5 mmol/g) werden in 20 ml trockenem Dichlormethan 30 min quellen gelassen (50 ml PET-Spritze mit Teflon Filter am Spritzenboden). Nach Filtrieren des Lösungsmittels wird die Spritze mit einer Lösung von 3,5 mmol der entsprechenden ω-Teoc-α-Fmoc-diaminocarbonsäure (hergestellt nach: D.H. Rich et al., Synthesis 198; 346), 3,5 mmol Diisopropylcarbodiimid und 0,5 mmol N,N-Dimethylaminopyridin in etwa 10 ml trockenem Dichlormethan gefüllt und 16 Stunden (h) bei Raumtemperatur (RT) geschüttelt.

Nach Abfiltrieren der Reaktionsmischung wird das Harz mehrmals mit Dichlormethan gewaschen und zur Bestimmung der Ausbeute getrocknet und gewogen.

### B. Abspaltung der α-Fmoc-Schutzgruppe

Das wie in A. vorbereitete Harz wird in der Spritze in etwa 20 ml trockenem Dimethylformamid (DMF) quellen gelassen und anschließend, nach Abfiltrieren des Lösungsmittels, mit einer 25 %igen Piperidin/DMF-Lösung versetzt und 45 Minuten (min) bei RT geschüttelt. Die entstandene Mischung wird filtriert und das in der Spritze verbleibende Harz mehrmals mit trockenem DMF gewaschen. (Filtrat und alle Waschlösungen können zur Bestimmung der Fmoc-Abspaltung aufbewahrt werden; Durchführung siehe: Solid Phase Peptide Synthesis - a practical approach, E. Atherton and R.C. Sheppard, IRL Press at Oxford University Press 1989).

### C. Sulfonierung der freien α-Aminogruppe

Der Spritzeninhalt wird nun z.B. auf 4 kleinere, mit eingelegter Filterplatte versehene Spritzen gleichmäßig aufgeteilt und mit Lösungen von verschiedenen Sulfonsäurederivaten der Formel IV (jeweils 1 mmol) und Diisopropylethylamin (jeweils 1 mmol) in 3 ml trockenem DMF versetzt und 24 h bei RT geschüttelt. Anschließend wird die Reagenzienlösung ausgewaschen und das Harz mehrmals mit DMF gewaschen.

### D. Abspaltung der Teoc-Schutzgruppe

Das wie in C. vorbereitete Harz wird mit einer molaren N-Tetrabutylammoniumfluorid-Lösung in DMF (jeweils etwa 3 ml) versetzt und 2 Stunden bei RT geschüttelt. Die Reagenzlösungen werden filtriert und das verbleibende Harz mehrmals mit DMF gewaschen. Der Spritzeninhalt jeder der 4 Einzelspritzen wird nun z.B. auf je weitere 3 vorbereitete Spritzen aufgeteilt. (Jeweils 1x 0,05 mmol und 2x 0,1 mmol).

### E.

1: Abspaltung vom festen Träger
   Jeweils etwa 1/5 eines Spritzeninhalts wird zur Abspaltung der Substanz vom festen Träger mit Dichlormethan gewaschen (etwa 10 ml), getrocknet und mit etwa 1 ml einer Lösung von 95 % Trifluoressigsäure, 2 % H₂O und 3 % Triisopropylsilan 1 Stunde bei RT geschüttelt. Die aus der Spritze filtrierte Lösung wird evaporiert, und mit Diethylether ausgefällt. Der feste Rückstand wird zur weiteren Reinigung filtriert und getrocknet.
2: Acylierung mit Carbonsäurederivaten der Formel R⁶-C(O)-Y:
   Die übrigen Spritzen werden jeweils mit 1 molaren Lösungen von Acetanhydrid (1 Äquivalent bezogen auf freigesetzen Amin, oder 3 Äquivalente für Bis-Acylierungen) und entsprechender Menge Triethylamin in DMF gefüllt und 16 Stunden bei RT geschüttelt (Vollständigkeit der Acylierung kann z.B. durch Kaiser-Ninhydrin Test/Durchführung siehe: Solid Phase Peptide Synthesis - a practical approach, E. Atherton and R.C. Sheppard, JRL Press at Oxford University Press 1989, überprüft werden).
3: Abspaltung der Verbindungen der Formel XV vom festen Träger
   Die wie in 2: vorbereiteten Harze werden, wie in 1: beschrieben, mit Dichlormethan gewaschen, getrocknet und mit Trifluoressigsäure/H₂O/Triisopropylsilan 95/2/3 1 h bei RT behandelt. Die erhaltenen Lösungen werden, wie in 1: beschrieben, aufgearbeitet.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzolsulfon-, Cyclohexylamidosulfon-, Trifluormethylsulfon-, Essig-, Oxal-, Wein-, Bernstein-oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Metalloproteinasen beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 200-MHz-Gerät der Firma Varian aufgenommen worden, in der Regel mit Tetramethylsilan (TMS) als internem Standard und bei Raumtemperatur (RT). Die verwendeten Lösemittel sind jeweils angegeben. Endprodukte werden in der Regel durch massenspektroskopische Methoden (FAB-, ESI-MS) bestimmt. Temperaturangaben in Grad Celsius, RT bedeutet Raumtemperatur (22-26°C). Verwendete Abkürzungen sind entweder erläutert oder entsprechen den üblichen Konventionen.

### Beispiel 1 (R)- (4-Chlorbiphenylsulfonyl)-citrullin

### Hergestellt nach Verfahrensvariante a)

1,7 g (9,7 mmol) R-Citrullin werden in 19,4 ml 0,5 n NaOH gelöst und nach Zugabe von 40 ml THF langsam bei 0 °C mit weiteren 19,4 ml der Natronlauge und gleichzeitig mit 9,7 ml einer 1 molaren Lösung des 4-Chlorbiphenylsulfonsäurechlorids versetzt. Nach 16 Stunden (h) rühren bei Raumtemperatur wird die Reaktionsmischung am Rotationsverdampfer eingeengt und mit 20 ml Essigester versetzt. Beim Ansäuern mit 1 m HCI fällt ein weißer Niederschlag aus, der abgesaugt und getrocknet wird.
- Ausbeute:: 2,26 g (54 % d. Theorie)
- ¹H-NMR (DMSO-d₆):: 1,2-1,7 (2m, 4 H); 2,9 (dd, 2 H); 3,7 (dd, 1 H); 5,4 (s, 2 H); 5,9 (t, 1 H); 7,5-7,9 (2 d, s, 8 H); 8,2 (d, 1H)

### Beispiel 2 R-(4-Chlorbiphenylsulfonyl)- Lys(Boc)-OH

### Hergestellt nach Verfahrensvariante c)

Die Umsetzung von 5,15g (21mmol) H-D-Lys(Boc)-OH zu (4-Chlorbiphenylsulfonyl)-R-Lys(Boc)-OH erfolgt wie in Beispiel 1 beschrieben; die Aufarbeitung erfolgt jedoch durch Extraktion mit Essigester und Abdampfen des Lösemittels unter verminderten Druck.
- Ausbeute:: 9,3 g (89% der Theorie)
- ¹H-NMR (DMSO-d₆):: 1,1-1,7 (m,15H),2,8(dd,2H), 3,7 (m,1H), 6,7 (t,1H), 7,6; 7,8 (2d, 4H), 7,9 (m,4H), 8,2 (d,1H)

### Beispiel 3 R-(4-Chlorbiphenylsulfonyl)- Lys-OH

4,97g (10mmol) der Verbindung aus Beispiel 2 werden 30 min bei RT mit 15 ml 50%-iger TFA in Methylenchlorid behandelt. Eindampfen unter verminderten Druck ergibt die gewünschte Verbindung.
- Ausbeute:: 3,73 g (94 % der Theorie)
- ¹H-NMR (DMSO-d₆):: 1,1-1,7 (m, 6H),2,8 (dd,2H), 3,7 (m,1H), 6,6 (m,2H), 7,6; 7,8 (2d, 4H), 7,9 (m,4H), 8,2 (d,1H)

### Beispiel 4 4-Chlorbiphenylsulfonyl-N-epsilon- (5-methylisoxazol-4-carbonyl)- Lys-OH

0,15 g (0,345 mmol) des (4-Chlorbiphenylsulfonyl)-lysins aus Beispiel 3 werden mit 50,1 mg (0,345 mmol) 5-Methylisoxazol-4-carbonsäurechlorid und 86,9 mg (1,035 mmol) NaHCO₃ in 5 ml Acetonitril für 6 h bei RT gerührt. Anschließend wird das Lösemittel unter verminderten Druck abdestilliert, der Rückstand in Essigester aufgenommen und mehrfach salzsauer sowie neutral ausgeschüttelt. Nach dem Trocknen der organischen Phase und abfiltrieren des Trockenmittels wird unter verminderten Druck eingedampft.
- Ausbeute:: 0,11 g (63 % der Theorie)
- ¹H-NMR (DMSO-d₆):: 1,1-1,7 (mm, 7 H); 2,6 (2 s, 3 H); 2,8; 3,1(2 m, 2 H); 3,7 (m, 1 H); 7,6; 7,8 (2 d, 4 H); 7,9 (m, 5 H); 8, 2 (d, 1 H); 8,8 (2 s, 1H)

### Beispiel 5 (4-Chlorbiphenylsulfonyl)-N-delta-(phenylsulfonylamino-carbonyl)- Orn-OH

### Hergestellt nach Verfahrensvariante d)

### 5 a. Umsetzung von H-Orn(Z)-OtBu zu 4-Chlorbiphenylsulfonyl-Orn(Z)-OtBu:

11,27 g (31,4 mmol) H-Orn(Z)-OtBu-Hydrochlorid werden mit 9,02 g (31,4 mmol) 4-Chlorbiphenylsulfonsäurechlorid und 10,7 ml (61,8 mmol) Diisopropylethylamin bei 0 °C in 200 ml THF umgesetzt. Nach 4 h wird der Ansatz unter verminderten Druck eingedampft und der Rückstand nach Aufnehmen in Essigester salzsauer, neutral und basisch (Natriumcarbonatlösung) ausgeschüttelt. Nach dem Trocknen der organischen Phase erhält man nach dem Eindampfen bis zur Trockne das gewünschte Produkt.
- Ausbeute:: 16,7 g (93 % der Theorie)
- ¹H-NMR (DMSO-d₆):: 1,5 (s, 9 H); 1,3-1,5 (m, 4 H); 2,9 (m, 2 H); 3,6 (m, 1 H); 5,0 (s, 2 H); 7,3 (m, 6 H); 7,5; 7,7 (2d, 4 H); 7,8 (s, 4 H); 8,2 (d, 1 H)

### 5 b. Abspaltung der Benzyloxycarbonyl-Schutzgruppe (Z)

16,7 g (29 mmol) vom Produkt aus 5a wird in Methanol-Essigester 1:1 gelöst und mit 4 g 10 % Pd/C unter leichtem Überdruck für 16 h hydriert. Anschließend wird vom Katalysator abfiltriert und der Rückstand unter verminderten Druck eingedampft.
- Ausbeute:: 11,2 g (91 % der Theorie)
- ¹H-NMR:: Die charakteristischen Signale der Schutzgruppe fehlen (5,0; 7,3).

### 5 c. Umsetzung von 5b zum Phenylsulfonylharnstoff-derivat:

0,5 g (1,14 mmol) der unter 5 b genannten Verbindung werden in Dimethylacetamid bei RT mit 0,23 ml Phenylsulfonylisocyanat umgesetzt. Nach 16 wird das Lösemittel entfernt und das aus Essigester ausfallende, kristalline Produkt mit Ether nachbehandelt. Diethyletherreste werden unter verminderten Druck entfernt.
- Ausbeute:: 0,53 g (75 % der Theorie)
- ¹H-NMR (DMSO-d₆):: 1,1 (s, 9 H); 1,3-1,5 (m, 4 H); 2,9 (m, 2 H); 3,6 (m, 1 H); 6,5 (t, 1 H); 7,4-7,9 (mm, 14 H); 8,2 (d, 1 H); 10,6 (s, 1 H)

### 5d. Abspaltung der Schutzgruppe von Beispiel 5c:

0,52 g des oben angeführten Produktes 5 c wird mit 5 ml TFA für 45 min bei RT gerührt. TFA wird unter verminderten Druck entfernt; zweimal wird mit Toluol coevaporiert, der Rückstand in Diethylether suspendiert und wie in Beispiel 5 als weißer kristalliner Feststoff abgetrennt.
- Ausbeute:: 0,4 g (84 % d. Theorie)
- ¹H-NMR (DMSO-d₆):: 1,3-1,5 (m, 4 H); 2,9 (m, 2 H); 3,6 (m, 1 H); 6,5 (t, 1 H); 7,4-7,9 (mm, 14 H); 8,2 (d, 1 H); 10,6 (s, 1 H)

### Beispiel 6 2-(2R)-(4-Chlorbiphenylsulfonylamino)-5-phthalimidoyl-pentansäure

0,7 g (1,67 mmol) 2-(2R)-(4-Chlorbiphenylsulfonylamino)-5-amino-pentansäure-Hydrochlorid (hergestellt nach Verfahrensvariante c) werden mit 0,358 g (2,42 mmol) Phthalsäureanhydrid für 1 Stunde auf 150 °C erhitzt. Nachdem die Gasentwicklung abgeklungen ist, wird das Reaktionsgemisch in Dichlormethan aufgenommen und über eine Kieselgelsäule chromatographiert (Eluent: Essigester/Petroiether/Eisessig 10/10/1).
- Ausbeute:: 29,6 mg (34,6 % der Theorie)
- Schmelzpunkt:: 178°C
- ¹H-NMR (DMSO-d₆):: 1,3 - 1,7 (m, 4H); 3,4 - 3,6 (t, 2H); 3,7 - 3,8 (m, 1H); 7,5 (d, 2H); 7,7 (d, 2H); 7,7 - 7,9 (m, 8H); 8,2 (d, 1H, NH); 12,6 (s, 1H, breit, OH)

### Beispiel 7 2-(2R)-(4 -Chlorbiphenylsulfonylamino)-5-(1-oxo-1,3-dihydro-isoindol-2-yl)-pentansäure

0,32 g (0,76 mmol) 2-(2R)-(4-Chlorbiphenylsulfonylamino)-5-amino-pentansäure-Hydrochlorid werden mit 0,186 g (1,35 mmol) Phthaldialdehyd in 30 ml Eisessig gelöst und 3 Stunden bei 100 °C gerührt. Die Lösung wird auf 0 °C abgekühlt, der ausfallende Niederschlag abgesaugt und über eine Kieselgelsäule chromatographiert (Eluent: Essigester/Petrolether/Eisessig 10/10/2).
- Ausbeute:: 185 mg (52 % der Theorie)
- Schmelzpunkt:: >234 °C (Zersetzung)
- ¹H-NMR (DMSO-d₆):: 1,4 - 1,7 (m, 4H); 3,1 (m, 1H); 3,4 - 3,6 (m, 2H); 4,4 (d, 1H); 4,5 (d, 1H); 6,9 (s, 1H, breit, OH); 7,4 - 7,9 (m, 13H)

### Beispiel 8 R-(4-Biphenylethylsulfonyl) Lys-OH

### Hergestellt nach Verfahrensvariante e)

α -Fmoc-ε -Teoc-D-Lys-OH (0,18 mmol) wird unter den oben genannten Bedingungen an 100 mg (0,05 mMol) Wang-Harz gekoppelt, und nach Abspaltung der a-Fmoc-Schutzgruppe mit 0,18 mmol 4-Biphenylethyl-sulfonylchlorid/ Diisopropylethylamin umgesetzt. Nach Abspaltung der ε-Teoc-Schutzgruppe mit 1- molarer Tetrabutylammoniumfluorid/DMF-Lösung und Abspaltung des erhaltenen Lysin-Derivates vom Harz (Trifluoressigsäure (TFA)/H₂O/Triisopropylsilan, 95/2/3) wird die erhaltene Lösung evaporiert. Der feste Rückstand wird mit Diethylether gewaschen, in einer 10% igen wäßrigen Essigsäure gelöst, bis zu Trockne lyophilisiert und liefert 20 mg der Titelverbindung in Form eines amorphes weißen Pulver.

HPLC (RP 18; UV 210 nm): Gradient 0-15 Min. B = 5-70% (A = 100% H₂O/0.1% Trifluoressigsäure; B = 100% Acetonitril/0,1% Trifluoressigsäure) T_{R} = 9,49 Min. (95%)

### Beispiel 9 R-(4-Biphenylethylsulfonyl)-N-epsilon-acetyl-Lys-OH

Wie in Beispiel 8 beschrieben werden 0,35 mMol α-Fmoc-epsilon-Teoc-D-Lysin an 200 mg (0,10 mMol) Wang-Harz gekoppelt, Fmoc entschützt und mit 4-Biphenlethylsulfonylchlorid/Diisopropylethylamin umgesetzt. Nach Abspaltung der ε-Teoc-Schutzgruppe wird das erhaltene Lysin-Derivat mit 0,15 mMol Acetanhydrid/ 0,15 mMol Diisopropylethylamin 15 Stunden bei Raumtemperatur gerührt. Nach ausgiebigem Waschen mit DMF; Dichlormethan und Trocknen des Harzes (0,1 Torr) über Nacht wird die gewünschte Verbindung mit Trifluoressigsäure/ H₂O/ Triisopropylsilan = 95/2/3 vom festen Träger abgespalten und wie in Beispiel 8 aufgearbeitet. Es werden 40 mg der Verbindung als amorphes weißes Pulver erhalten. HPLC (RP 18; UV 210 nm): Gradient 0-15 Min. B = 5-70% (A = 100% H₂O/0.1% Trifluoressigsäure; B = 100% Acetonitril/0.1% Trifluoressigsäure) T_{R} = 10,39 Min. (93%)

Die in der folgenden Tabelle 1 genannten Beispiele sind analog zu den vorherigen Beispielen hergestellt worden.

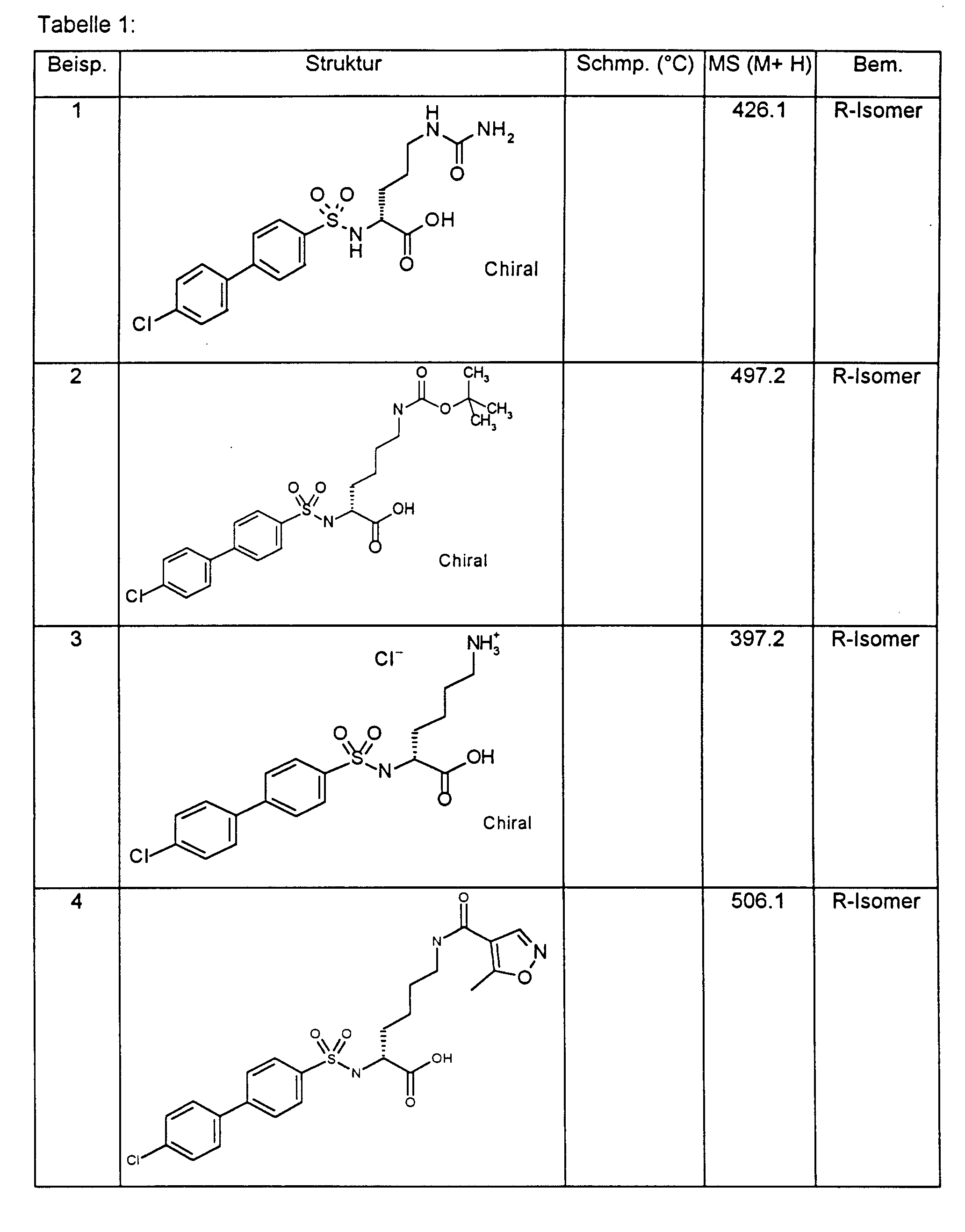

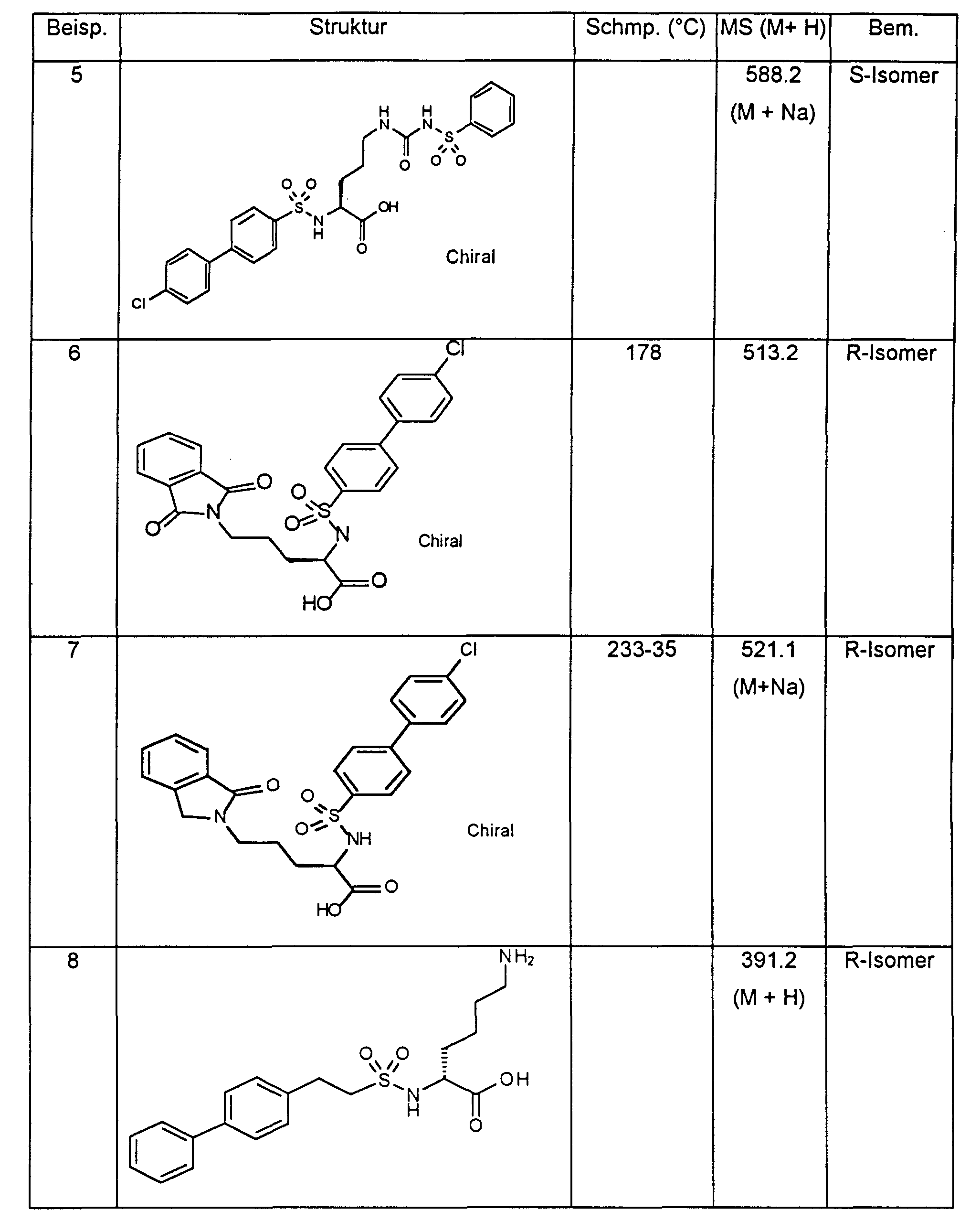

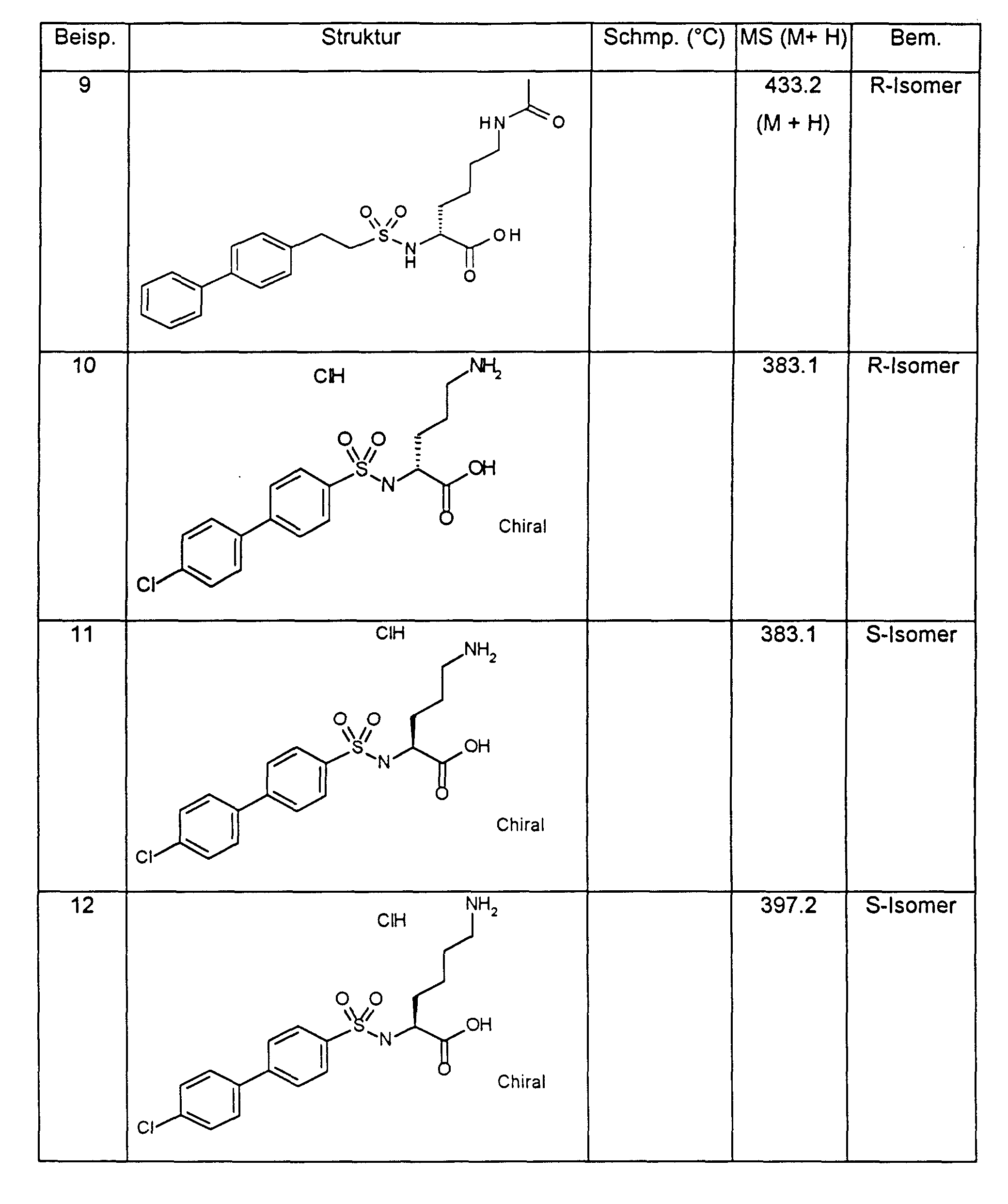

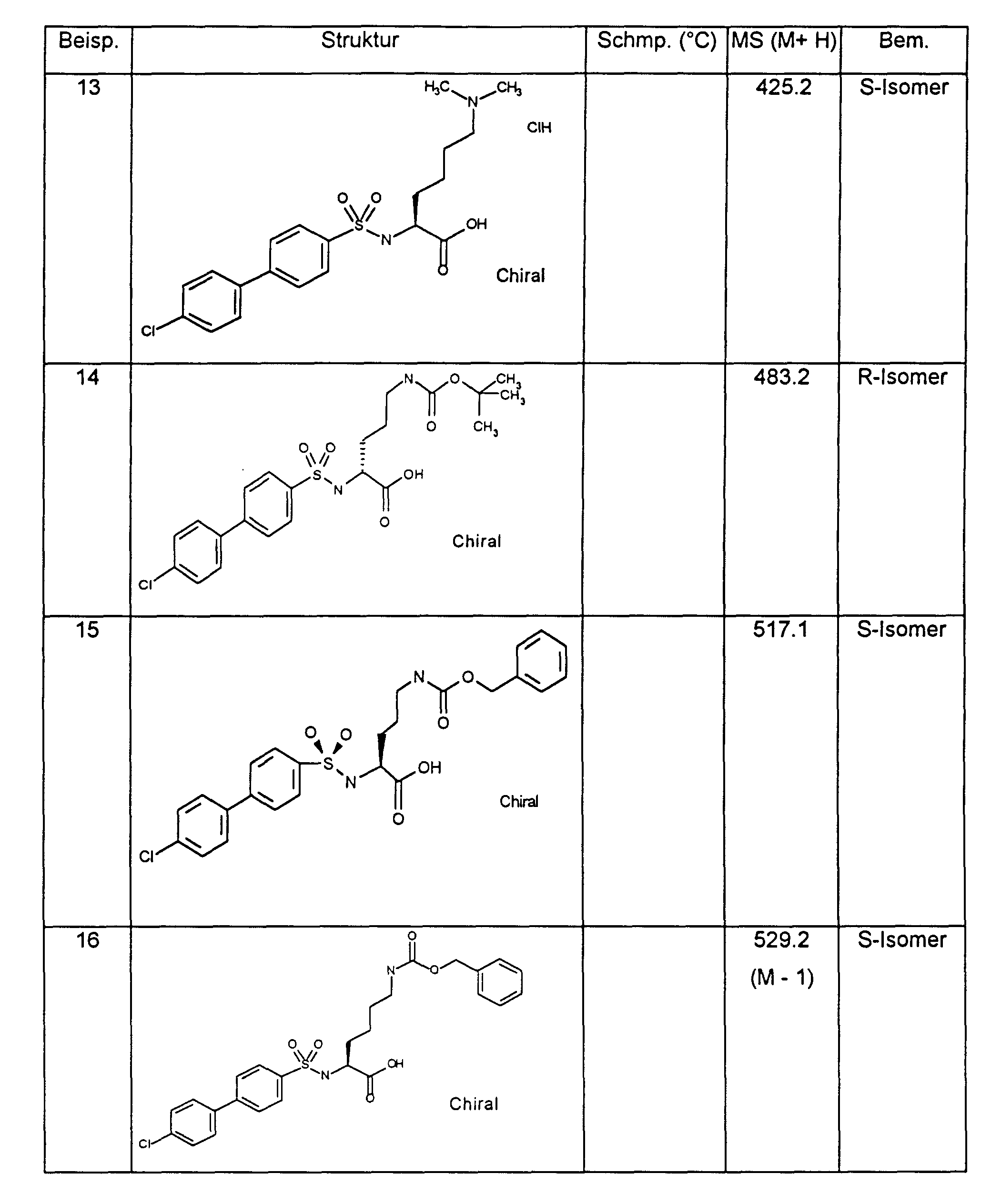

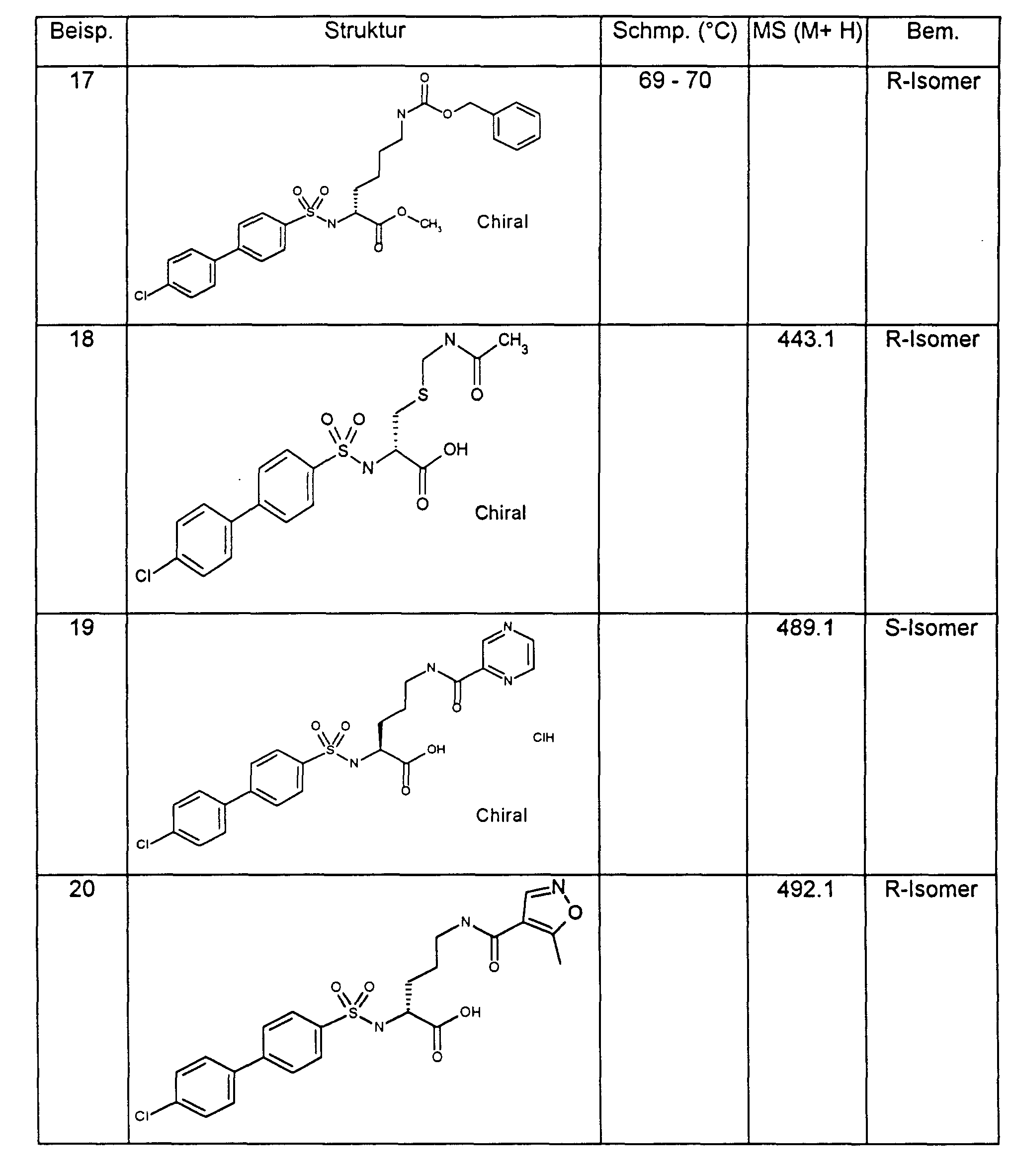

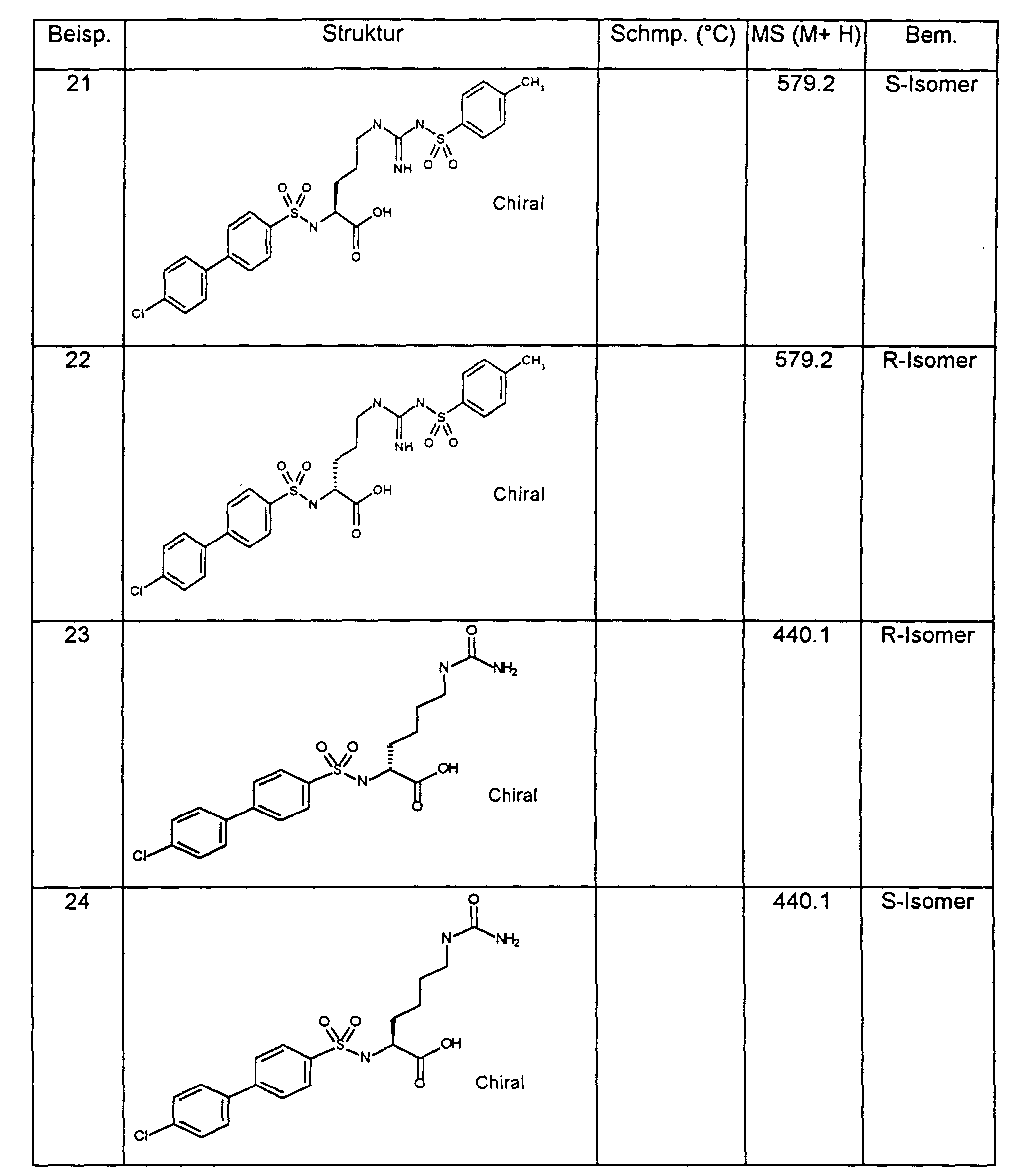

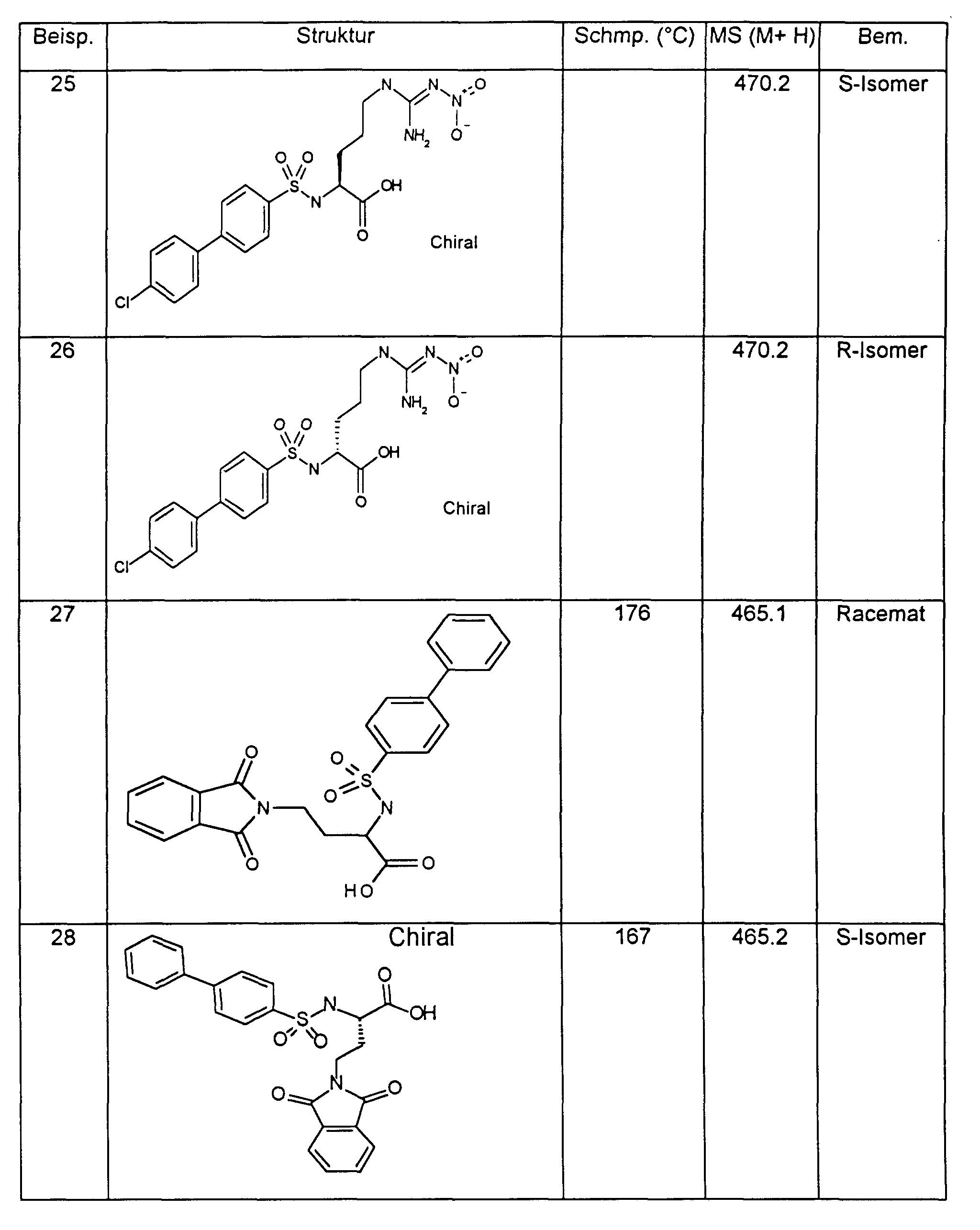

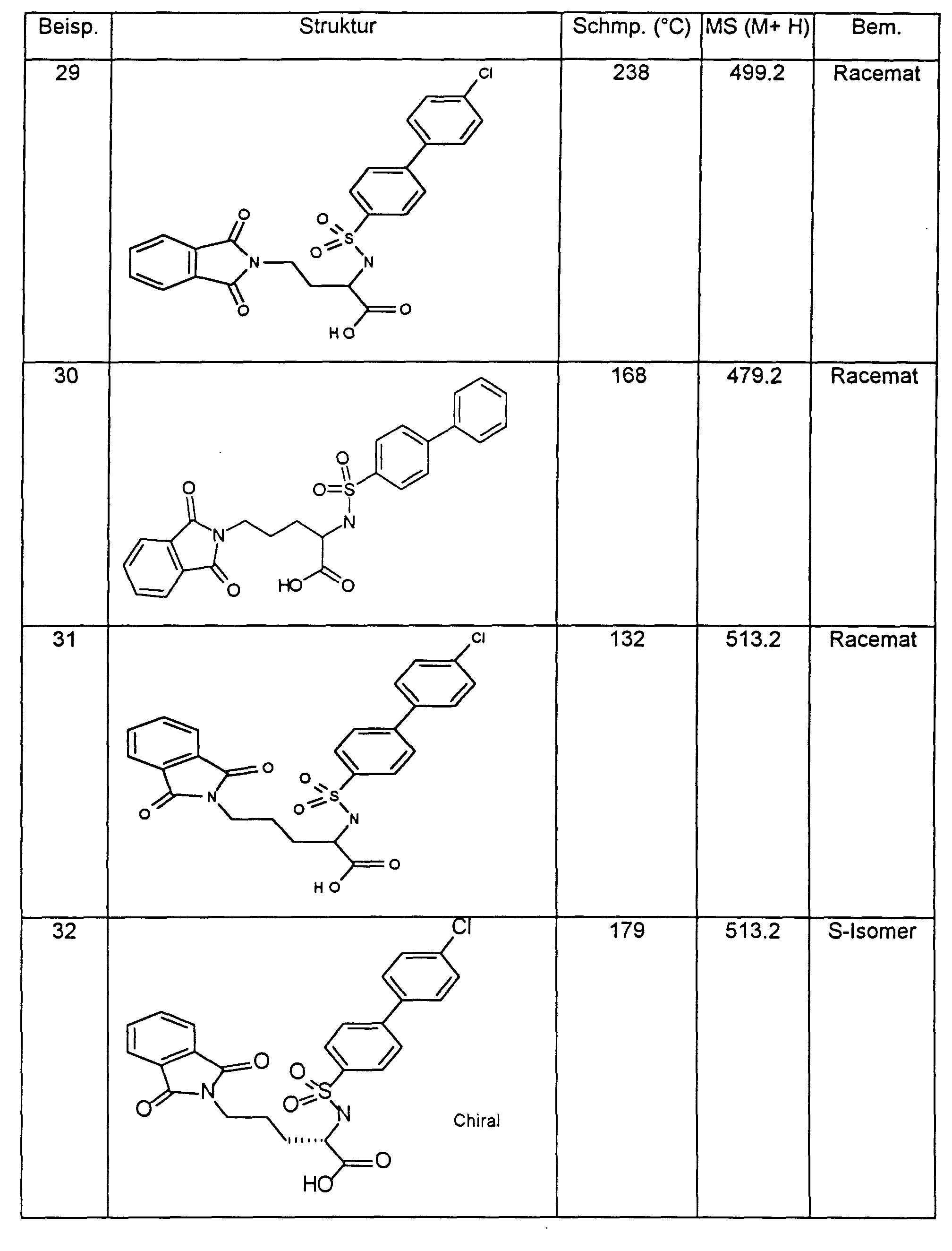

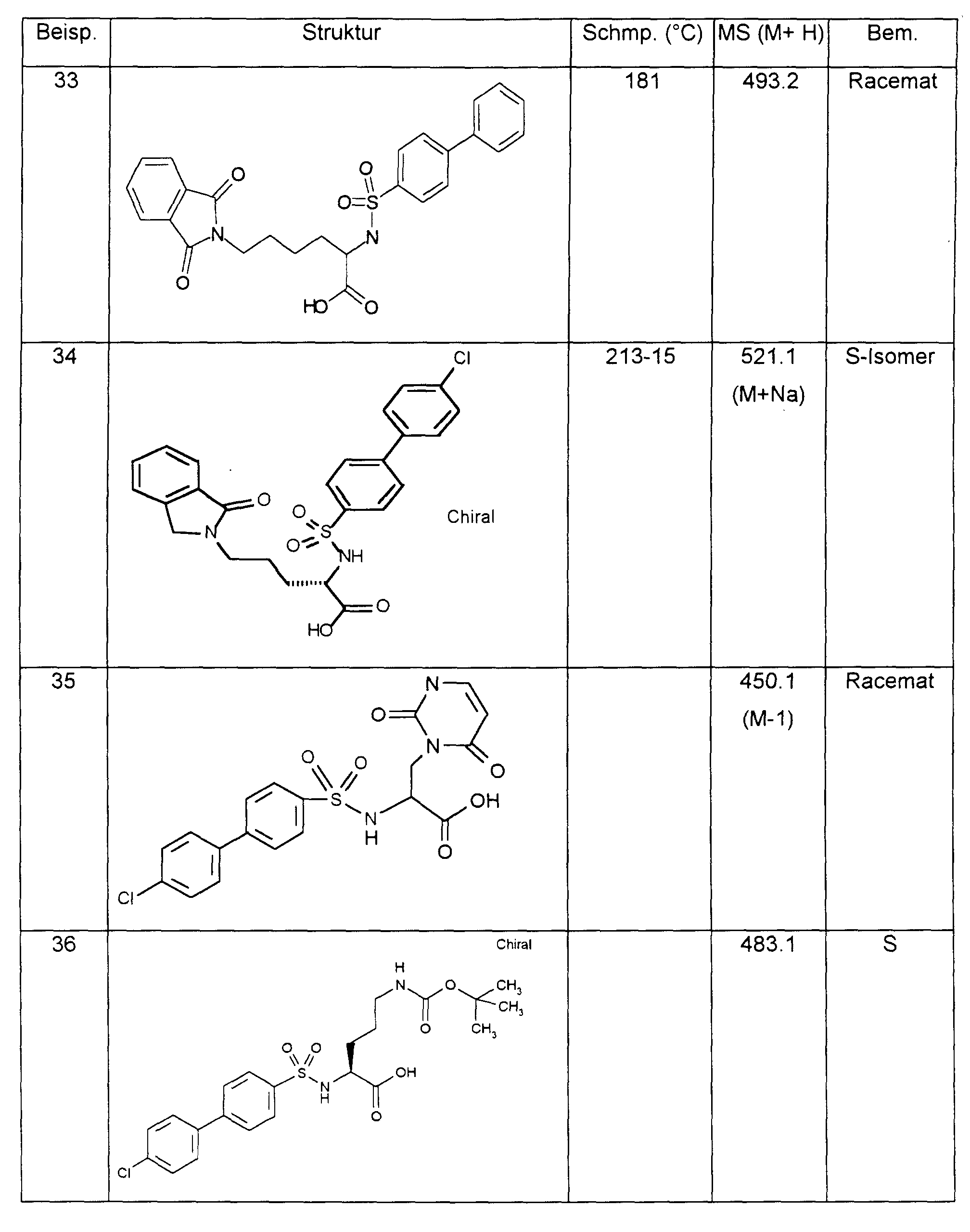

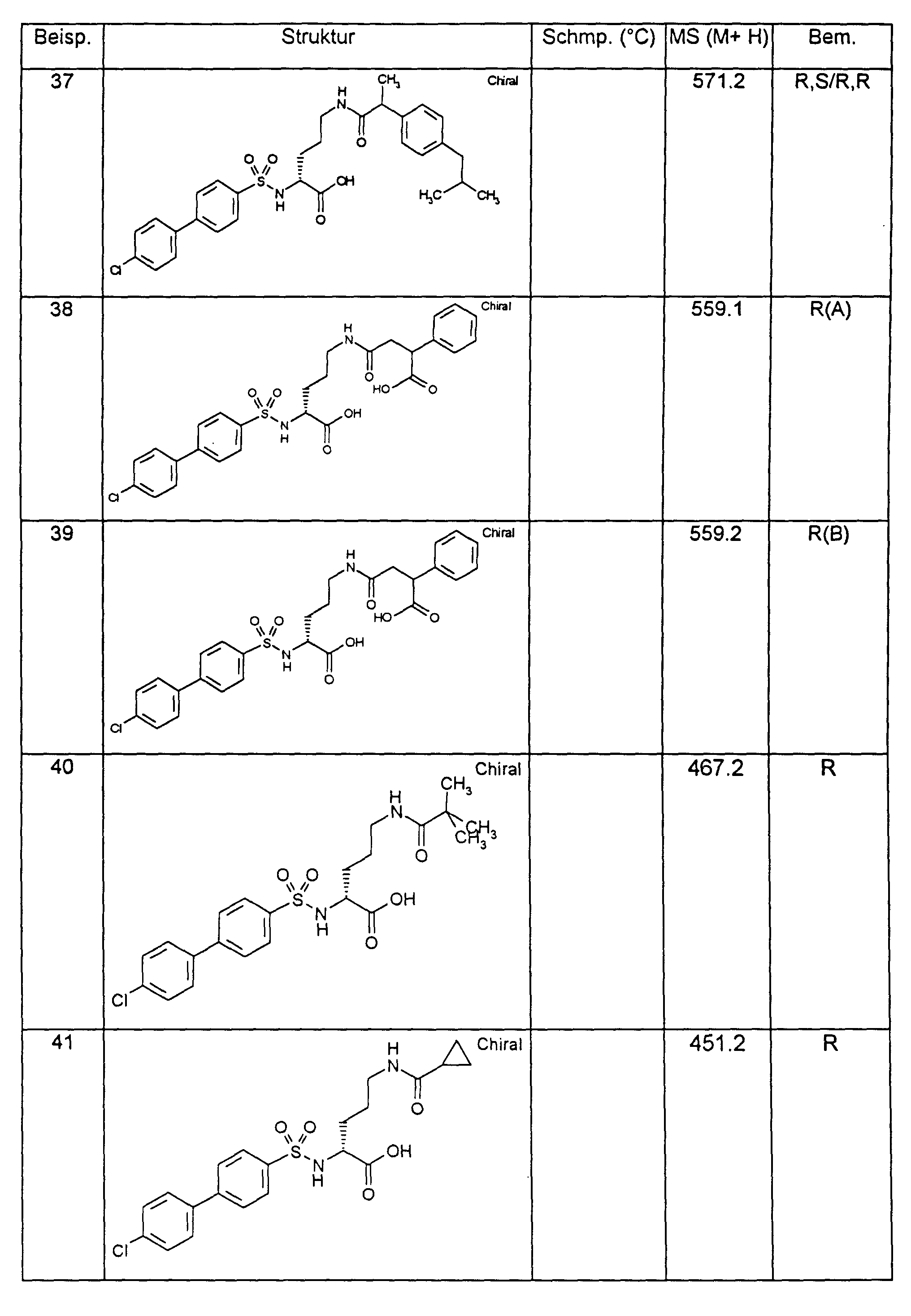

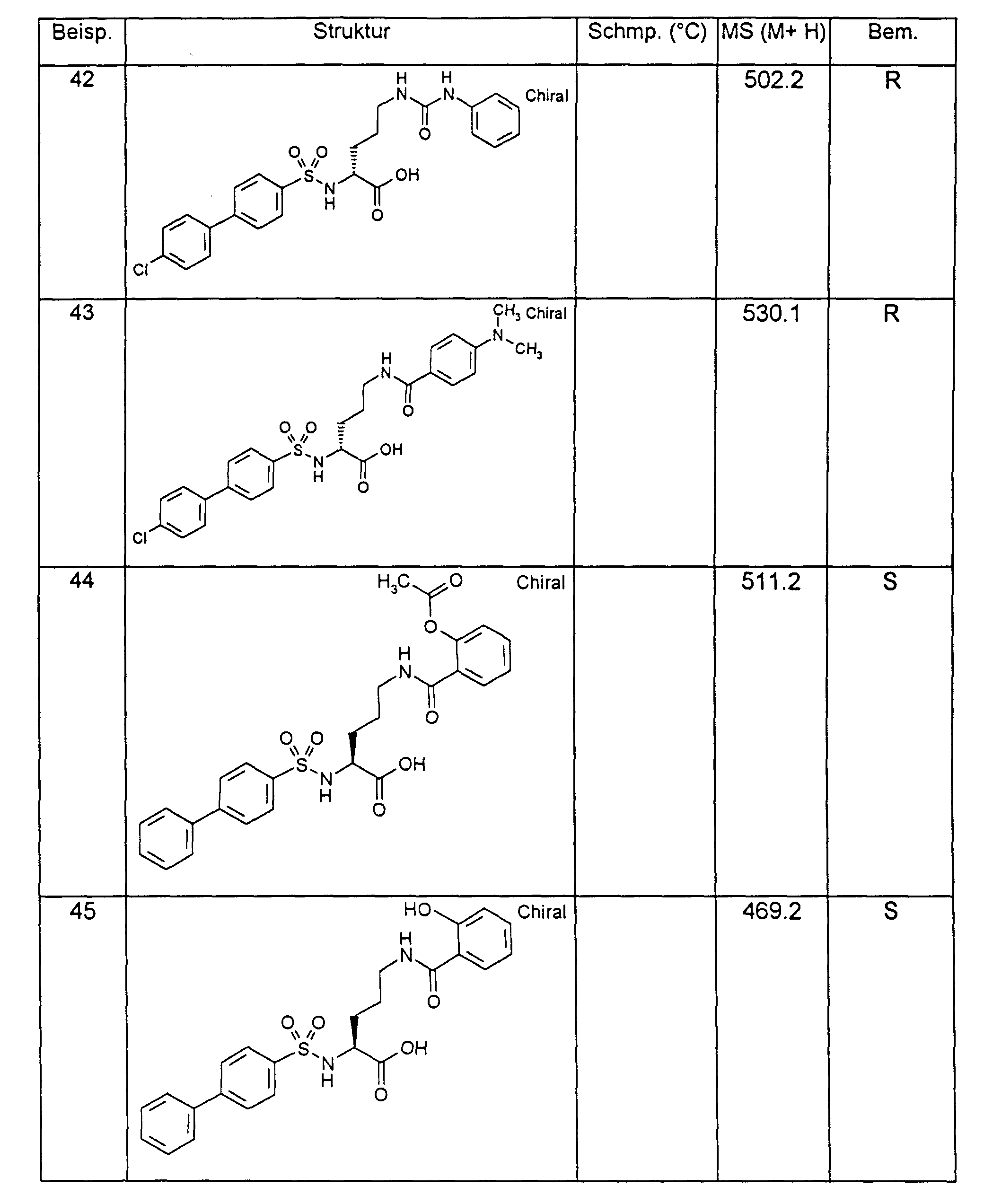

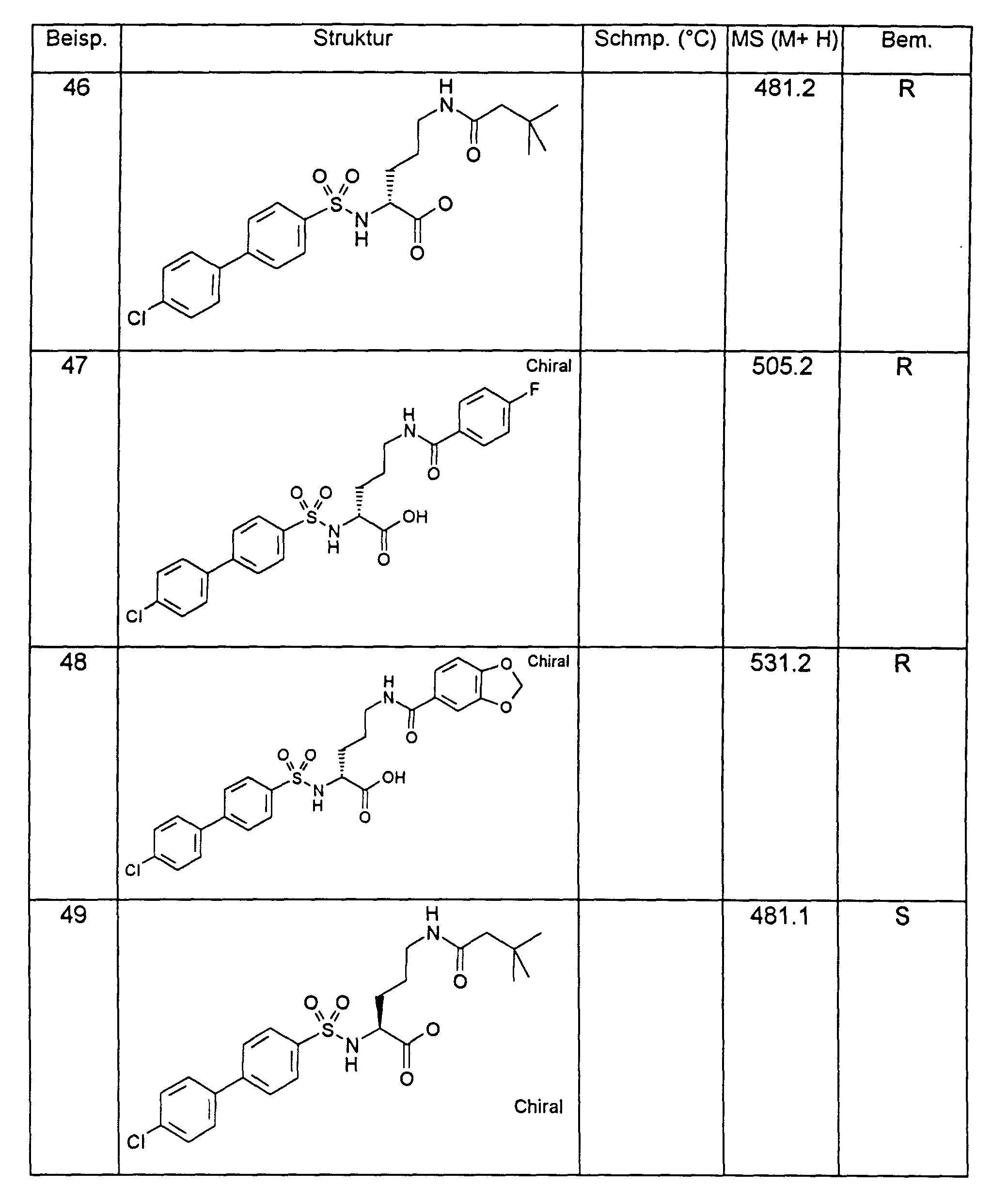

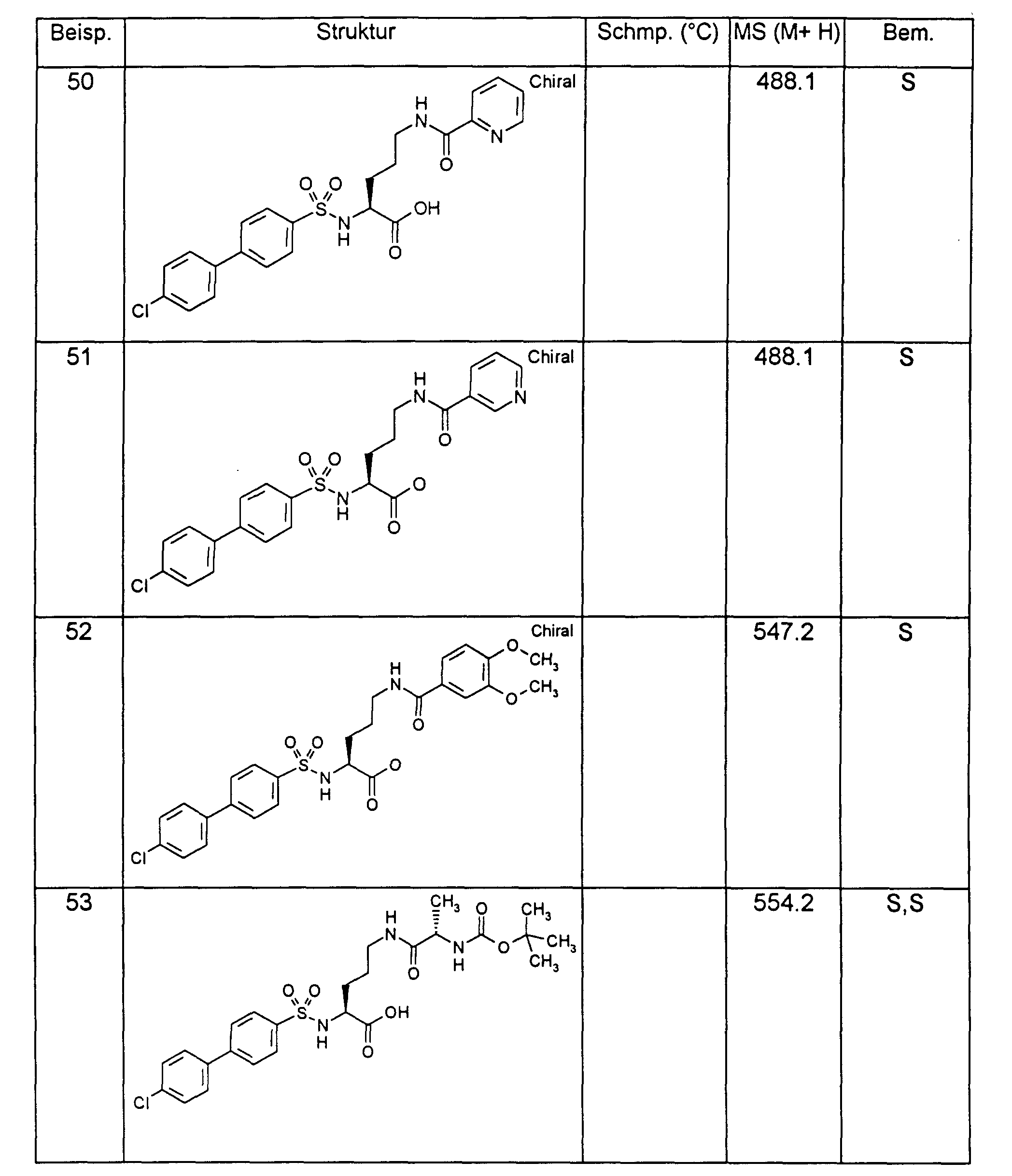

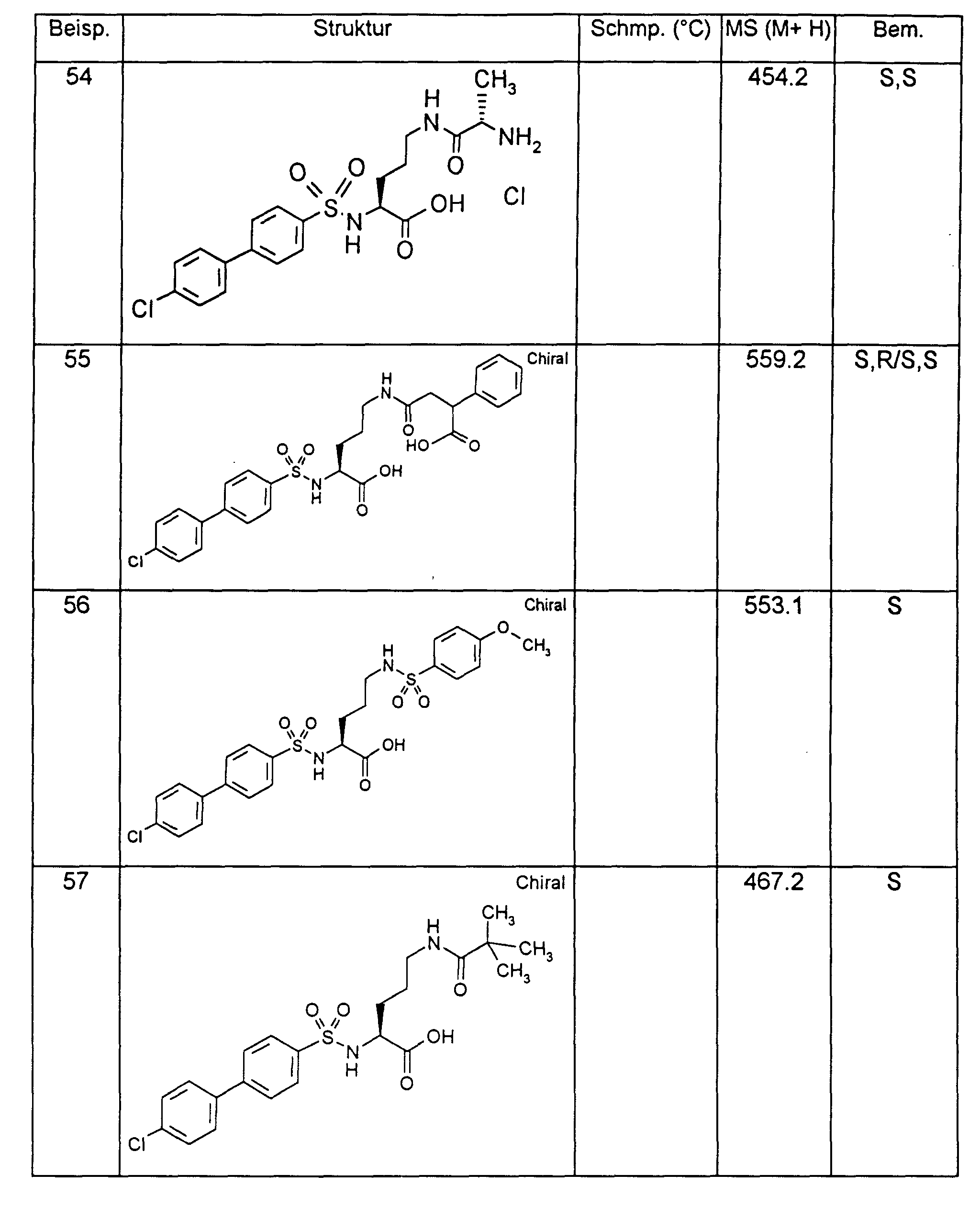

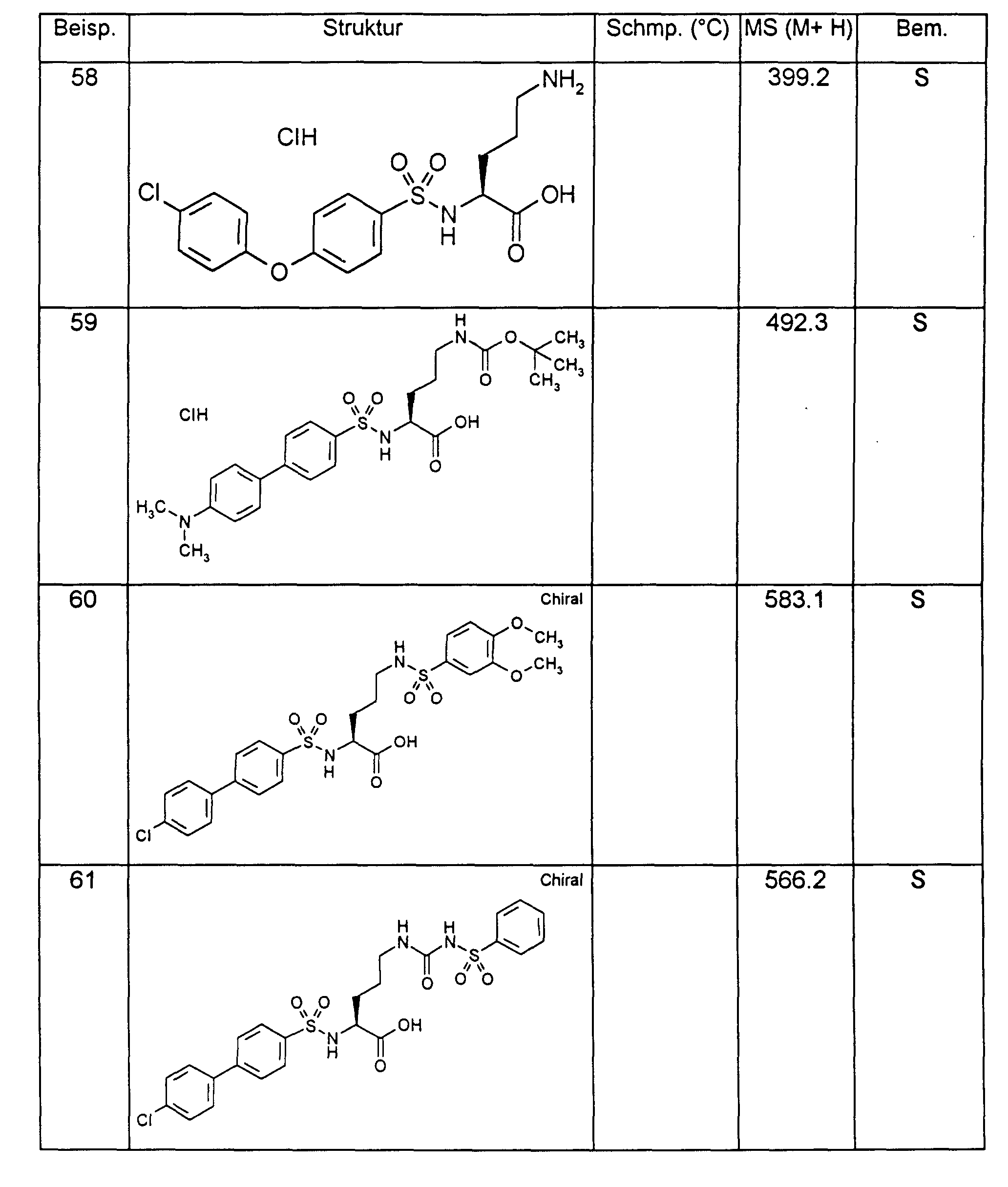

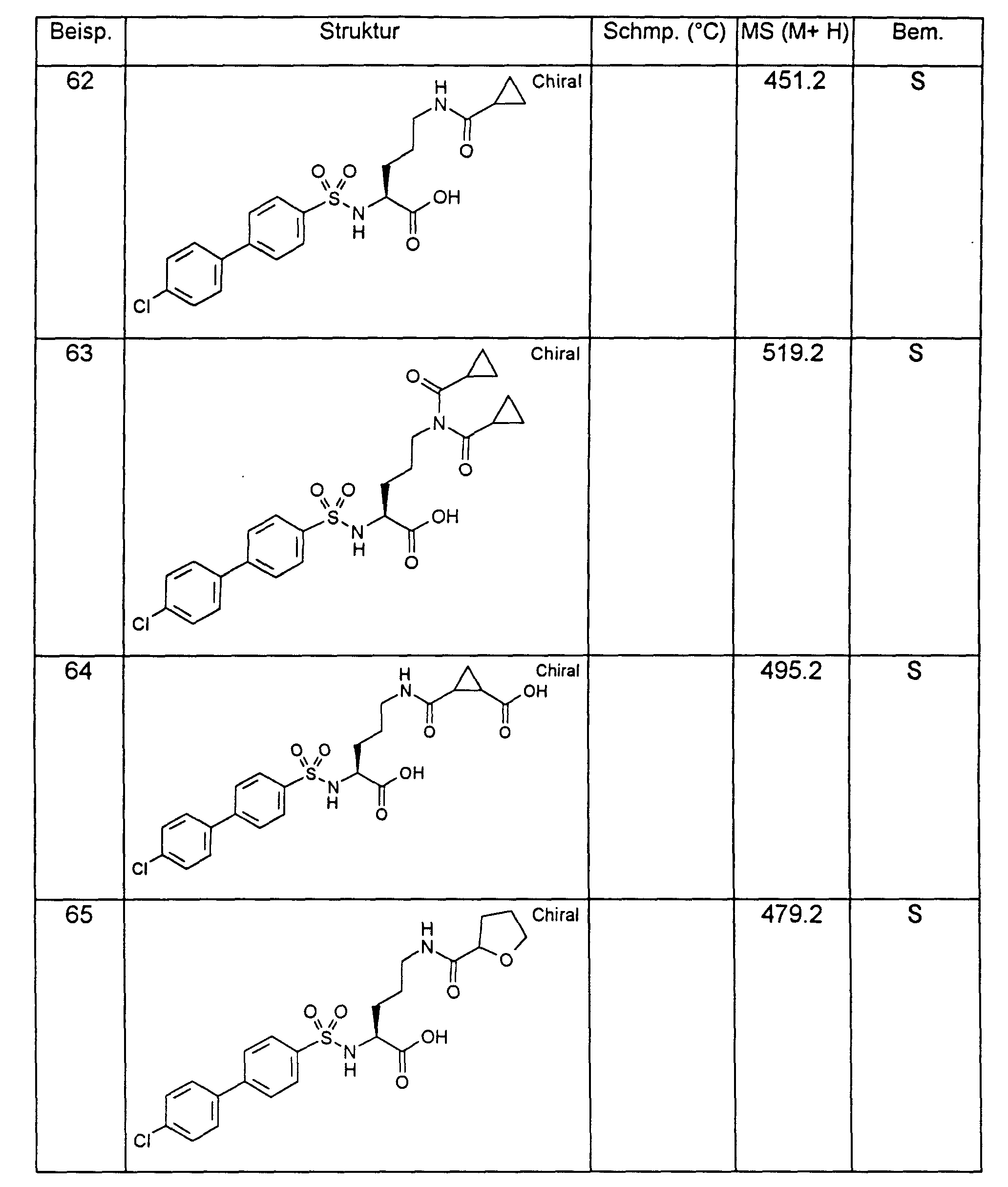

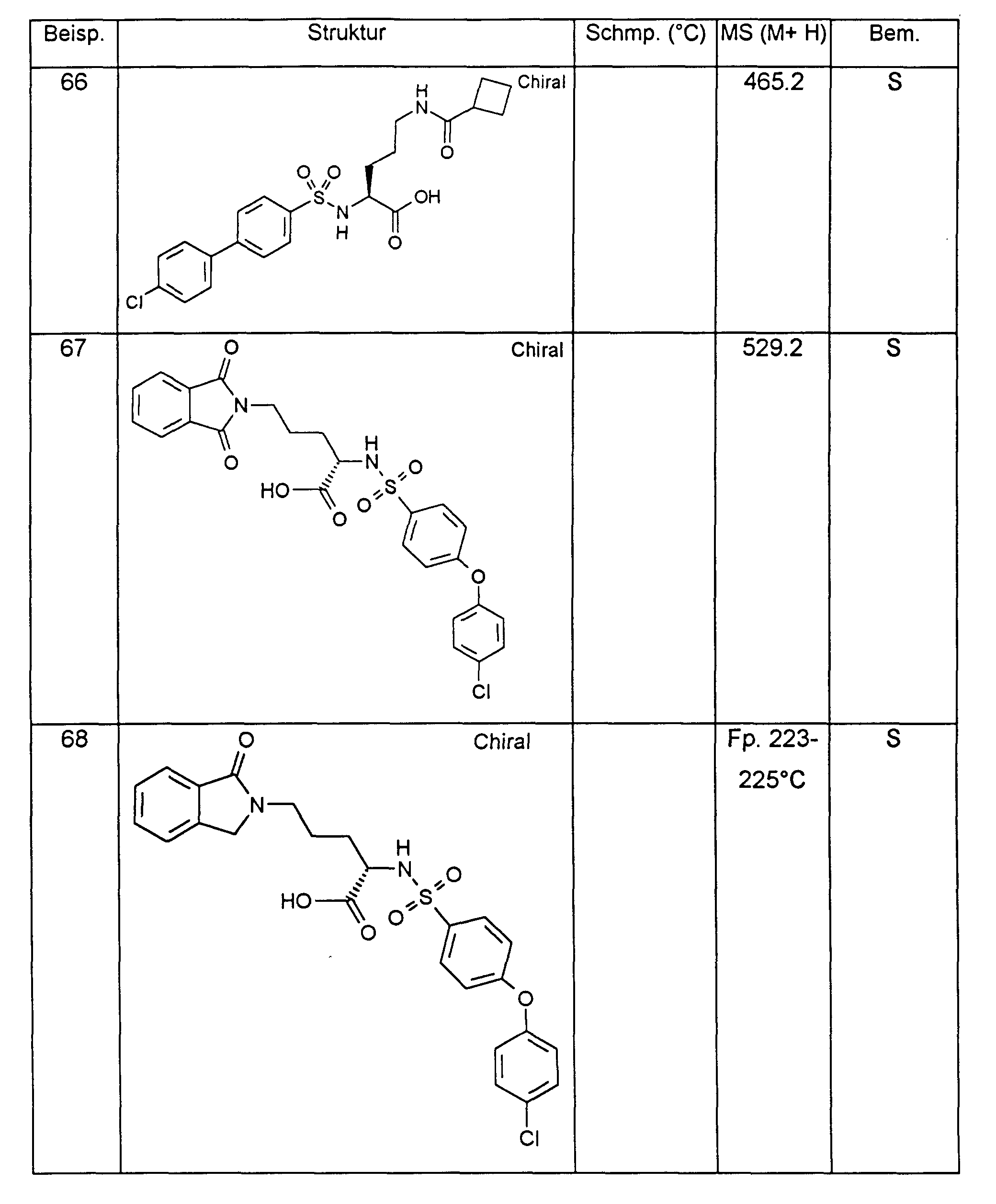

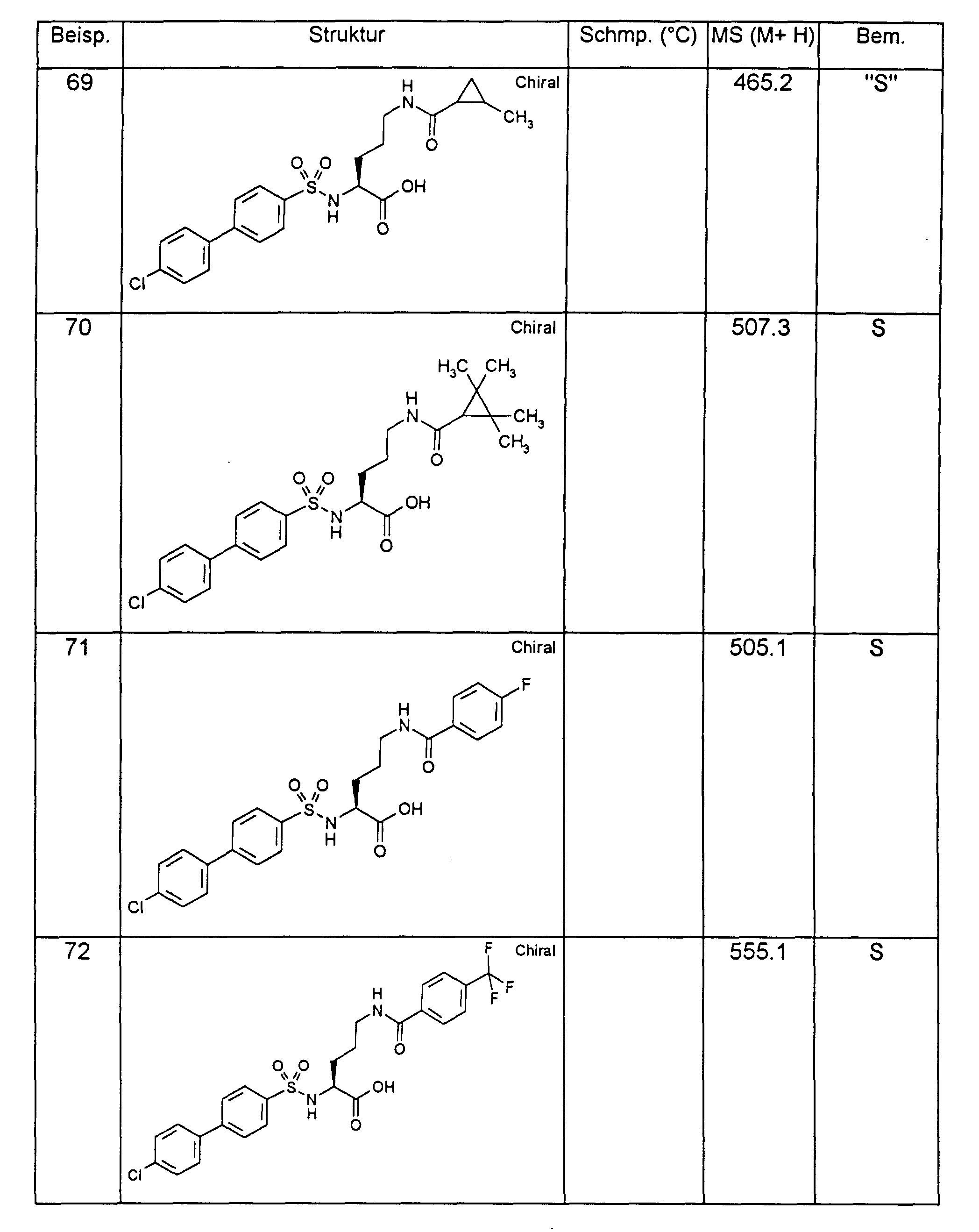

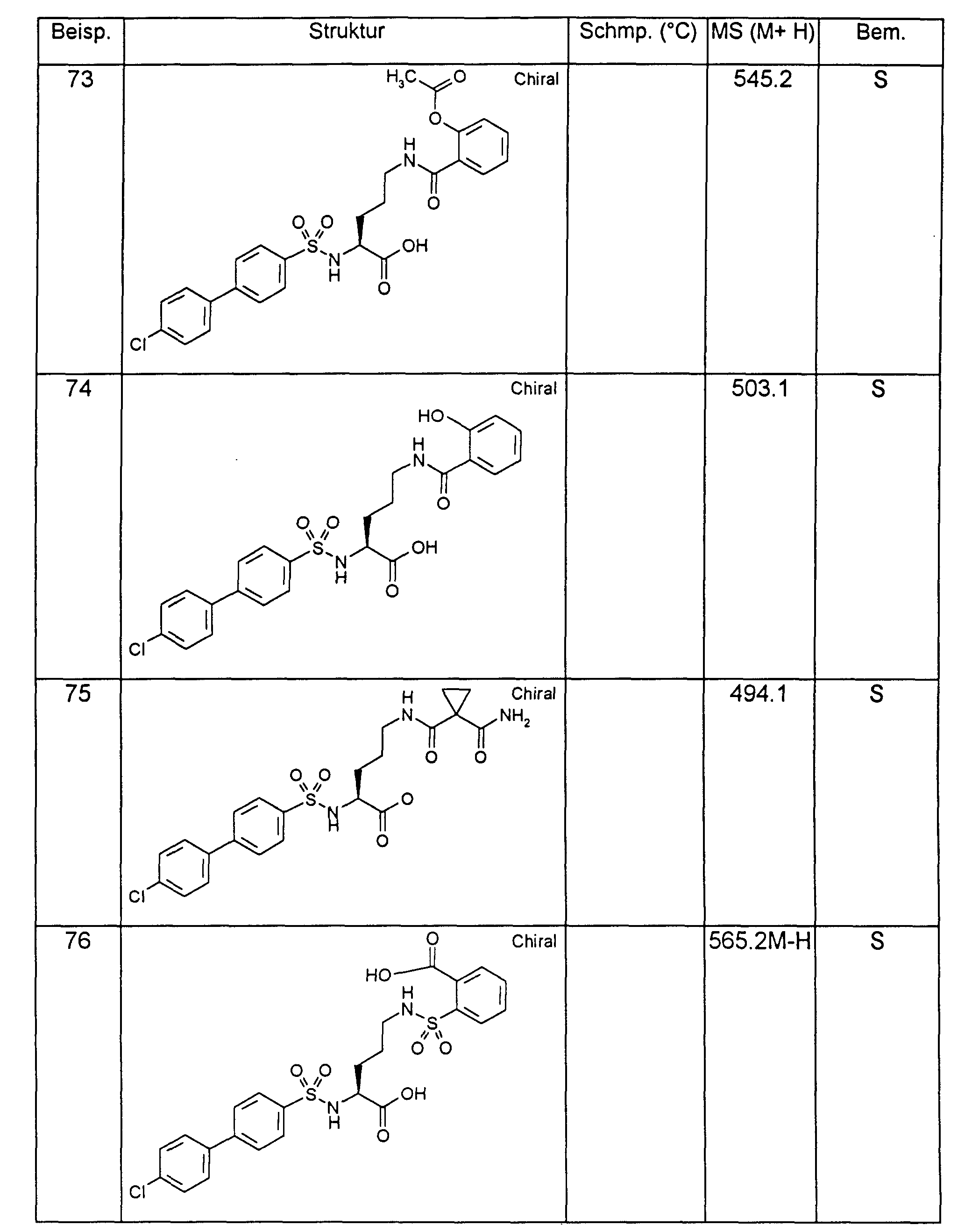

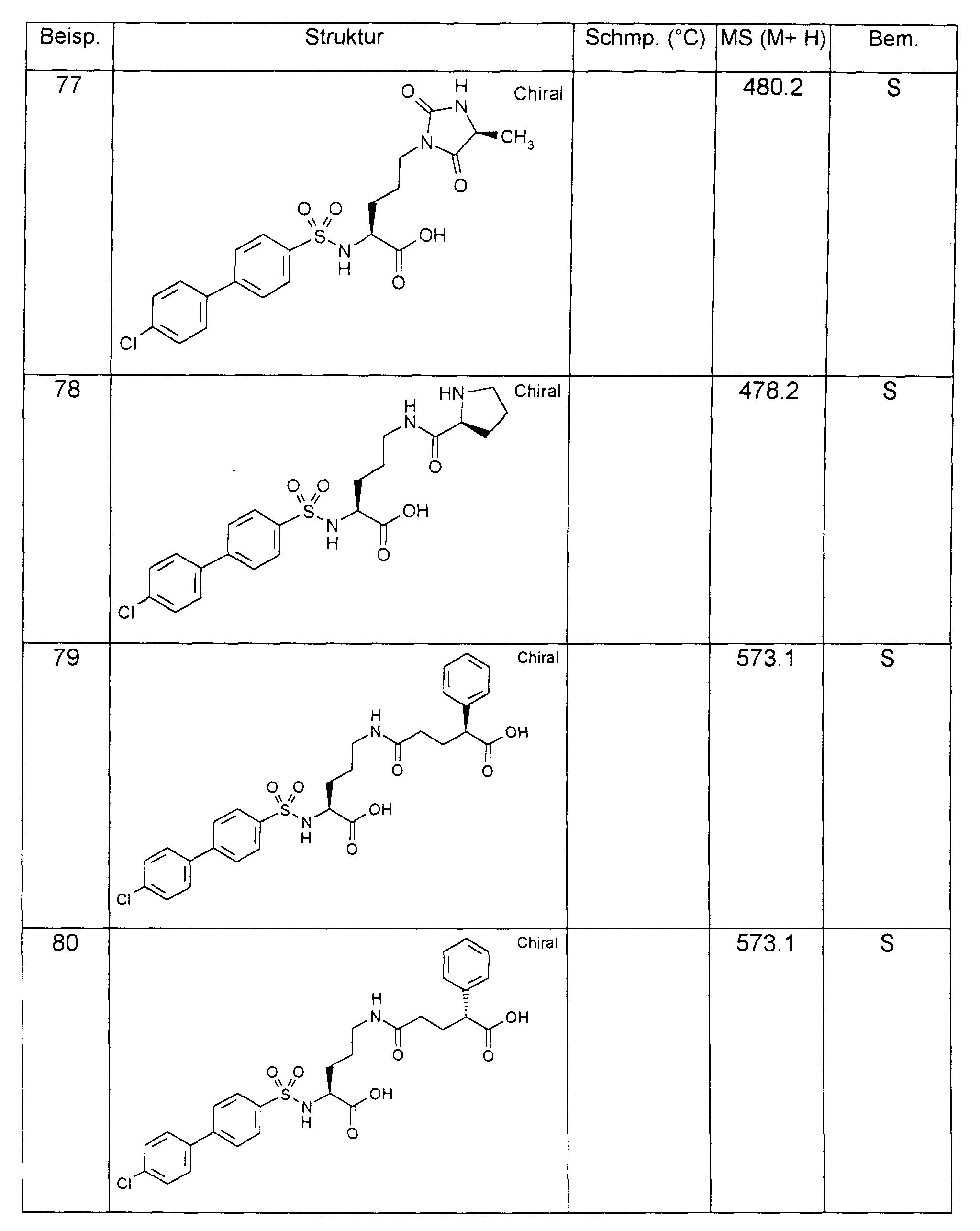

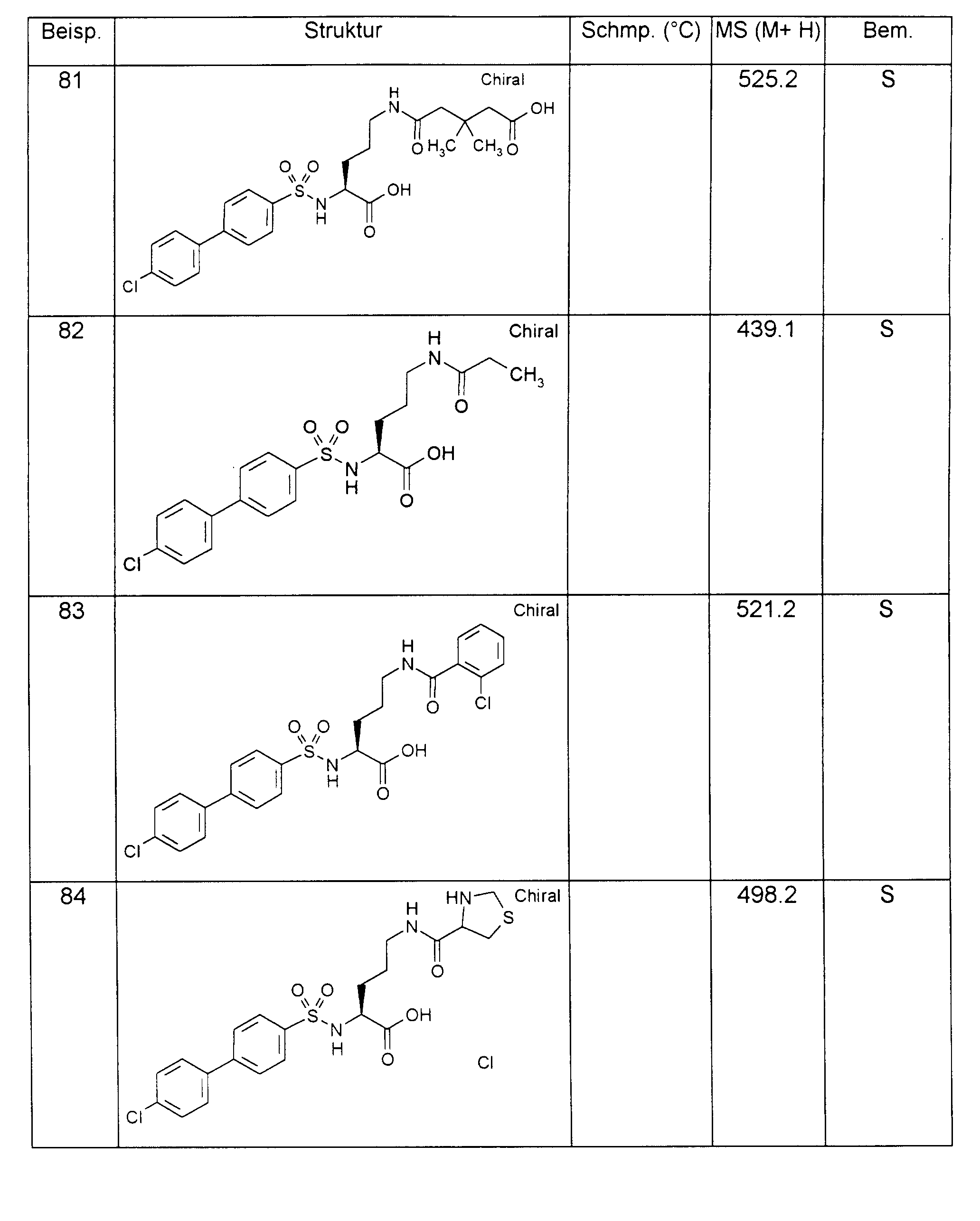

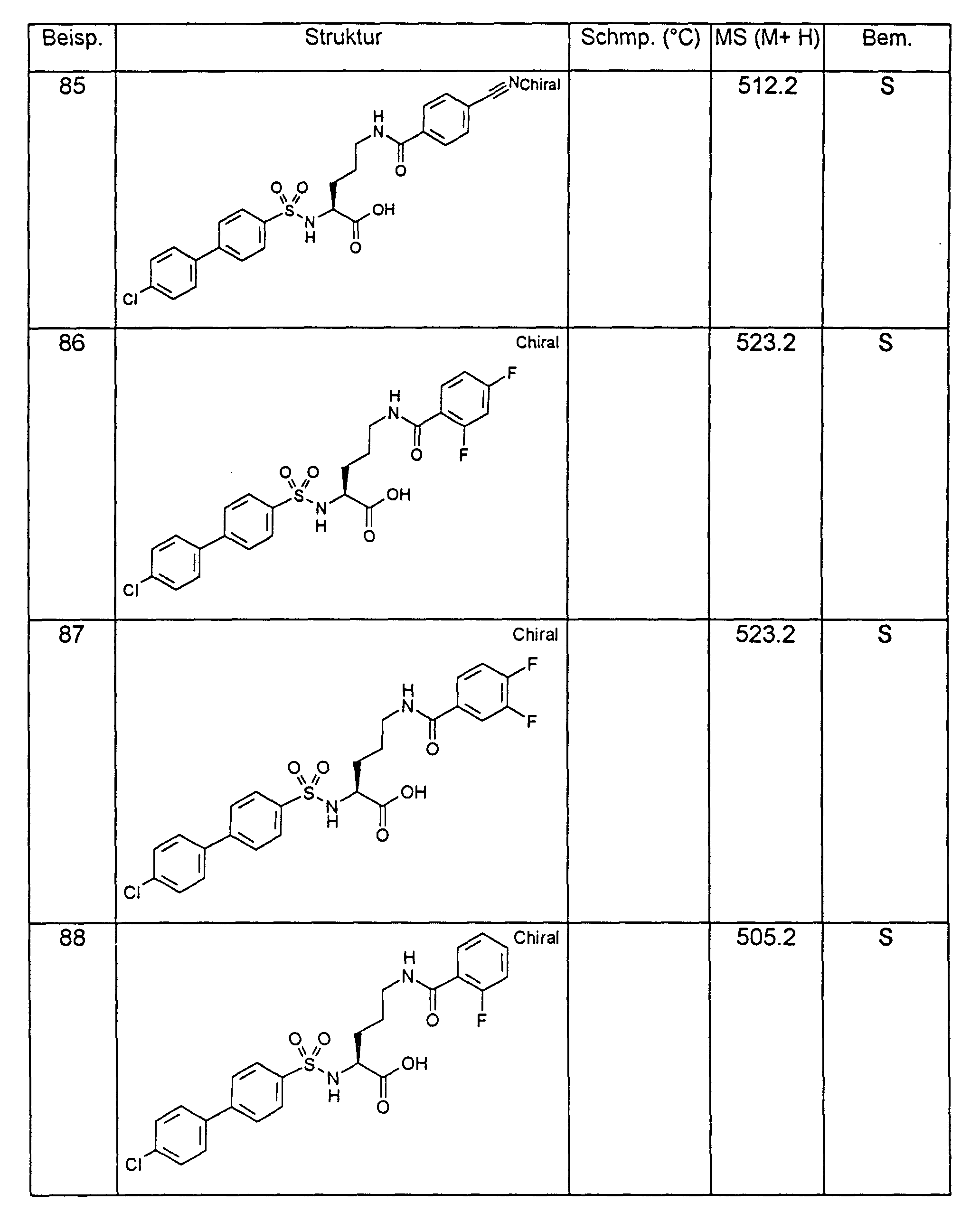

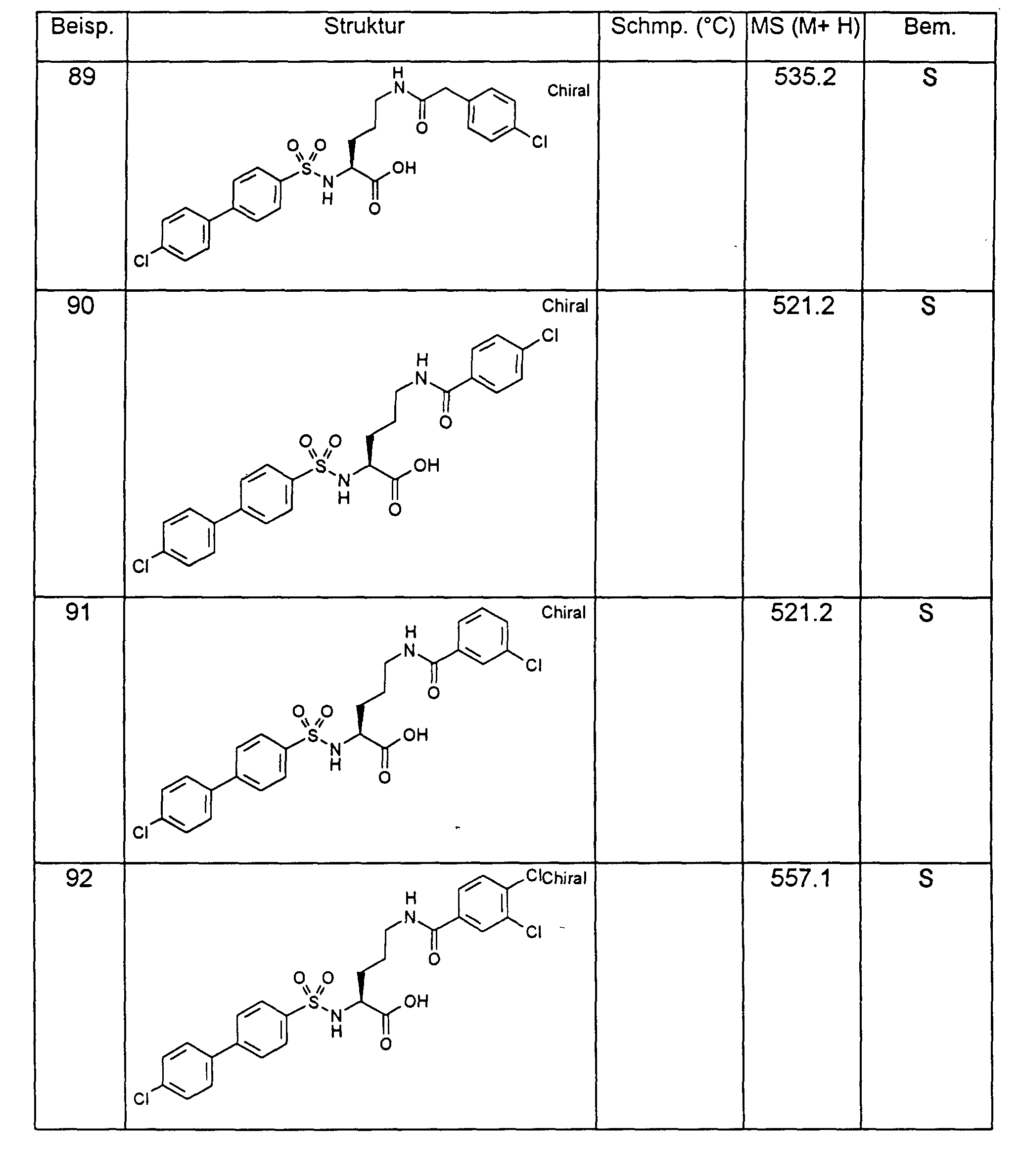

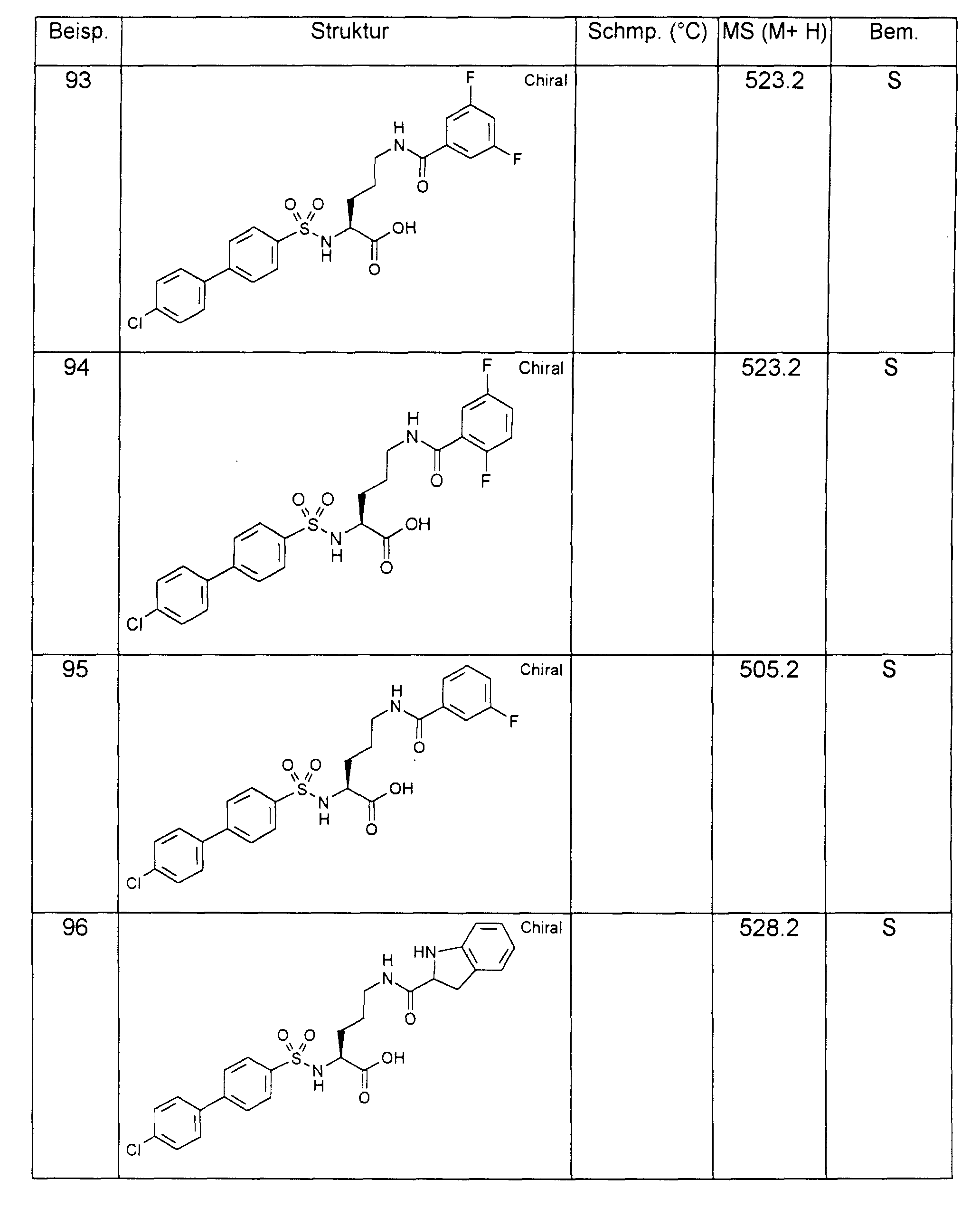

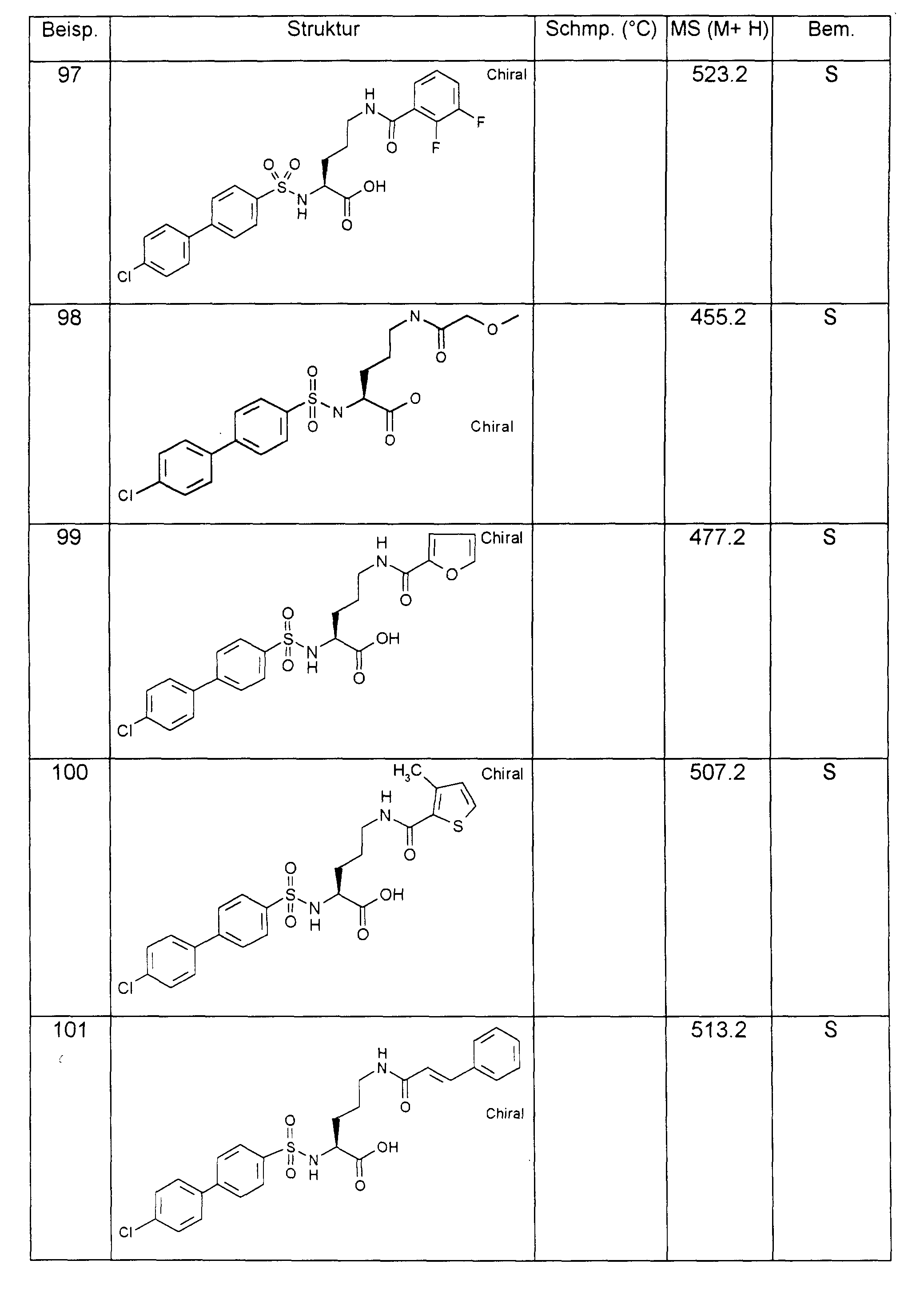

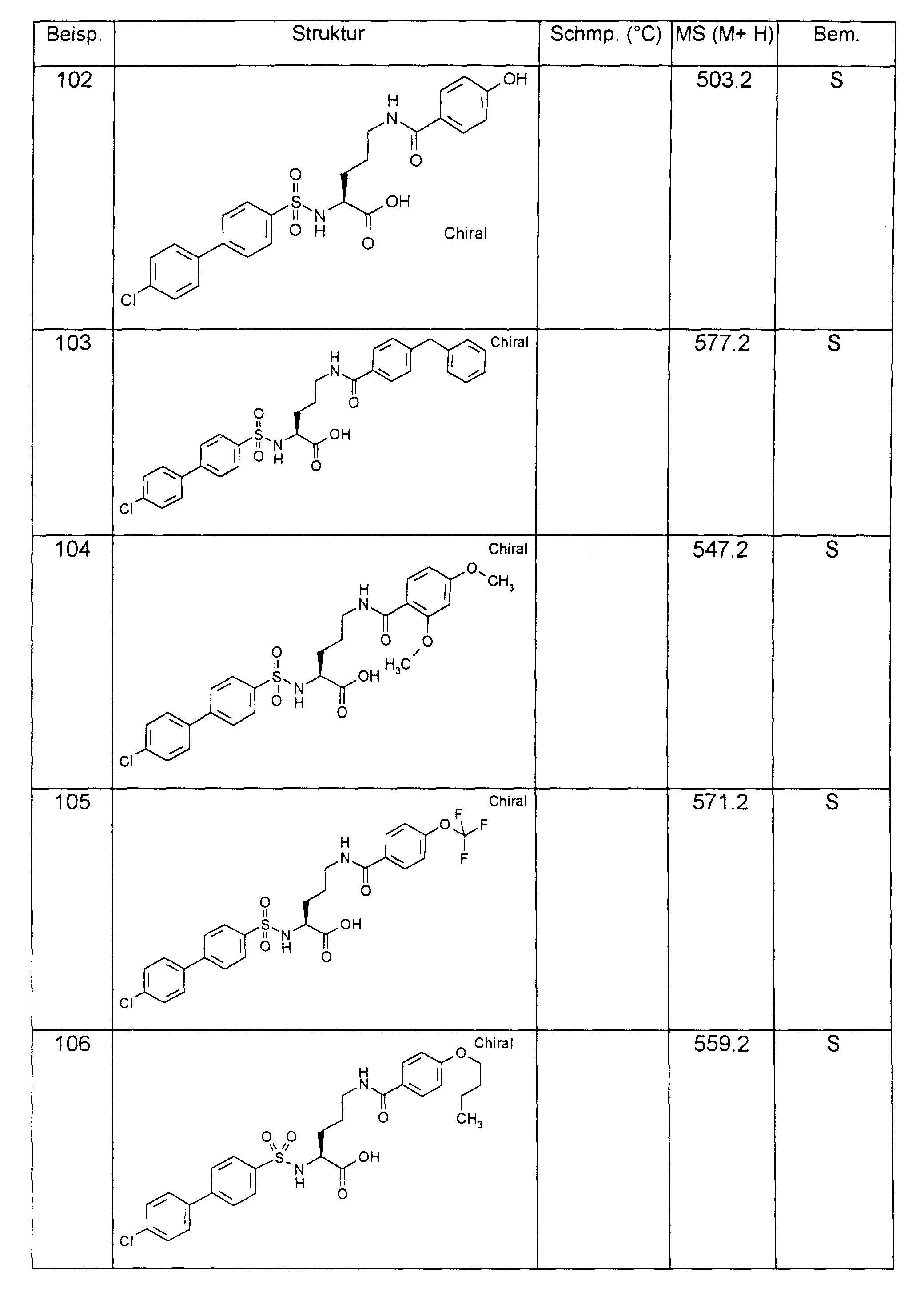

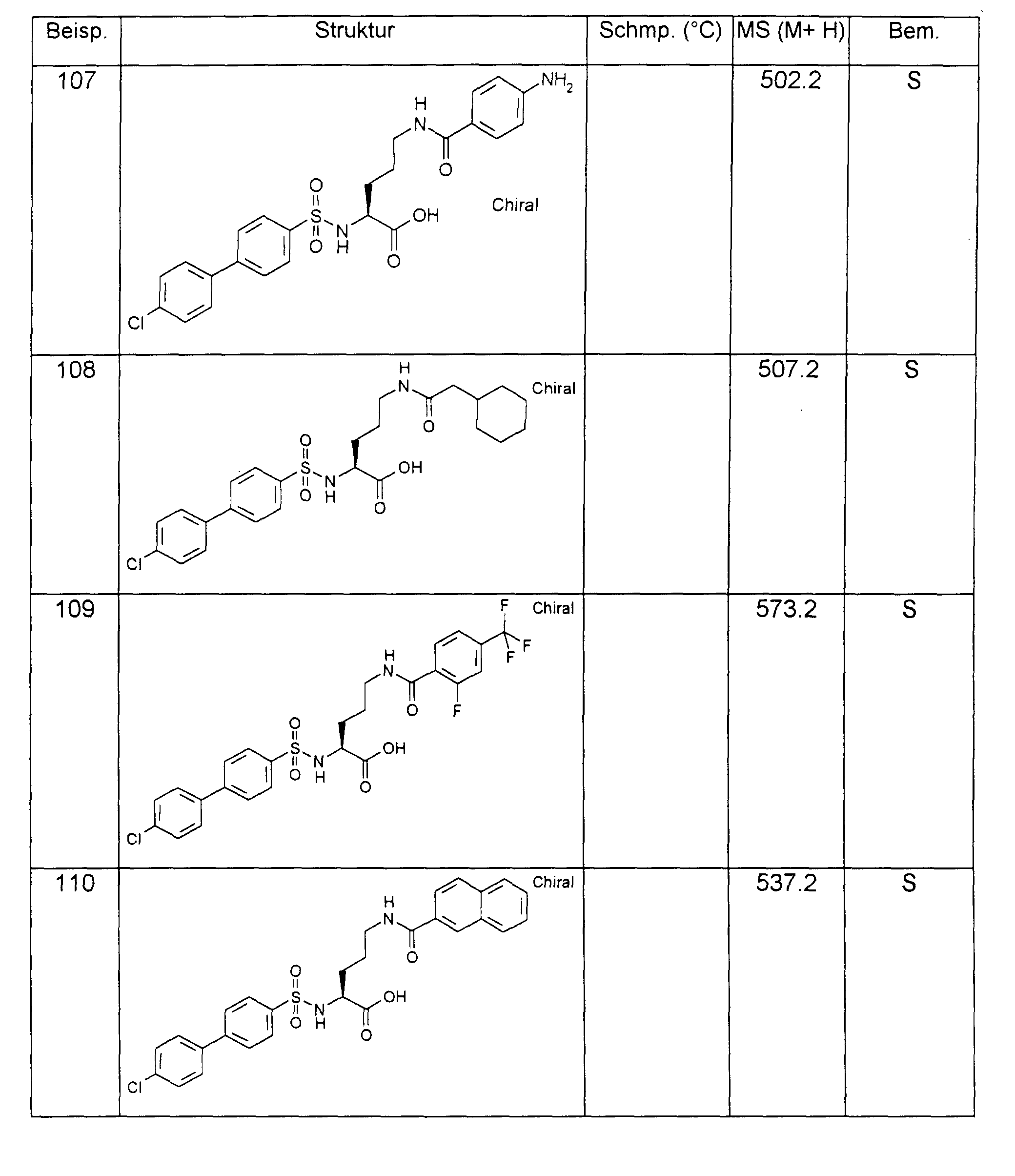

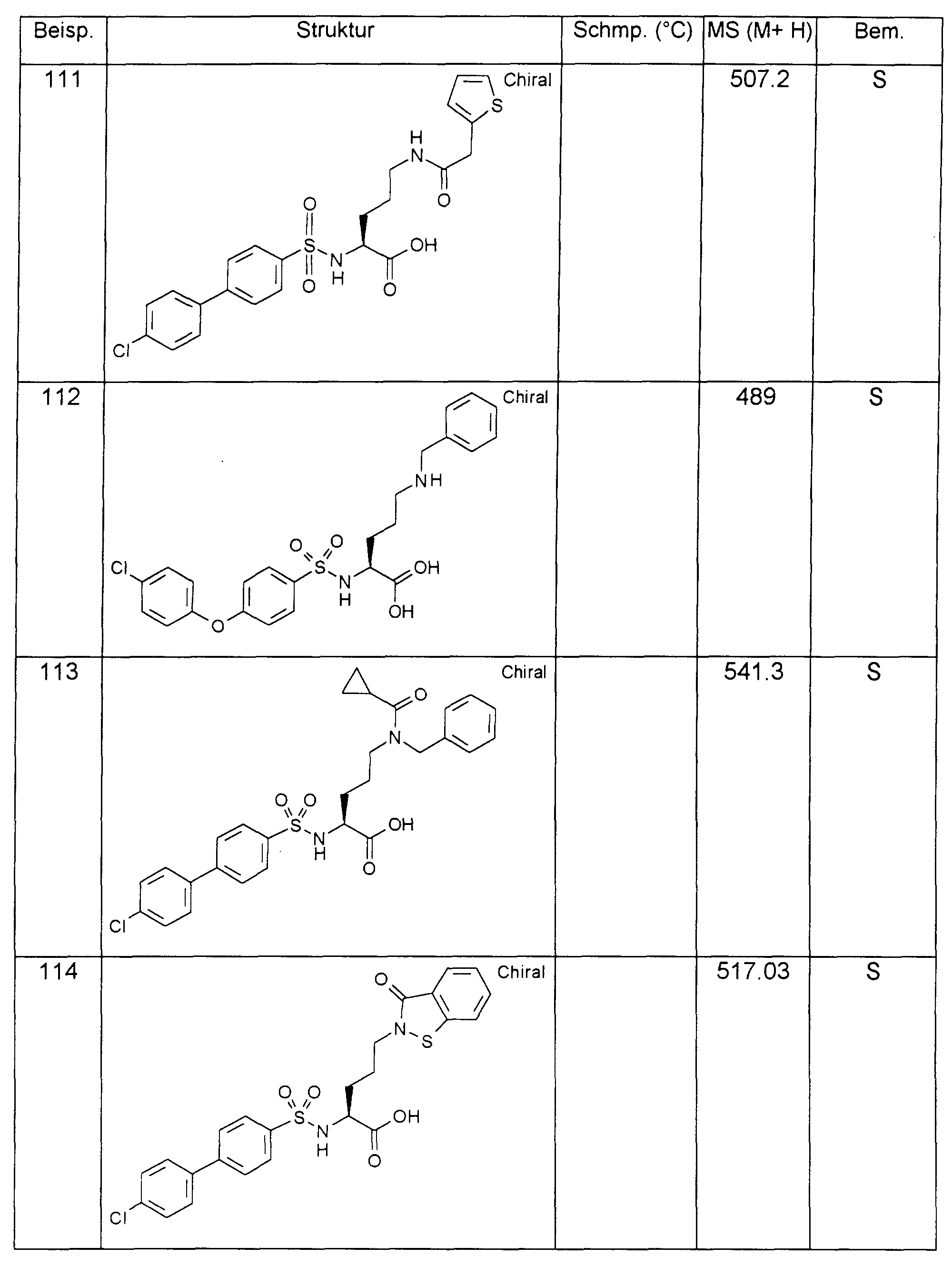

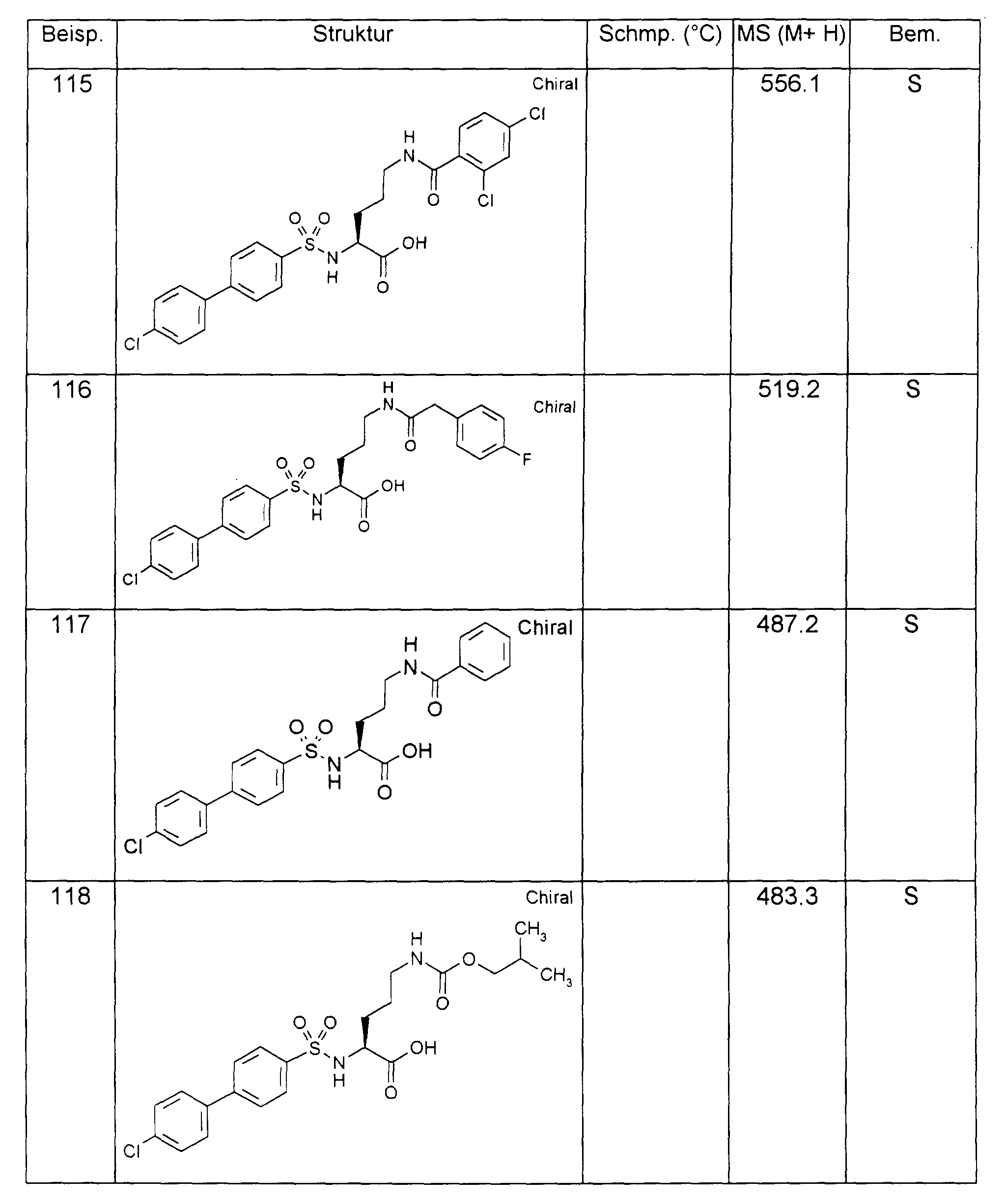

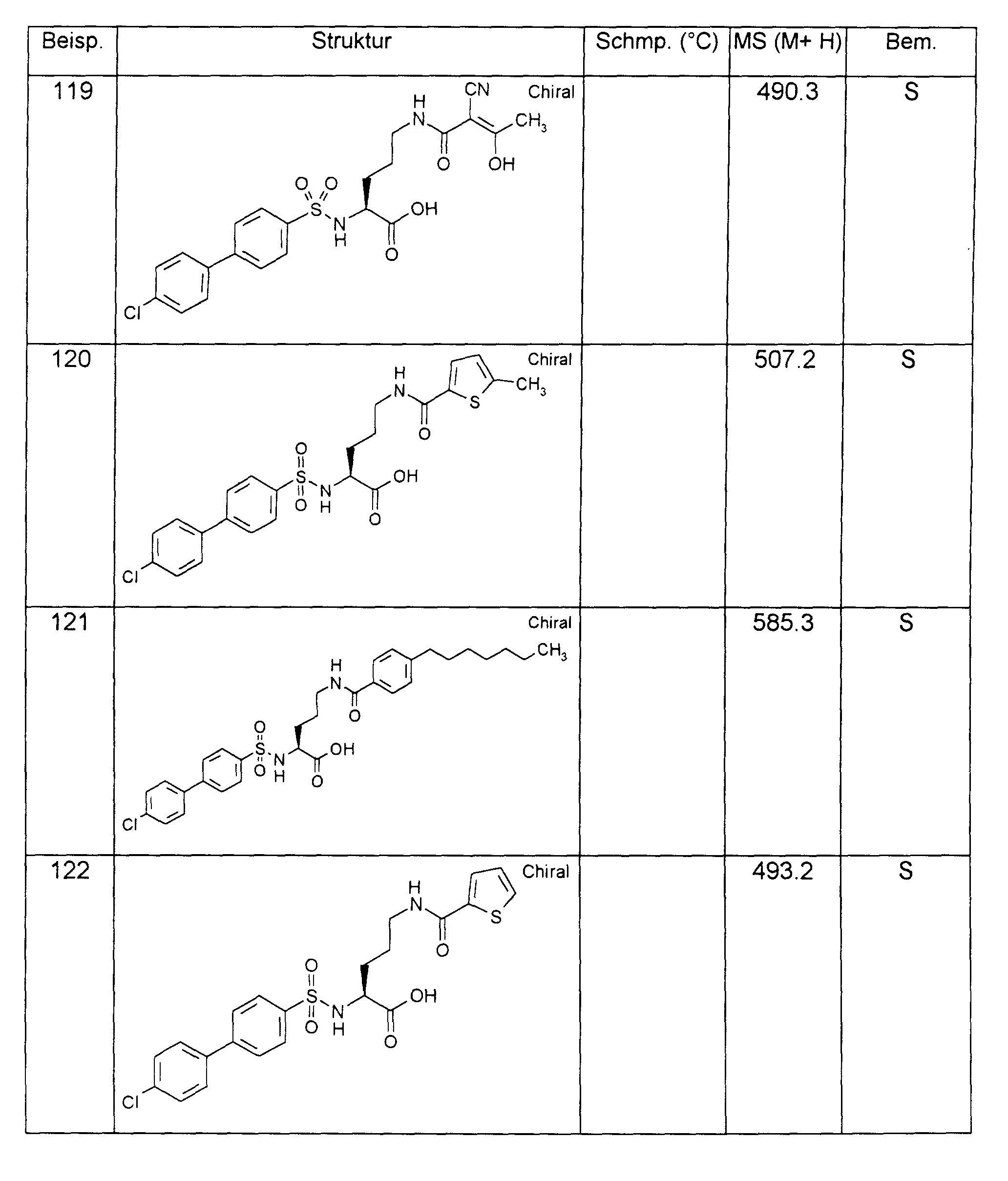

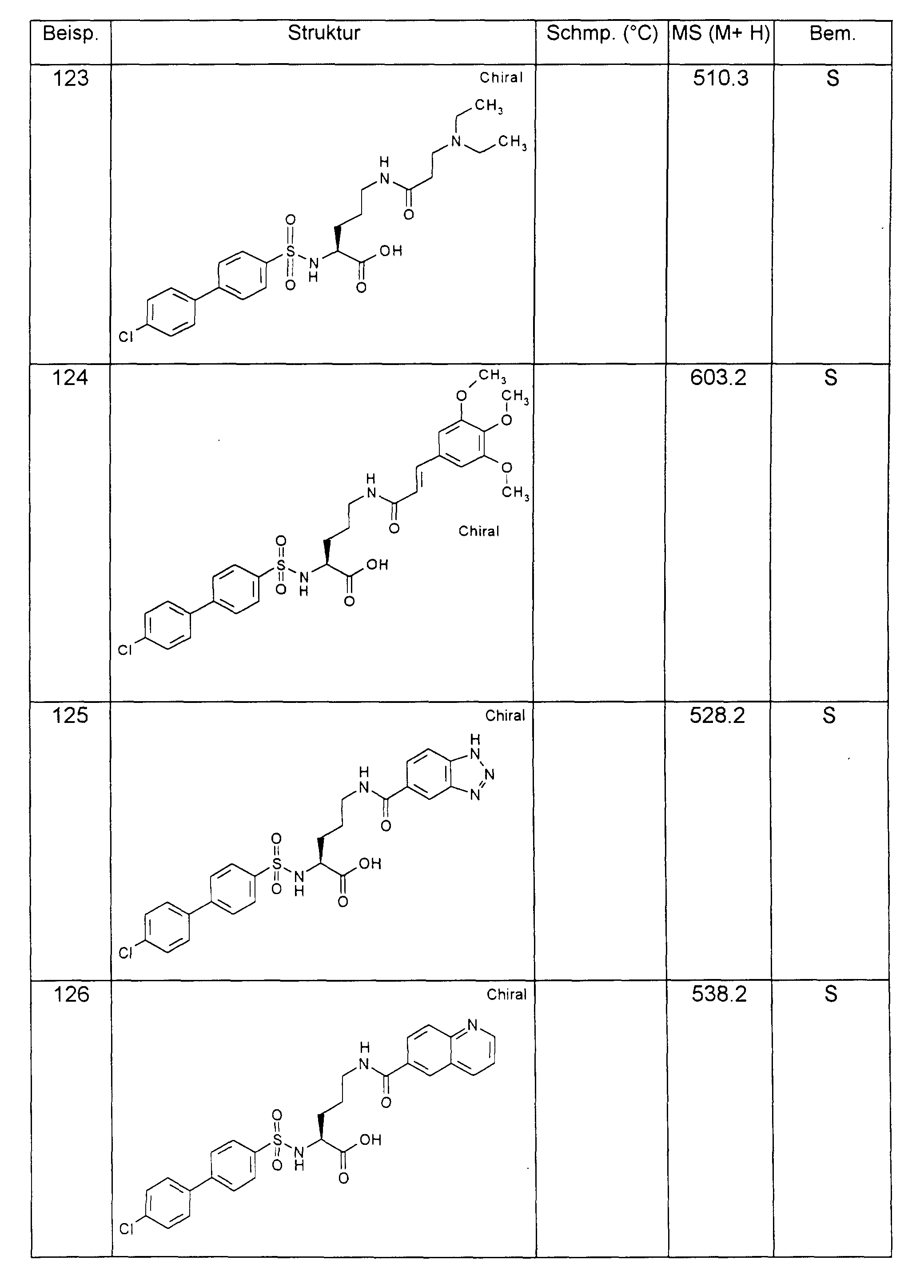

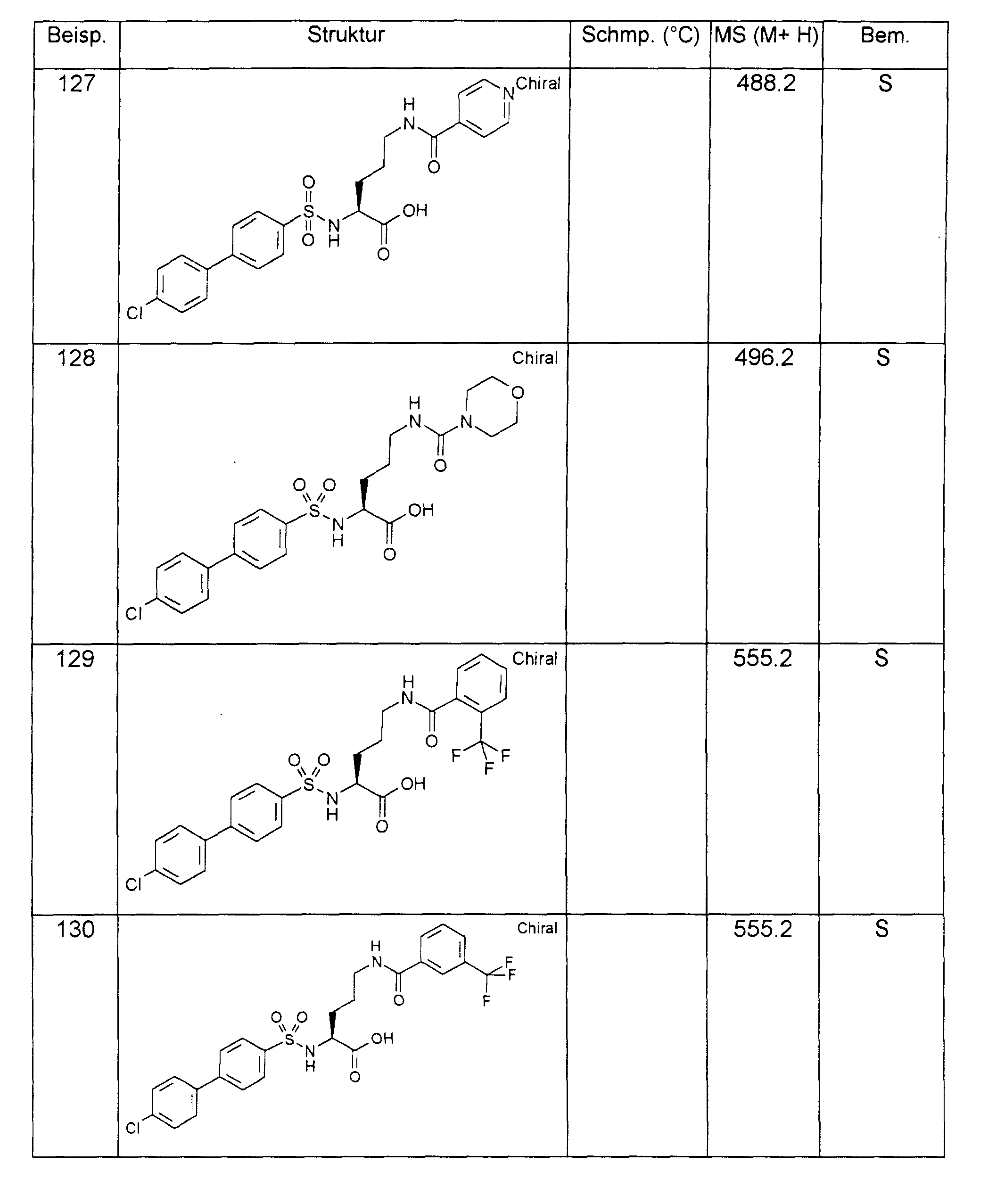

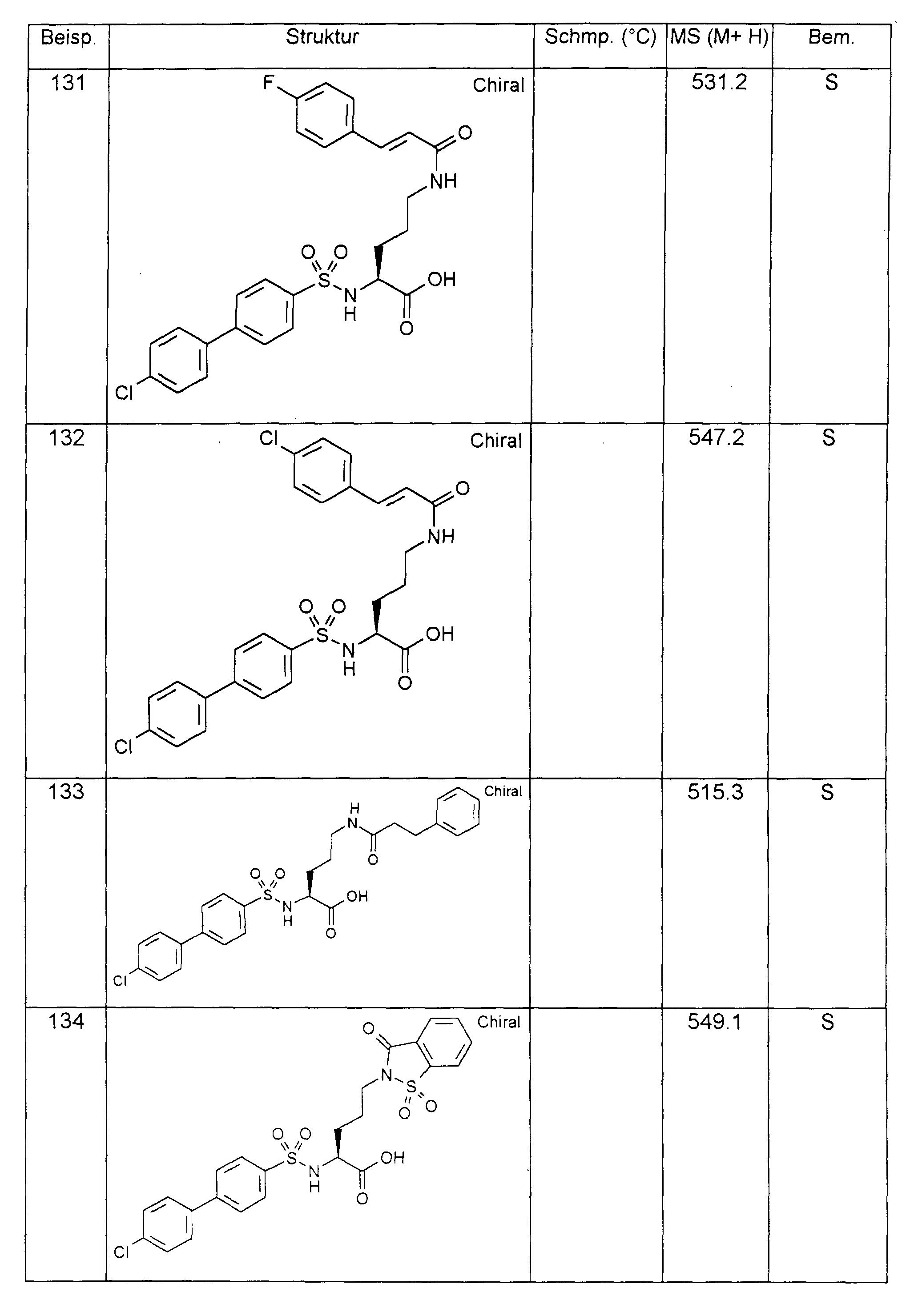

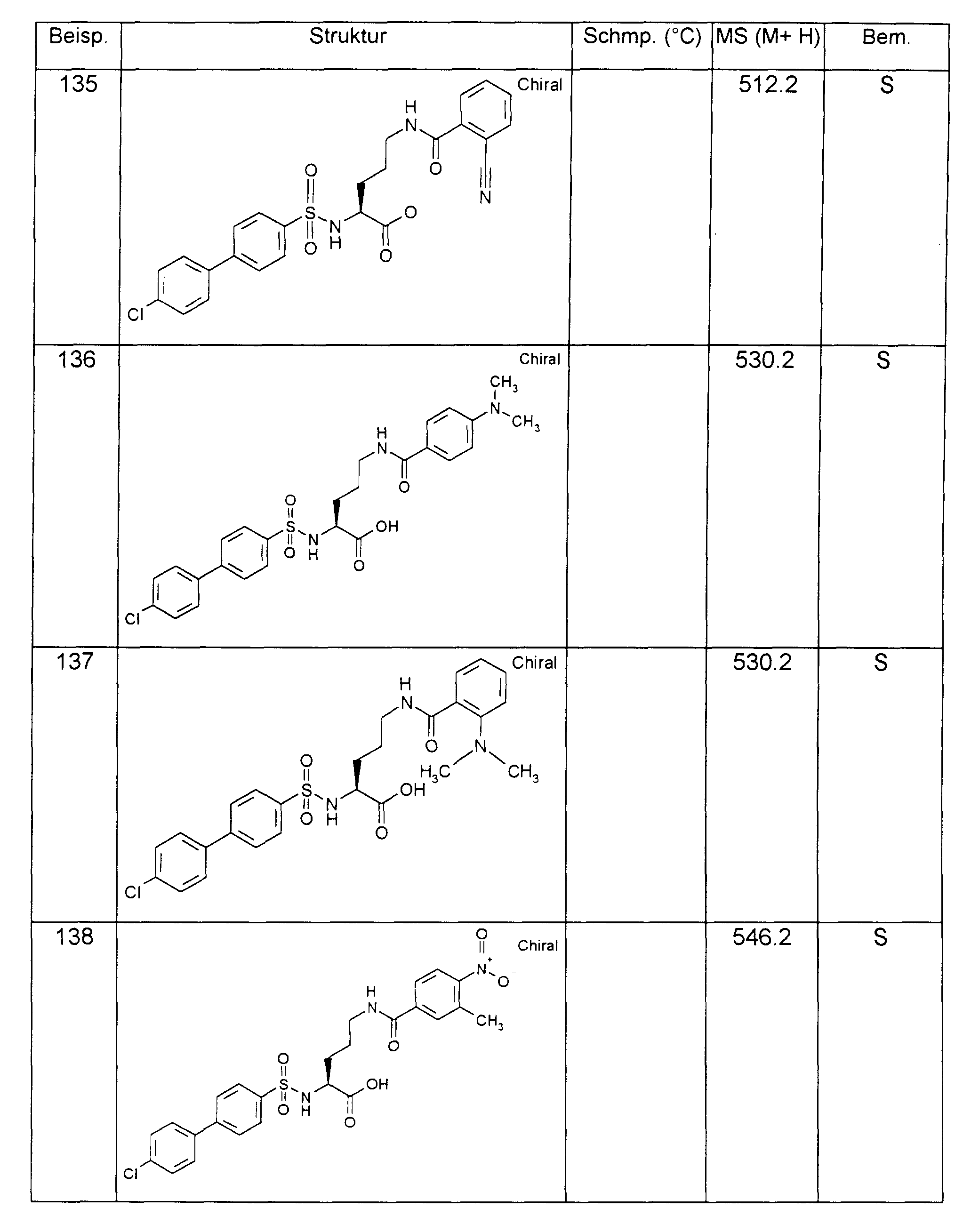

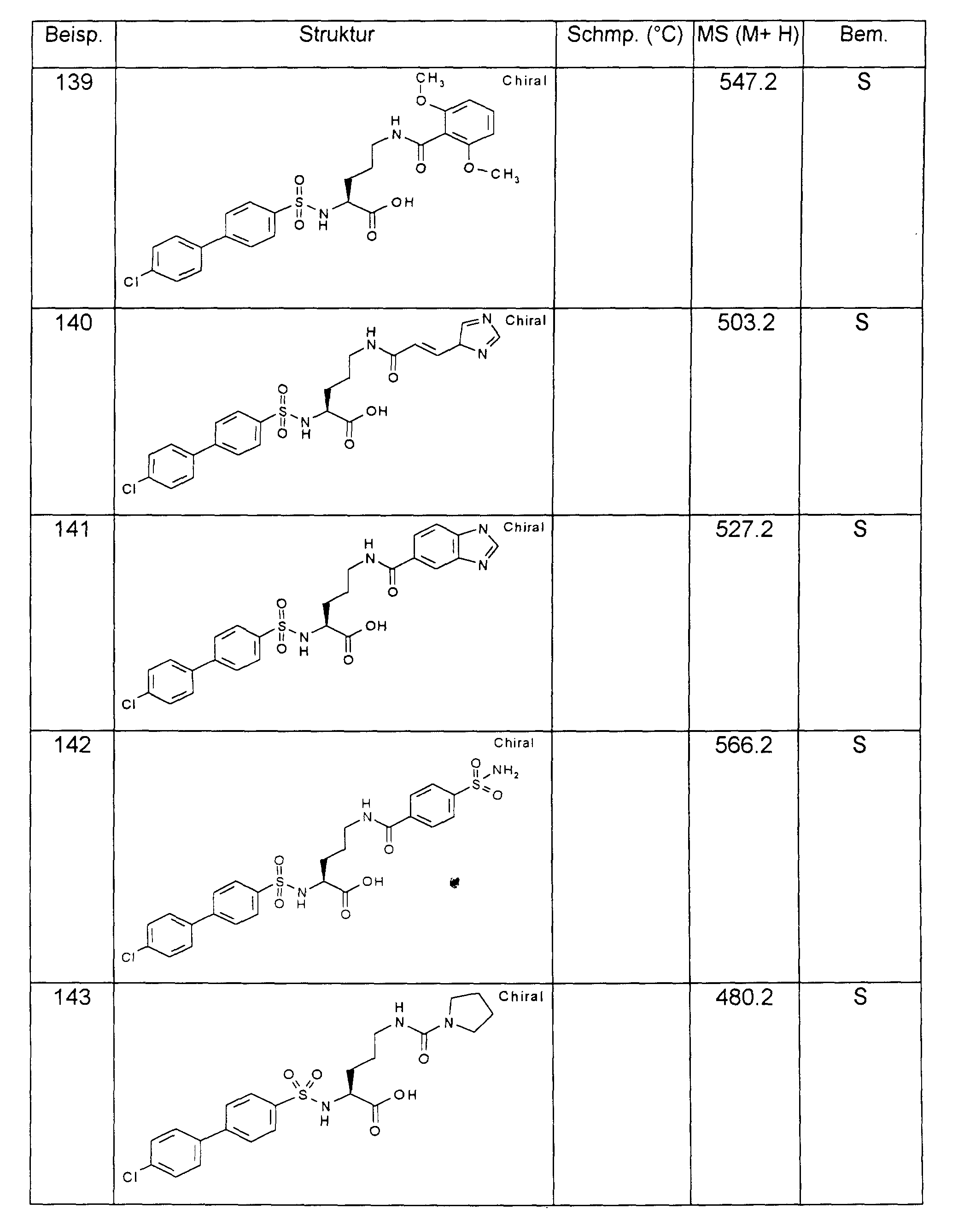

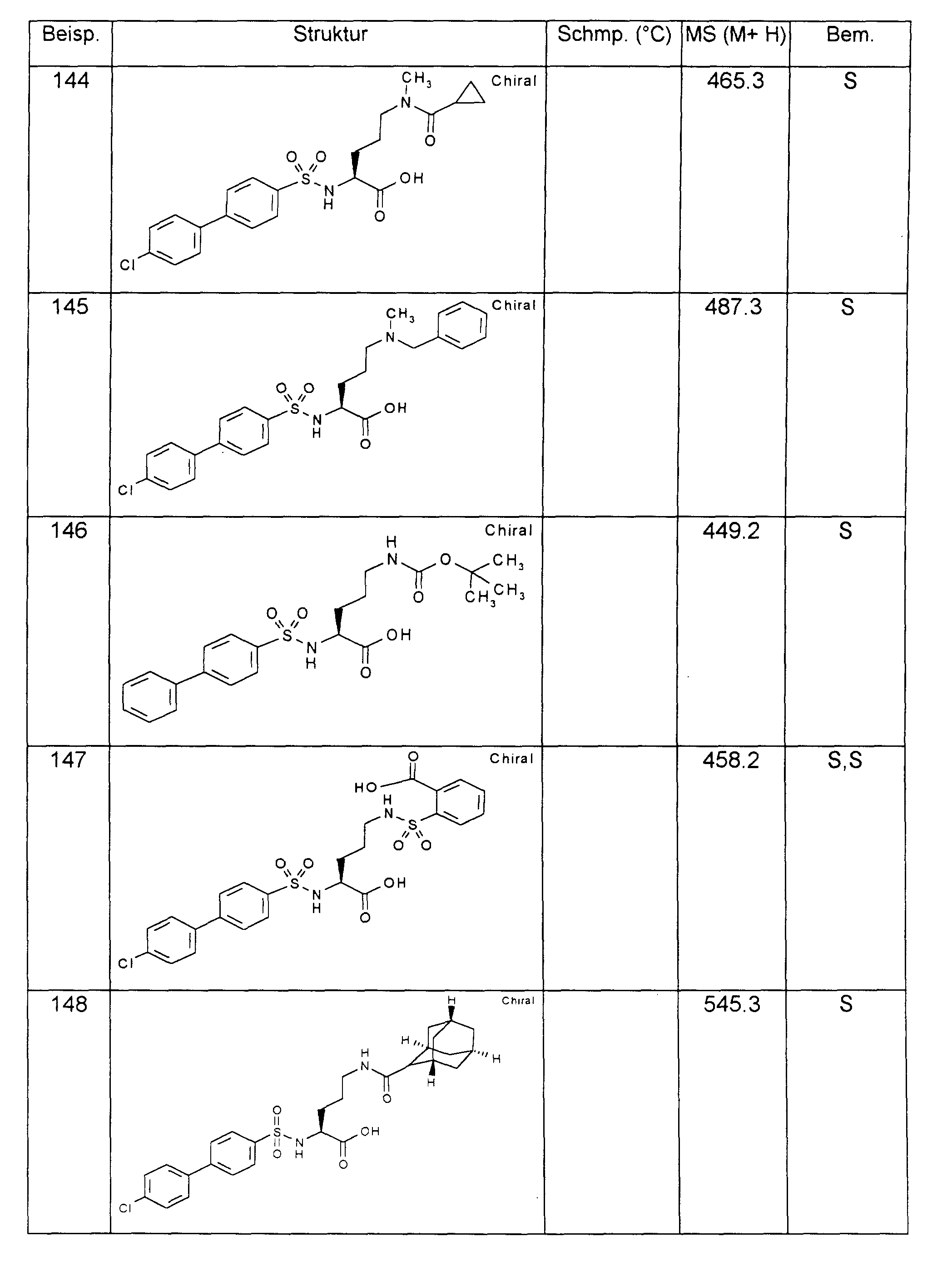

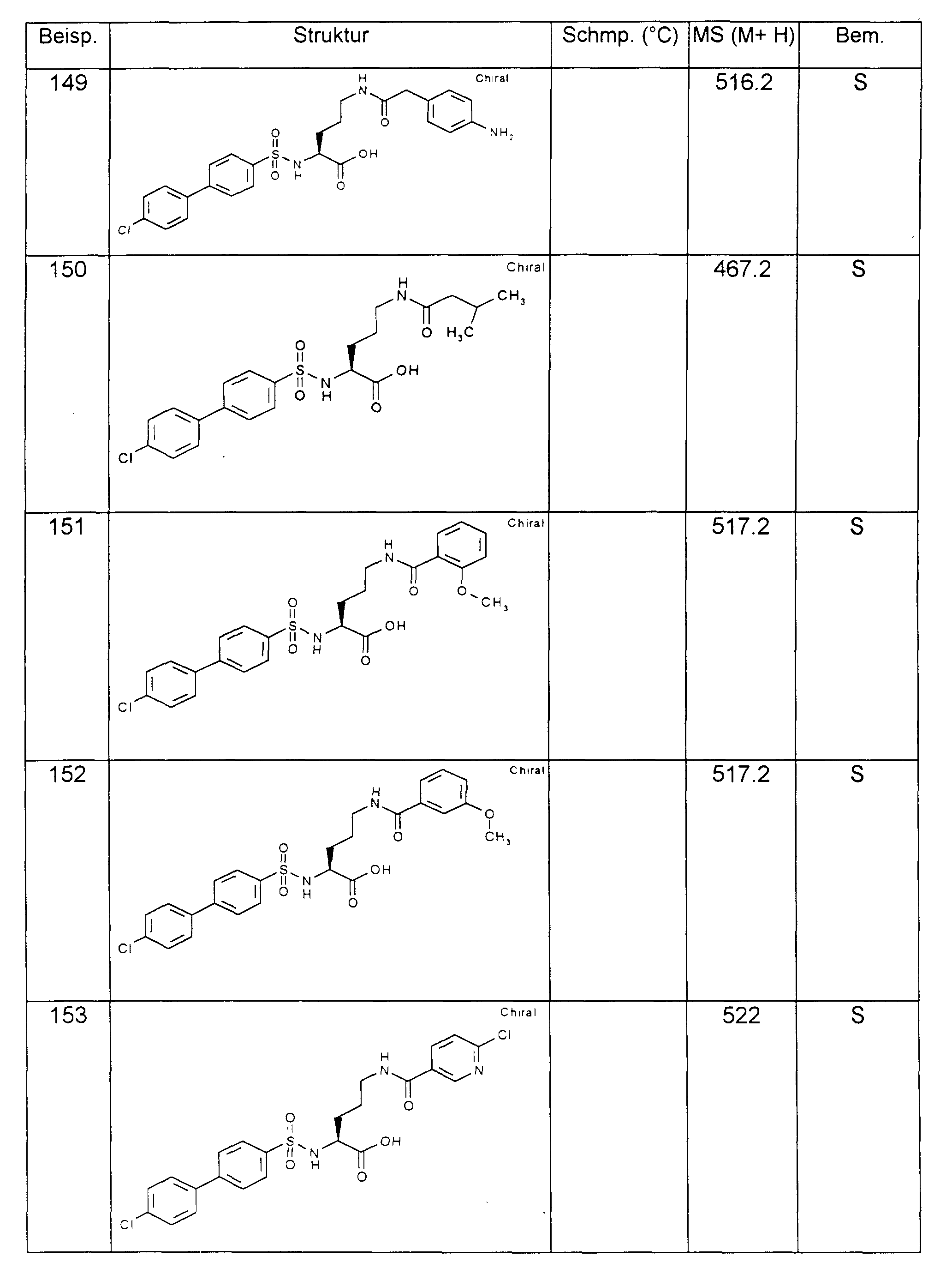

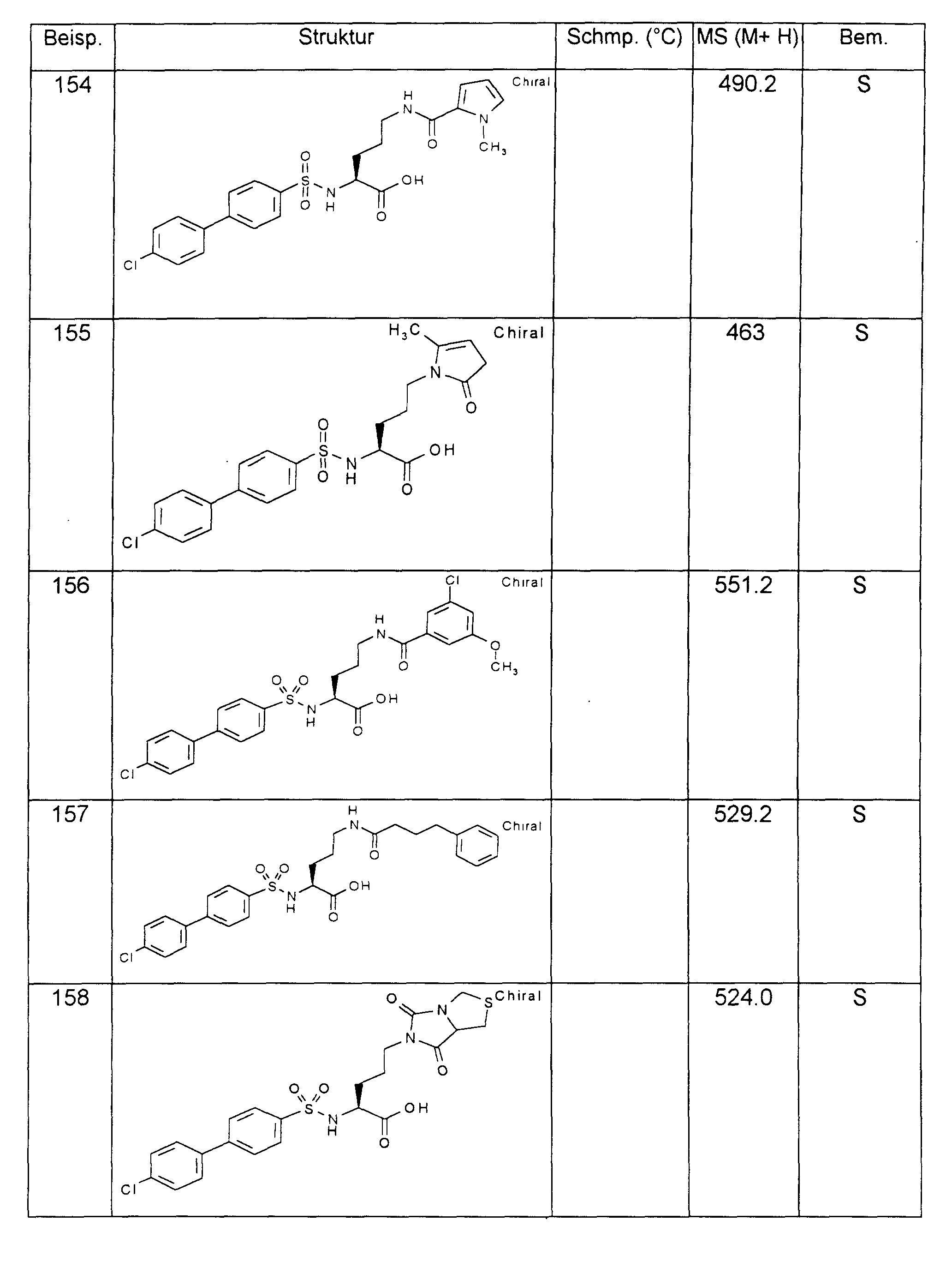

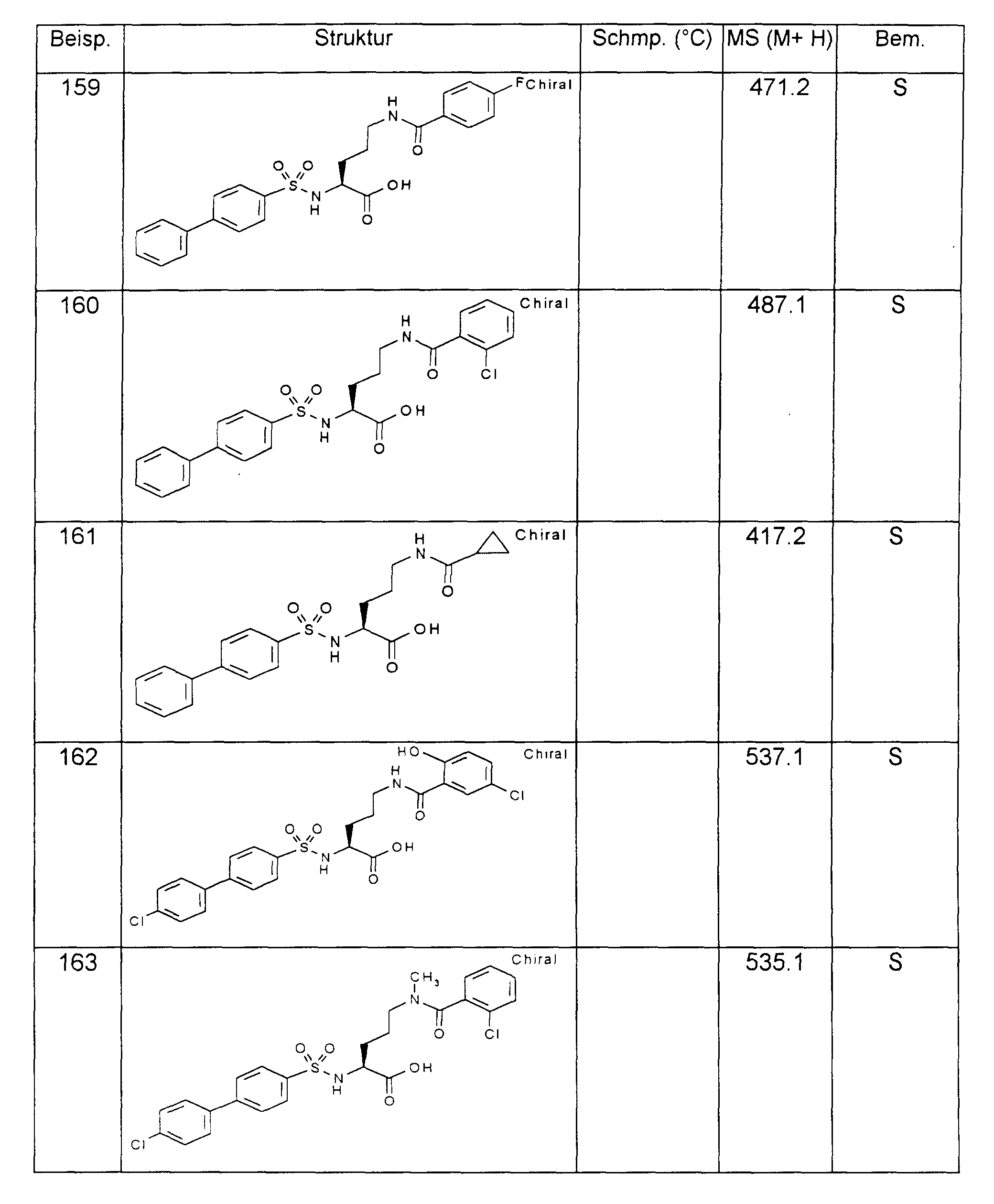

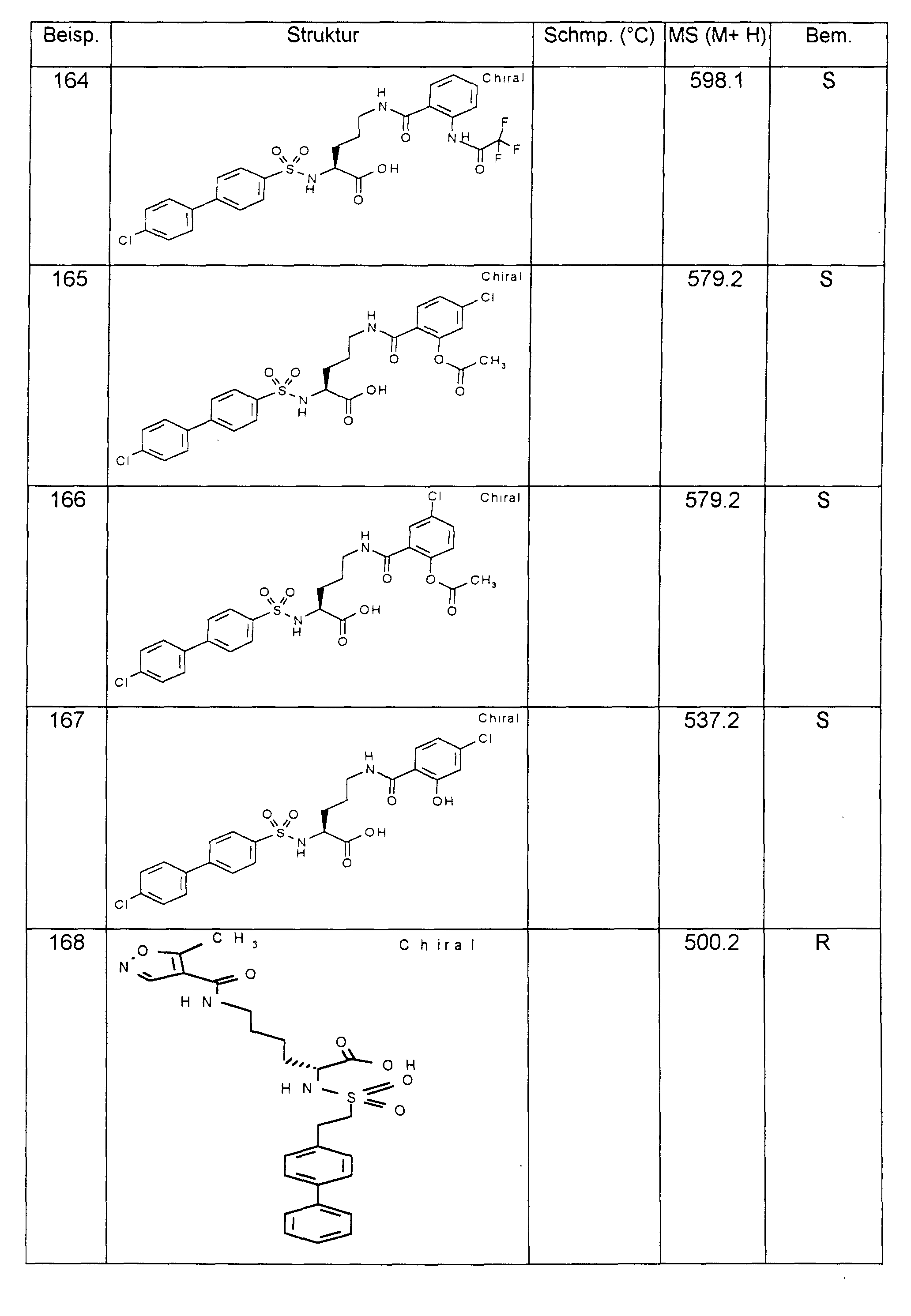

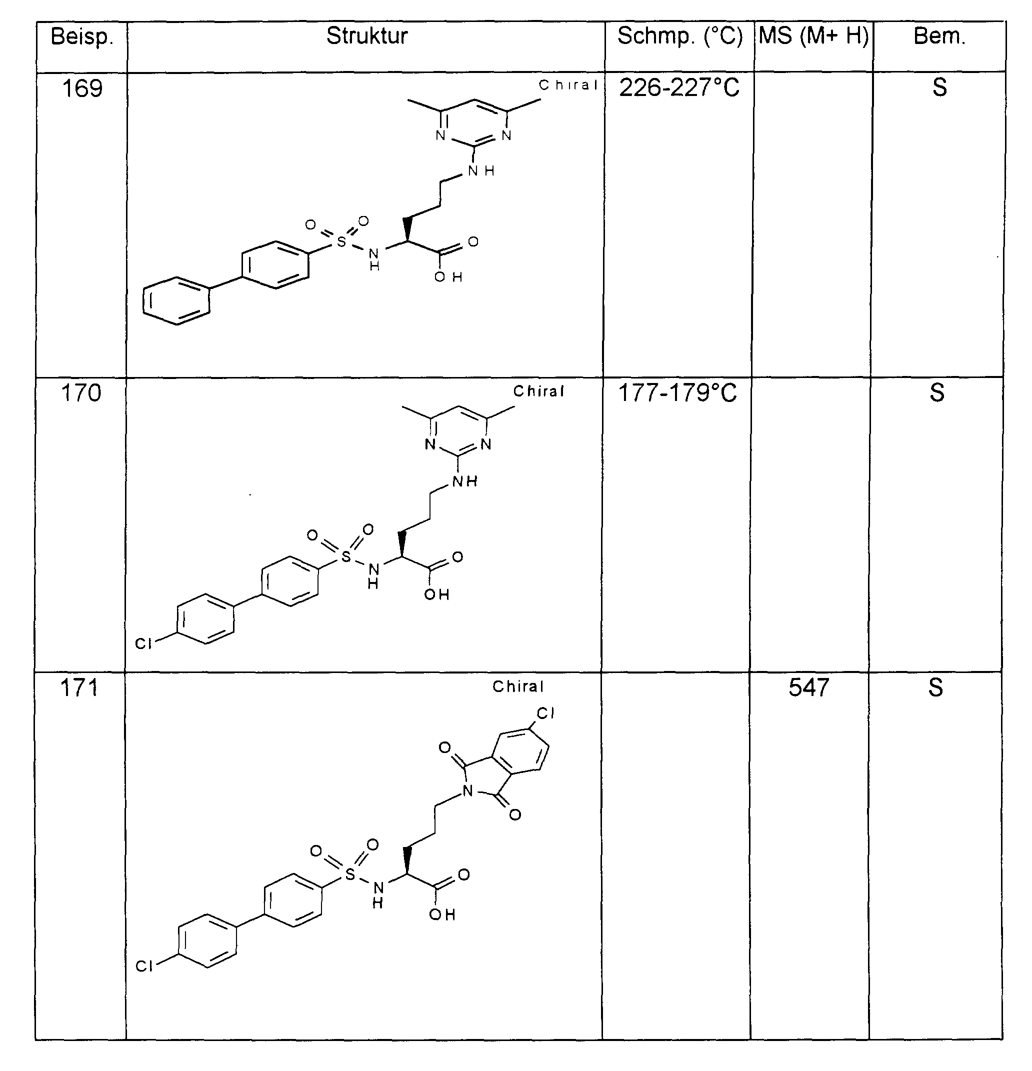

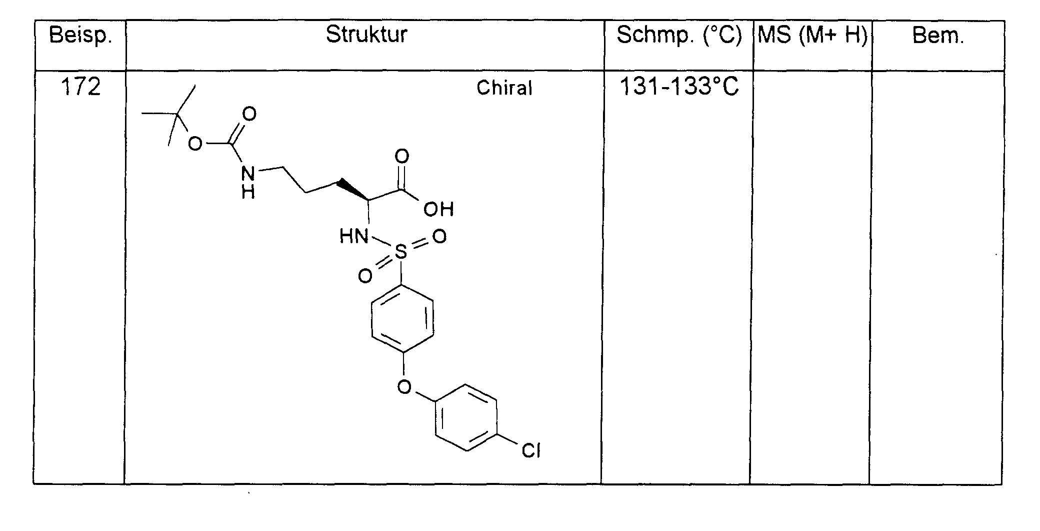

### Pharmakologische Beispiele

Darstellung und Bestimmung der enzymatischen Aktivität der katalytischen Domäne des humanen Stromelysins und der Neutrophilen-Kollagenase.

Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung werden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthält, für 15 Minuten inkubiert. Nach Zugabe von 10 µl einer 10%igen (v/v) wäßrigen Dimethylsulfoxid-Lösung, die 1 mmol/l des Substrates enthält, wird die Enzymreaktion fluoreszenzspektroskopisch verfolgt (328 nm (ex) / 393 nm(em)).
Die Enzymaktivität wird dargestellt als Extinktionszunahme/Minute. Die in Tabelle 2 aufgeführten IC₅₀-Werte werden als diejenige Inhibitorkonzentrationen ermittelt, die jeweils zu einer 50%igen Inhibierung des Enzyms führen.
Die Pufferlösung enthält 0,05% Brij (Sigma, Deisenhofen, Deutschland) sowie 0,1 mol/l Tris/HCl, 0,1 mol/l NaCI, 0,01 mol/l CaCl₂ und 0,1 mol/l Piperazin-N,N'-bis[2-ethan-sulfonsäure] (pH=6,5).
Die Enzymlösung enthält 5 µg/ml einer der nach Ye et al. dargestellten Enzymdomänen. Die Substratlösung enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

**Tabelle 2**

| ***Beispiel*** | ***IC50 MMP-3 [x 10***^{***-9***} ***Mol/l]*** | ***IC50 MMP-8 [x 10***^{***-***}^{***9***}***Mol/l]*** |
|---|---|---|
| 1 | 50 | 7 |
| 2 | 20 | 6 |
| 4 | 90 | 20 |
| 5 | 50 | 4 |
| 6 | 5 | 2 |
| 7 | 4 | 2 |
| 9 | 60 | 70 |
| 12 | 60 | 10 |
| 14 | 5 | 3 |
| 15 | 20 | 8 |
| 16 | 20 | 10 |
| 18 | 70 | 10 |
| 19 | 20 | 5 |
| 20 | 40 | 7 |
| 21 | 70 | 20 |
| 22 | 80 | 80 |
| 23 | 40 | 5 |
| 24 | 30 | 5 |
| 25 | 60 | 10 |
| 26 | 60 | 7 |
| 28 | 40 | 6 |
| 29 | 6 | 3 |
| 30 | 30 | 5 |
| 31 | 5 | 2 |
| 32 | 6 | 2 |
| 34 | 4 | 2 |
| 36 | 5 | 2 |
| 38 | 5 | 2 |
| 39 | 20 | 30 |
| 41 | 5 | 2 |
| 42 | 10 | 3 |
| 43 | 40 | 20 |
| 44 | 30 | 6 |
| 45 | 20 | 4 |
| 46 | 10 | 3 |
| 47 | 10 | 3 |
| 48 | 20 | 7 |
| 49 | 20 | 3 |
| 50 | 6 | 2 |
| 51 | 20 | 3 |
| 52 | 20 | 10 |
| 53 | 30 | 6 |
| 55 | 7 | 3 |
| 56 | 10 | 8 |
| 57 | 40 | 8 |
| 59 | 5 | 1 |
| 60 | 10 | 10 |
| 61 | 6 | 2 |
| 62 | 5 | 2 |
| 63 | 10 | 2 |
| 64 | 30 | 2 |
| 65 | 20 | 4 |
| 66 | 10 | 2 |
| 69 | 4 | 2 |
| 70 | 10 | 3 |
| 71 | 10 | 3 |
| 72 | 20 | 6 |
| 73 | 4 | 2 |
| 74 | 10 | 3 |
| 75 | 20 | 4 |
| 76 | 40 | 40 |
| 77 | 10 | 2 |
| 79 | 5 | 2 |
| 80 | 10 | 3 |
| 81 | 30 | 3 |
| 82 | 20 | 4 |
| 83 | 7 | 3 |
| 84 | 20 | 4 |
| 85 | 20 | 5 |
| 86 | 20 | 4 |
| 87 | 30 | 10 |
| 88 | 10 | 3 |
| 89 | 30 | 10 |
| 90 | 20 | 5 |
| 91 | 30 | 5 |
| 92 | 40 | 20 |
| 93 | 20 | 4 |
| 94 | 30 | 5 |
| 95 | 20 | 4 |
| 96 | 20 | 6 |
| 97 | 20 | 4 |
| 98 | 10 | 3 |
| 99 | 5 | 2 |
| 100 | 4 | 2 |
| 101 | 40 | 10 |
| 102 | 20 | 5 |
| 103 | 70 | 60 |
| 104 | 30 | 8 |
| 105 | 40 | 10 |
| 106 | 60 | 30 |
| 107 | 10 | 4 |
| 108 | 20 | 5 |
| 109 | 20 | 7 |
| 110 | 40 | 20 |
| 111 | 10 | 3 |
| 113 | 5 | 3 |
| 114 | 5 | 2 |
| 115 | 5 | 3 |
| 116 | 20 | 4 |
| 117 | 9 | 2 |
| 118 | 10 | 4 |
| 120 | 20 | 4 |
| 122 | 3 | 2 |
| 123 | 60 | 10 |
| 125 | 10 | 10 |
| 126 | 30 | 10 |
| 127 | 20 | 3 |
| 128 | 5 | 2 |
| 129 | 10 | 2 |
| 130 | 20 | 4 |
| 131 | 20 | 5 |
| 132 | 30 | 10 |
| 133 | 5 | 2 |
| 134 | 5 | 2 |
| 135 | 30 | 8 |
| 136 | 10 | 7 |
| 137 | 20 | 7 |
| 138 | 30 | 10 |
| 139 | 50 | 20 |
| 140 | 60 | 20 |
| 141 | 10 | 10 |
| 142 | 10 | 4 |
| 143 | 5 | 2 |
| 144 | 10 | 3 |
| 145 | 10 | 5 |
| 146 | 30 | 3 |
| 151 | 30 | 10 |
| 152 | 60 | 10 |
| 154 | 30 | 9 |
| 155 | 50 | 10 |
| 156 | 60 | 20 |
| 157 | 40 | 7 |
| 158 | 7 | 2 |
| 160 | 70 | 10 |
| 161 | 40 | 4 |
| 162 | 50 | 6 |
| 168 | 40 | 20 |
| 170 | 20 | 10 |
| 171 | 30 | 10 |

## Patentansprüche

1. Verbindung der Formel I 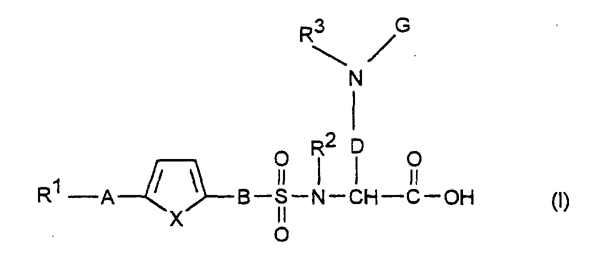 und/oder eine stereoisomere Form der Verbindung der Formel I und/oder ein physiologisch verträgliches Salz der Verbindung der Formel I, wobei
R¹ für
1. Phenyl,
2. Phenyl, welches ein- oder zweifach substituiert ist durch
2.1. (C₁-C₇)-Alkyl, gerade, cyclisch oder verzweigt,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁴-(R⁵)N-C(O)-,
2.14. R⁴-(R⁵)N-, oder
3. einen Heteroaromaten aus der nachfolgenden Gruppe 3.1. bis 3.16., der unsubstituiert oder wie unter 2.1 bis 2.14 beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12 Benzothiophen,
3.13. Benzimidazol,
3.14. Benzoxazol,
3.15. Benzothiazol oder
3.16 Benzotriazol steht,
R², R⁴ und R⁵ gleich oder verschieden sind und für
1. Wasserstoffatom,
2. (C₁-C₆)-Alkyl-,
3. HO-C(O)-(C₁-C₆)-Alkyl-,
4. Phenyl-(CH₂)ₒ-, worin Phenyl unsubstituiert oder ein- oder zweifach wie unter 2.1. bis 2.14. beschrieben substituiert ist und o die ganze Zahl Null, 1 oder 2 darstellt, oder
5. Picolyl stehen oder
6. R⁴ und R⁵ zusammen mit der ringständigen Aminogruppe einen 4-bis 7-gliedrigen Ring bilden, worin gegebenenfalls eines der Kohlenstoffatome durch -O-, -S- oder -NH- ersetzt ist,
R³ und G gleich oder verschieden sind und für
1. Wasserstoffatom,
2. (C₁-C₆)-Alkyl-, worin Alkyl gerade, verzweigt oder cyclisch ist,
3. (C₂-C₆)-Alkenyl-,
4. Phenyl-(CH₂)ₘ-, worin Phenyl unsubstituiert oder ein- oder zweifach wie unter 2.1. bis 2.14. beschrieben substituiert ist und m die ganze Zahl Null, 1,2 oder 3 ist,
5. Heteroaryl-(CH₂)ₘ-, worin Heteroaryl wie unter 3.1. bis 3.16. definiert und/oder wie unter 2.1 bis 2.14 beschrieben substituiert ist und m die ganze Zahl Null, 1, 2 oder 3 darstellt,
6. R⁶-C(O)-, worin
R⁶ für
6.1 (C₁-C₆)-Alkyl-, worin Alkyl unsubstituiert oder wie unter 2.1. bis 2.14. beschrieben oder durch (C₃-C₆)-Cycloalkyl substituiert ist,
6.2 (C₃-C₆)-Cycloalkyl, worin Cycloalkyl unsubstituiert oder wie unter 2.1. bis 2.14. beschrieben substituiert ist,
6.3 (C₂-C₆)-Alkenyl-, worin Alkenyl unsubstituiert oder ein- bis dreifach substituiert ist durch
6.3.1 Phenyl, worin Phenyl unsubstiituiert oder ein- bis dreifach wie unter 2.1. bis 2.14. beschrieben substituiert ist
6.3.2 Heteroaryl -, worin Heteroaryl wie unter 3.1. bis 3.16. definiert und unsubstituiuert oder ein- bis dreifach wie unter 2.1. bis 2.14. beschrieben substituiert ist oder
6.3.3 die unter 2.1. bis 2.14. beschriebenen Reste,
6.4 Phenyl-(CH₂)ₘ-, worin Phenyl unsubstituiert oder ein-bis dreifach wie unter 2.1. bis 2.14. beschrieben, durch -O-CF₃, -SO₂-NH₂, -NH-C(O)-CF₃ oder durch Benzyl substituiert ist und gegebenenfalls ein Wasserstoffatom des -(CH₂)-Restes durch den Rest -COOH substituiert ist und m die ganze Zahl Null, 1, 2 oder 3 bedeutet,
6.5 Naphthyl,
6.6 Adamantyl oder
6.7 Heteroaryl-(CH₂)ₘ-, worin Heteroaryl wie unter 3.1. bis 3.16. definiert und/oder wie unter 2.1. bis 2.14. beschrieben substituiert ist und m die ganze Zahl Null, 1, 2 oder 3 darstellt, steht,
7. R⁶-O-C(O)-, worin R⁶ wie oben genannt definiert ist,
8. R⁶-CH(NH₂)-C(O)-, worin R⁶ wie oben genannt definiert ist,
9. R⁸-N(R⁷)-C(O)-, worin
R⁸ für
9.1 Wasserstoffatom
9.2 (C₁-C₆)-Alkyl-,
9.3 Phenyl-(CH₂)ₘ, worin Phenyl unsubstituiert oder ein- oder zweifach wie unter 2.1. bis 2.14. beschrieben substituiert ist und m die ganze Zahl Null, 1,2 oder 3 darstellt, oder
9.4 Heteroaryl-(CH₂)ₘ, worin Heteroaryl wie unter 3.1. bis 3.16. definiert und/oder wie unter 2.1 bis 2.14 beschrieben substituiert ist und m die ganze Zahl Null, 1, 2 oder 3 darstellt, steht und worin
R⁷ Wasserstoffatom oder (C₁-C₆)-Alkyl bedeutet oder worin R⁷ und R⁸ zusammen mit dem Stickstoffatom an das sie gebunden sind einen 4- bis 7-gliedrigen Ring bilden und der Ring unsubstituiert ist oder ein Kohlenstoffatom im Ring durch -O-, -S- oder -NH- ersetzt ist,
10. R⁶-SO₂-, worin R⁶ wie oben genannt definiert ist,
11. R⁶-SO₂-N(R⁷)-C(O)-, worin R⁶ und R⁷ wie oben genannt definiert sind,
12. R⁶-NH-C(=NR⁷)-, worin R⁶ und R⁷ wie oben genannt definiert sind oder
12.1 (C₁-C₆)-Alkyl-C(O)-,
12.2 -NO₂ oder
12.3 -SO₂-(CH₂)ₘ-Phenyl, worin Phenyl unsubstituiert oder ein- oder zweifach wie unter 2.1. bis 2.14. beschrieben substituiert ist und m die ganze Zahl Null, 1,2 oder 3 ist, darstellen,
13. 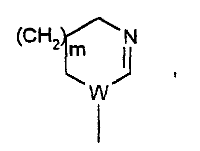 worin m die ganze Zahl Null, 1, 2 oder 3 bedeutet und W für ein Stickstoff-, Sauerstoff- oder Schwefelatom steht, oder
R³ und G zusammen mit dem Stickstoffatom an das sie gebunden sind einen Ring der Teilformel lla bis llp bilden 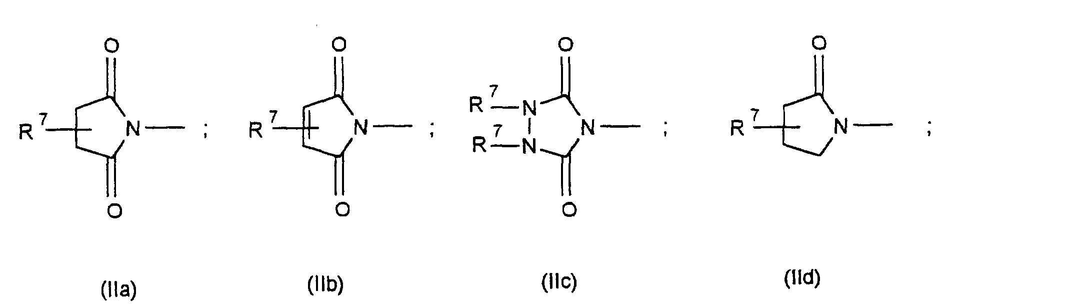 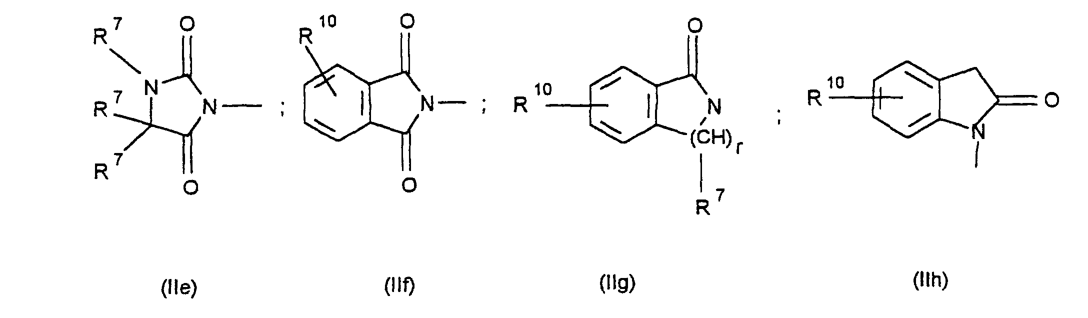 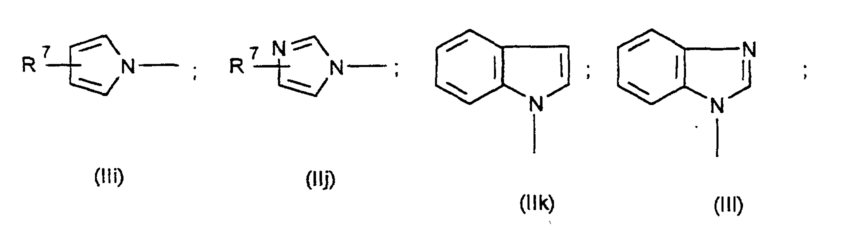 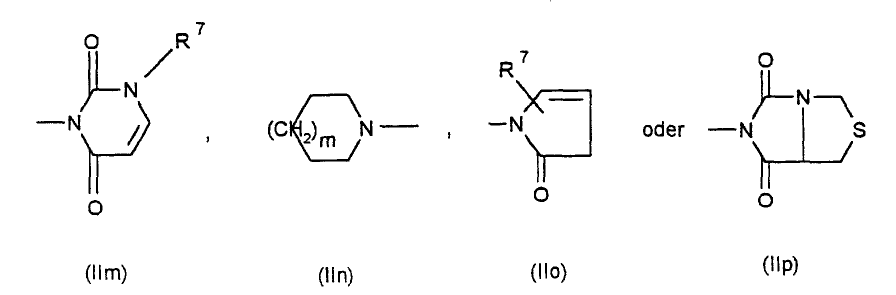 wobei r die ganze Zahl 1 oder 2 bedeutet, R¹⁰ einen Rest wie unter 2.1. bis 2.14. beschrieben bedeutet, und R⁷ und m die oben genannte Bedeutung haben und gegebenenfalls bei der Teilformel IIg ein Kohlenstoffatom im Ring durch Sauerstoff-, Schwefel-, SO₂ oder unsubstituiertes oder mit R2 substituiertes Stickstoffatom ersetzt ist,
A für
a) eine kovalente Bindung,
b) -O-,
c) -CH=CH- oder
d) -C≡C- steht,
B für
a) -(CH₂)ₘ-, worin m die obengenannte Bedeutung hat,
b) -O-(CH₂)_{q}, worin q die ganze Zahl 1, 2, 3, 4 oder 5 bedeutet, oder
c) -CH=CH- steht,
D -(CH₂)ₛ bedeutet, worin s für die ganze Zahl 1, 2, 3, 4, 5 oder 6 steht und gegebenenfalls einer der Ketten-C-Atome durch ein gegebenenfalls substituiertes -NH-, -O- oder -S- Atom ersetzt ist, und
X für -CH=CH-, Sauerstoffatom oder Schwefelatom steht.

2. Verbindung der Formel I gemäß Anspruch 1, **dadurch gekennzeichnet, daß**
R¹ für
1. Phenyl oder
2. Phenyl, welches einfach substituiert ist durch
2.1. Halogen, insbesondere Chlor oder Fluor oder
2.2. R⁴-(R⁵)N-, wobei R⁴ und R⁵ gleich oder verschieden sind und für
2.2.1. (C₁-C₃)-Alkyl stehen oder
2.2.2. R⁴ und R⁵ zusammen mit der ringständigen Aminogruppe einen 5-6-gliedrigen Ring bilden, wobei gegebenenfalls eines der C-Atome durch -O- oder -NH- ersetzt ist, steht,
R² für Wasserstoffatom steht,
G und R³ verschieden sind, wobei
G für Wasserstoffatom oder (C₁-C₄)-Alkyl steht und
R³ für
1. Phenyl-(CH₂)ₘ steht, worin Phenyl unsubstituiert ist oder ein- oder zweifach wie unter 2.1 bis 2.14 beschrieben substituiert ist und m die ganze Zahl 1 ist,
2. für Heteroaryl-(CH₂)ₙ steht, worin Heteroaryl wie unter 3.10 definiert ist und unsubstituiert ist oder wie unter 2.1 bis 2.14 beschrieben substituiert ist und n für Null steht,
3. für R⁶-C(O)- steht, worin
R⁶ für
3.1 (C₁-C₆)-Alkyl-, worin Alkyl gerade, verzweigt oder cyclisch ist,
3.2 Phenyl-(CH₂)ᵣ- worin Phenyl unsubstituiert oder ein- oder zweifach wie unter 2.1 bis 2.14 beschrieben substituiert ist und gegebenenfalls ein Wasserstoffatom des -(CH₂)-Restes durch den Rest -COOH ersetzt ist und r Null 1, 2 oder 3 bedeutet, oder
3.3 Heteroaryl-(CH₂)ₒ-, worin Heteroaryl wie unter 3.1 bis 3.15 definiert und unsubstituiert oder wie unter 2.1 bis 2.14 beschrieben substituiert ist und o Null 1, 2, oder 3 bedeutet, oder
4. für R⁸-N(R⁷)-C(O)- steht, worin
R⁸ und R⁷ zusammen mit dem Stickstoffatom an das sie gebunden sind einen 5- oder 6-gliedrigen Ring bilden und der Ring unsubstituiert oder ein Ring-C-atom durch ein Sauerstoffatom ersetzt ist, oder
R³ und G zusammen mit dem Stickstoffatom, an das sie gebunden sind, einen Ring der Teilformel IIg bilden, worin r 1 ist,
A für eine kovalente Bindung steht,
B für -(CH₂)ₚ- steht und p Null bedeutet,
D -(CH₂)_{q}- bedeutet und q für eine ganze Zahl 2, 3 oder 4 steht, und
X für -CH=CH- steht.

3. Verbindung der Formel X 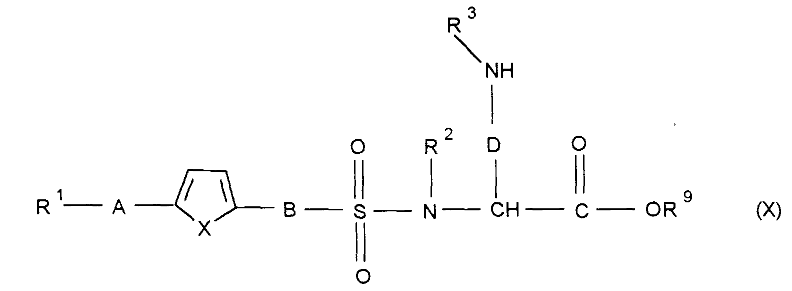 und/oder eine stereoisomere Form der Verbindung der Formel X und/oder ein physiologisch verträgliches Salz der Verbindung der Formel X, wobei R¹, A, X, B, R², R³ und D die in der Verbindung der Formel I gemäß Anspruch 1 genannte Bedeutung haben und R⁹ Wasserstoffatom, (C₁-C₆)-Alkyl, Phenyl, Succinimidyl, Benzotriazolyl oder Benzyl bedeutet.

4. Verfahren zur Herstellung der Verbindung der Formel I gemäß einem oder mehreren der Ansprüche 1 bis 3, **dadurch gekennzeichnet, daß** man
a) eine Diaminocarbonsäure der Formel III 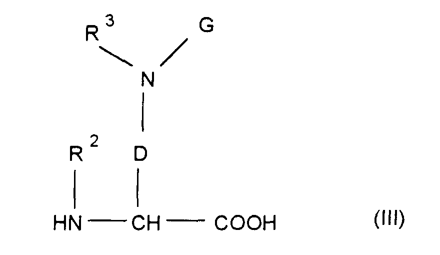 worin R², R³, D und G wie in Formel I definiert sind, mit einem Sulfonsäurederivat der Formel IV 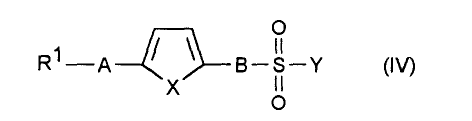 worin R¹, A und B wie in Formel I definiert sind und Y ein Halogenatom, Imidazolyl oder -OR⁹ bedeutet, worin R⁹ Wasserstoffatom, (C₁-C₆)-Alkyl, Phenyl, Succinimidyl, Benzotriazolyl oder Benzyl, gegebenenfalls substituiert darstellt,
in Gegenwart einer Base oder gegebenenfalls eines wasserentziehenden Mittels zu einer Verbindung der Formel I umsetzt, oder
b) einen Diaminocarbonsäureester der Formel V 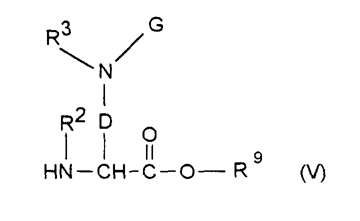 worin R², R³, D, G und R⁹ die obengenannte Bedeutung haben, mit einem Sulfonsäurederivat der Formel IV unter den obengenannten Bedingungen zu einer Verbindung der Formel VI 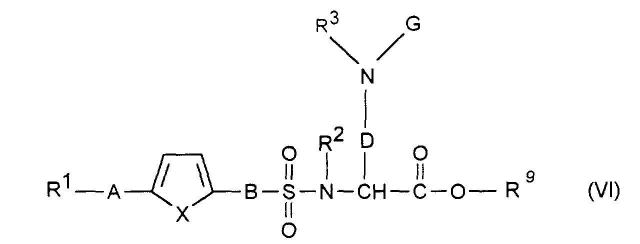 umsetzt, und die Verbindung der Formel VI unter Abspaltung des Restes R⁹, bevorzugt in Gegenwart einer Base oder Säure in eine Verbindung der Formel I umwandelt, oder
c) die geschützten Diaminocarbonsäuren der Formel VII, 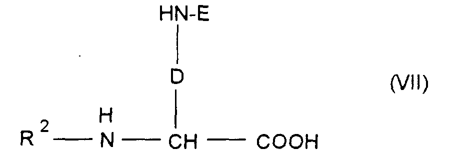 worin R² und D die oben genannte Bedeutungen haben und E für eine Schutzgruppe der Aminofunktion steht, mit einem Sulfonsäurederivat der Formel IV zu einer Verbindung der Formel VIII 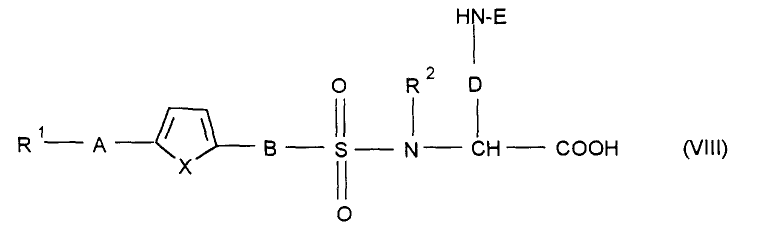 umsetzt, anschließend die Verbindung der Formel VIII unter Abspaltung der Schutzgruppe E mit Hilfe geeigneter Spaltreagenzien in eine Verbindung der Formel I überführt, 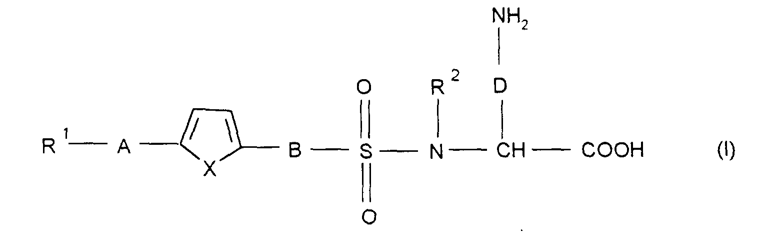 worin R¹, R², A, B, D und X die oben genannte Bedeutung haben und R³ und G ein Wasserstoffatom bedeuten, und
diese Verbindung der Formel I gegebenenfalls mit Hilfe von R³-Y, worin R³ und Y die oben angegebenen Bedeutungen haben, in Gegenwart einer Base zu einer Verbindung der Formel I, 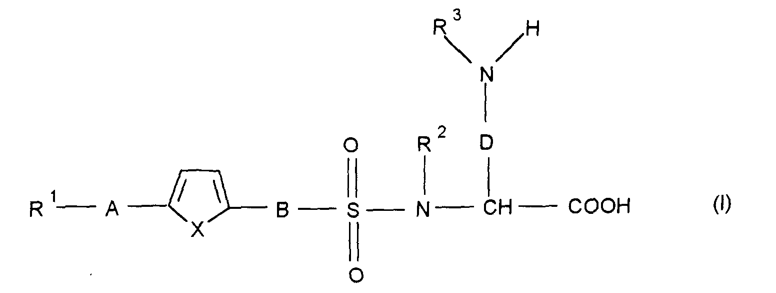 worin R¹, R², R³, A, B und X die oben genannte Bedeutungen haben und G ein Wasserstoffatom darstellt, umsetzt, oder
d) für den Fall, daß als Ausgangsverbindungen geschützte Diaminosäureester der Formel IX, 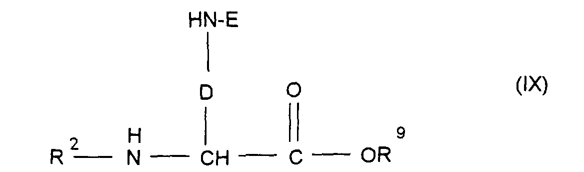 worin R², R⁹, D und E die oben genannte Bedeutung haben, in gleicher Weise wie in der Verfahrensvariante c) beschrieben in die Ester der Formel X umwandelt, 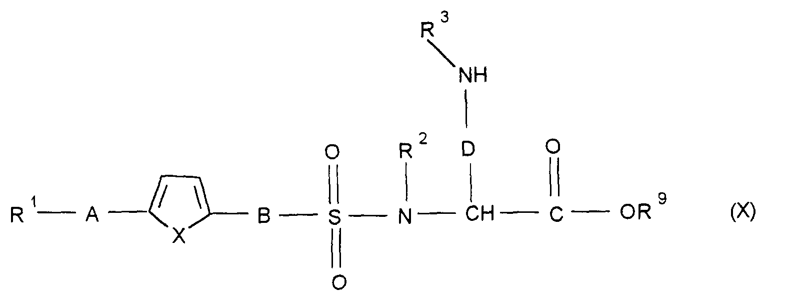 die gegebenenfalls nach Verfahrensvariante b) in die entsprechenden Verbindungen der Formel I umgewandelt werden, oder
e) eine Diaminocarbonsäure der Formel XI, 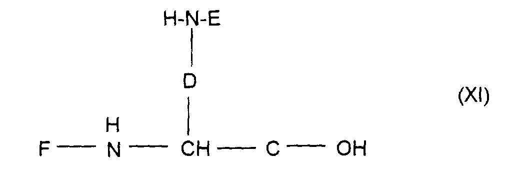
worin D wie in Formel I definiert ist und E und F untereinander verschiedene N-Aminoschutzgruppen darstellen, mit ihrer Carboxylgruppe über eine Zwischenkette L an ein polymeres Harz der allgemeinen Formel PS ankoppelt, wobei eine Verbindung der Formel XII 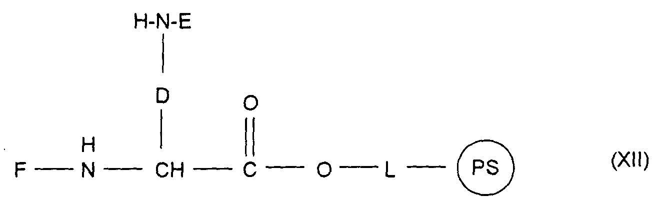 entsteht, die sich nach selektiver Abspaltung der Schutzgruppe F mit einem Sulfonsäurederivat der Formel IV 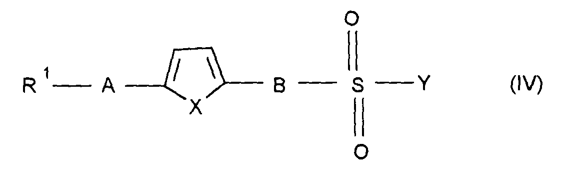 wobei R¹, A, B und Y die oben genannten Bedeutungen haben, in Gegenwart einer Base oder gegebenenfalls eines wasserentziehenden Mittels zu einer Verbindung der Formel XIII 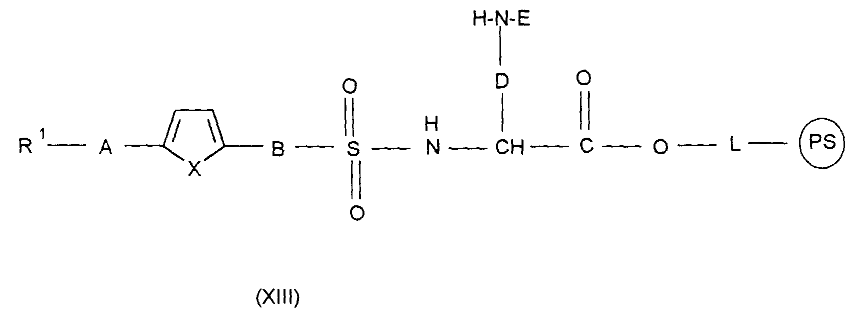 umsetzt, und die Verbindung der Formel XIII nach Abspaltung der Schutzgruppe E mit einem Carbonsäurederivat der Forme XIV
R⁶-C(O)-Y (XIV)
worin R⁶ und Y die oben genannte Bedeutung haben, in Gegenwart einer Base oder eines wasserentziehenden Mittels zu einer Verbindung der Formel XV 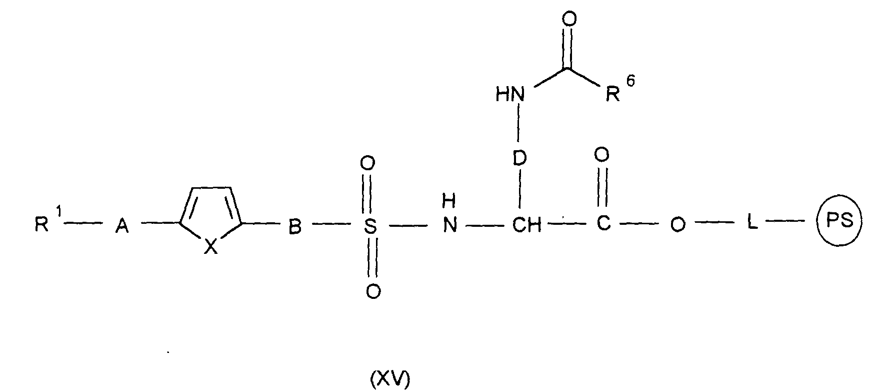 umsetzt, und diese nach Abspaltung vom Trägermaterial in eine Verbindung der Formel I, 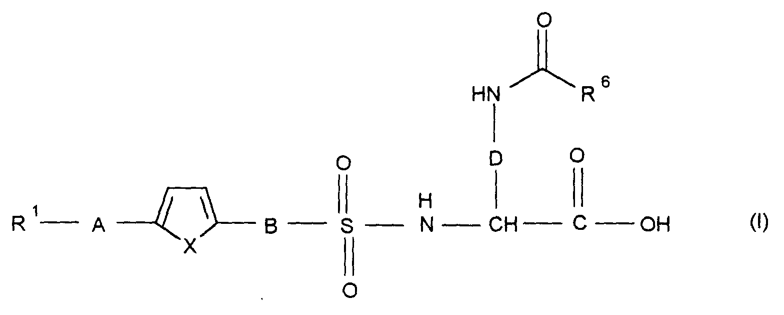 worin R¹, R⁶, A, B, D und X die oben genannte Bedeutung haben, überführt.

5. Arzneimittel, **gekennzeichnet durch** einen wirksamen Gehalt an mindestens einer Verbindung der Formel I gemäß einem oder mehreren der Ansprüche 1 bis 3, zusammen mit einem pharmazeutisch geeigneten und physiologisch verträglichen Trägerstoff, Zusatzstoff und/oder anderen Wirk- und Hilfsstoffen.

6. Verwendung von mindestens einer Verbindung der Formel I gemäß einem oder mehreren der Ansprüche 1 bis 3, zur Herstellung von Arzneimitteln zur Prophylaxe und Therapie von Erkrankungen, an deren Verlauf eine verstärkte Aktivität von Matrix-abbauenden Metalloproteinasen beteiligt ist.

7. Verwendung gemäß Anspruch 6, für die Behandlung von degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen, Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels, der Ulceration, Atherosklerose und Stenosen, aber auch zur Behandlung von Entzündungen, Krebserkrankungen, Tumormetastasenbildung, Kachexie, Anorexie und septischem Schock.

8. Verfahren zur Herstellung eines Arzneimittels , **dadurch gekennzeichnet, daß** man mindestens eine Verbindung der Formel I gemäß einem oder mehreren der Ansprüche 1 bis 3 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 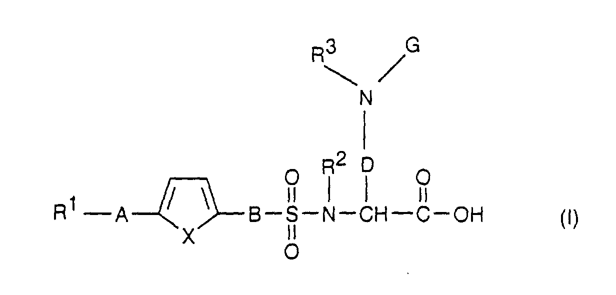 and/or a stereoisomeric form of the compound of the formula I and/or a physiologically tolerable salt of the compound of the formula I, where
R¹ is
1. phenyl,
2. phenyl, which is mono- or disubstituted by
2.1. (C₁-C₇)-alkyl, which is linear, cyclic or branched,
2.2. -OH,
2.3. (C₁-C₆)-alkyl-C(O)-O-,
2.4. (C₁-C₆)-alkyl-O-,
2.5. (C₁-C₆)-alkyl-O-(C₁-C₄)-alkyl-O-,
2.6. halogen,
2.7. -CF₃,
2.8. -CN,
2.9. -NO₂,
2.10. HO-C(O)-,
2.11. (C₁-C₆)-alkyl-O-C(O)-,
2.12. methylenedioxo,
2.13. R⁴-(R⁵)N-C(O)-,
2.14. R⁴-(R⁵)N-, or
3. a heteroaromatic from the following group 3.1. to 3.16., which is unsubstituted or substituted as described under 2.1. to 2.14.,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12. benzothiophene,
3.13. benzimidazole,
3.14. benzoxazole,
3.15. benzothiazole or
3.16. benzotriazole,
R², R⁴ and R⁵ are identical or different and are
1. a hydrogen atom,
2. (C₁-C₆)-alkyl-,
3. HO-C(O)-(C₁-C₆)alkyl-,
4. phenyl-(CH₂)ₒ-, in which phenyl is unsubstituted or mono- or disubstituted as described under 2.1. to 2.14. and o is the integer zero, 1 or 2, or
5. picolyl or
6. R⁴ and R⁵ together with the ring amino group form a 4-to 7-membered ring, in which one of the carbon atoms is optionally replaced by -O-, -S- or -NH-,
R³ and G are identical or different and are
1. a hydrogen atom,
2. (C₁-C₆)-alkyl-, in which alkyl is linear, branched or cyclic,
3. (C₂-C₆)-alkenyl-,
4. phenyl-(CH₂)ₘ-, in which phenyl is unsubstituted or mono- or disubstituted as described under 2.1. to 2.14. and m is the integer zero, 1, 2 or 3,
5. heteroaryl-(CH₂)ₘ-, in which heteroaryl is substituted as defined under 3.1. to 3.16. and/or as described under 2.1 to 2.14 and m is the integer zero, 1, 2 or 3,
6. R⁶-C(O)-, in which
R⁶ is
6.1 (C₁ -C₆)-alkyl-, in which alkyl is unsubstituted or substituted as described under 2.1. to 2.14. or substituted by (C₃-C₆)-cycloalkyl,
6.2 (C₃-C₆)-cycloalkyl, in which cycloalkyl is unsubstituted or substituted as described under 2.1.to 2.14.,
6.3 (C₂-C₆)-alkenyl-, in which alkenyl is unsubstituted or mono- to trisubstituted by
6.3.1 phenyl, in which phenyl is unsubstituted or mono- to trisubstituted as described under 2.1. to 2.14.
6.3.2 heteroaryl , in which heteroaryl is as defined under 3.1. to 3.16. and is unsubstituted or mono- to trisubstituted as described under 2.1. to 2.14. or
6.3.3 the radicals described under 2.1. to 2.14,
6.4 phenyl-(CH₂)ₘ-, in which phenyl is unsubstituted or mono- to trisubstituted as described under 2.1. to 2.14. by -O-CF₃, -SO₂-NH₂ , -NH-C(O)-CF₃ or by benzyl and a hydrogen atom of the -(CH₂)- radical is optionally substituted by the radical -COOH and m is the integer zero, 1, 2 or 3,
6.5 naphthyl,
6.6 adamantyl or
6.7 heteroaryl-(CH₂)ₘ-, in which heteroaryl is as defined under 3.1. to 3.16. and/or is substituted as described under 2.1. to 2.14. and m is the integer zero, 1, 2 or 3,
7. R⁶-O-C(O)-, in which R⁶ is defined as mentioned above,
8. R⁶-CH(NH₂)-C(O)-, in which R⁶ is defined as mentioned above,
9. R⁸-N(R⁷)-C(O)-, in which
R⁸ is
9.1 a hydrogen atom
9.2 (C₁-C₆)-alkyl-,
9.3 phenyl-(CH₂)ₘ, in which phenyl is unsubstituted or mono- or disubstituted as described under 2.1. to 2.14. and m is the integer zero, 1, 2 or 3, or
9.4 heteroaryl-(CH₂)ₘ, in which heteroaryl is as defined under 3.1. to 3.16. and/or is substituted as described under 2.1 to 2.14 and m is the integer zero, 1, 2 or 3, and in which
R⁷ is a hydrogen atom or (C₁-C₆)-alkyl or in which R⁷ and R⁸ together with the nitrogen atom to which they are bonded form a 4- to 7-membered ring and the ring is unsubstituted or a carbon atom in the ring is replaced by -O-, -S- or -NH-,
10. R⁶-SO₂-, in which R⁶ is defined as mentioned above,
11. R⁶-SO₂-N(R⁷)-C(O)-, in which R⁶ and R⁷ are defined as mentioned above,
12. R⁶-NH-C(=NR⁷)-, in which R⁶ and R⁷ are defined as mentioned above or
12.1 (C₁-C₆)-alkyl-C(O)-,
12.2 -NO₂ or
12.3 -SO₂-(CH₂)ₘ-phenyl, in which phenyl is unsubstituted or mono- or disubstituted as described under 2.1. to 2.14. and m is the integer zero, 1, 2 or 3,
13. 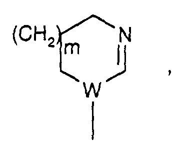 in which m is the integer zero, 1, 2 or 3 and W is a nitrogen, oxygen or sulfur atom, or
R³ and G together with the nitrogen atom to which they are bonded form a ring of the subformula IIa to IIp 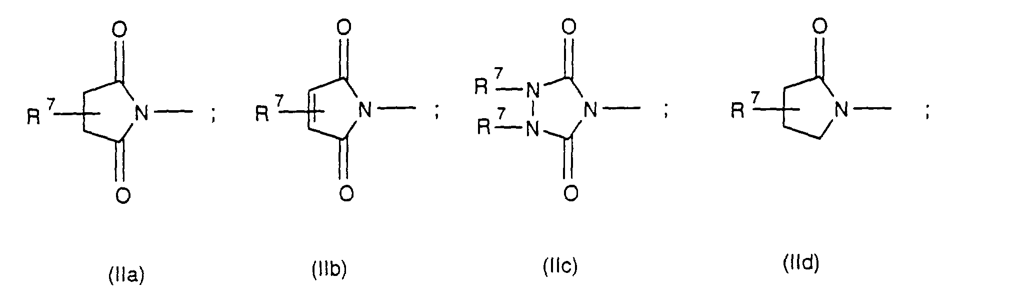 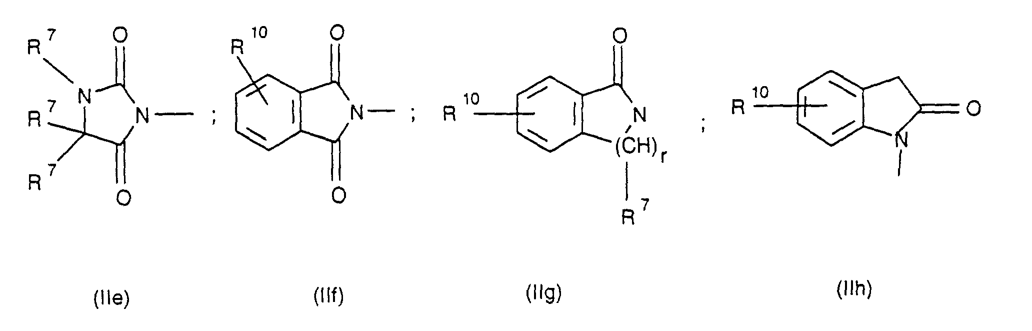 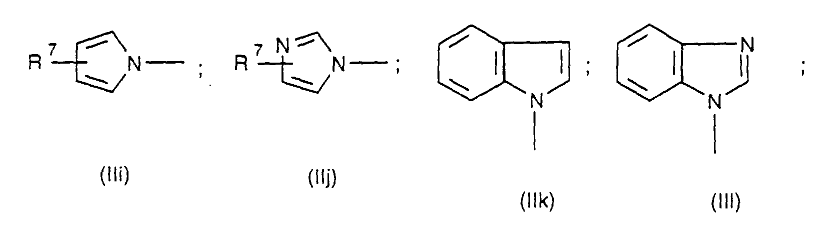 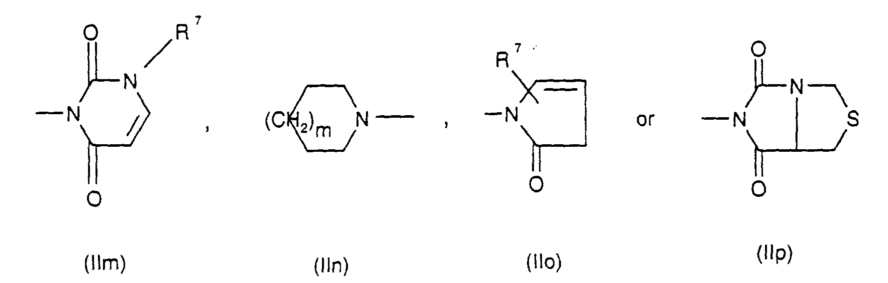 where r is the integer 1 or 2, R¹⁰ is a radical as described under 2.1. to 2.14. and R⁷ and m have the abovementioned meaning and in the subformula IIg a carbon atom in the ring is optionally replaced by oxygen, sulfur, SO₂ or a nitrogen atom which is unsubstituted or substituted by R²,
A is
a) a covalent bond,
b) -O-,
c) -CH=CH- or
d) -C≡C-,
B is
a) -(CH₂)ₘ-, in which m has the abovementioned meaning,
b) -O-(CH₂)_{q}, in which q is the integer 1, 2, 3, 4 or 5, or
c) -CH=CH-,
D is -(CH₂)ₛ- in which s is the integer 1, 2, 3, 4, 5 or 6 and one of the chain carbon atoms is optionally replaced by an optionally substituted -N=, -O- or -S- atom, and
X is -CH=CH-, an oxygen atom or a sulfur atom.

2. A compound of the formula I as claimed in claim 1, wherein
R¹ is
1. phenyl or
2. phenyl, which is monosubstituted by
2.1. halogen, in particular chlorine or fluorine or
2.2. R⁴-(R⁵)N-, in which R⁴ and R⁵ are identical or different and are
2.2.1. (C₁ -C₃)-alkyl or
2.2.2. R⁴ and R⁵ together with the ring amino group form a 5-6-membered ring, where one of the carbon atoms is optionally replaced by -O- or -NH-,
R² is a hydrogen atom,
G and R³ are different where
G is a hydrogen atom or (C₁-C₄)-alkyl and
R³ is
1. phenyl-(CH₂)ₘ in which phenyl is unsubstituted or
mono- or disubstituted as described under 2.1 to 2.14 and m is the integer 1,
2. is heteroaryl-(CH₂)n, in which heteroaryl is as defined under 3.10 and is unsubstituted or substituted as described under 2.1 to 2.14 and n is zero,
3. is R⁶ -C(O)-, in which
R⁶ is
3.1 (C₁-C₆)-alkyl-, in which alkyl is linear, branched or cyclic,
3.2 phenyl-(CH₂)ᵣ- in which phenyl is unsubstituted or mono- or disubstituted as described under 2.1 to 2.14 and a hydrogen atom of the -(CH₂)- radical is optionally replaced by the radical -COOH and r is zero, 1, 2 or 3, or
3.3 heteroaryl-(CH₂)ₒ-, in which heteroaryl is as defined under 3.1 to 3.15 and is unsubstituted or substituted as described under 2.1 to 2.14 and o is zero, 1, 2, or 3, or
4. is R⁸-N(R⁷)-C(O)-, in which
R⁸ and R⁷ together with the nitrogen atom to which they are bonded form a 5- or 6-membered ring and the ring is unsubstituted or a ring carbon atom is replaced by an oxygen atom, or
R³ and G together with the nitrogen atom to which they are bonded form a ring of the subformula IIg, in which r is 1,
A is a covalent bond,
B is -(CH₂)ₚ- and p is zero,
D -(CH₂)_{q}- and q is an integer 2, 3 or 4, and
X is -CH=CH-.

3. A compound of the formula X 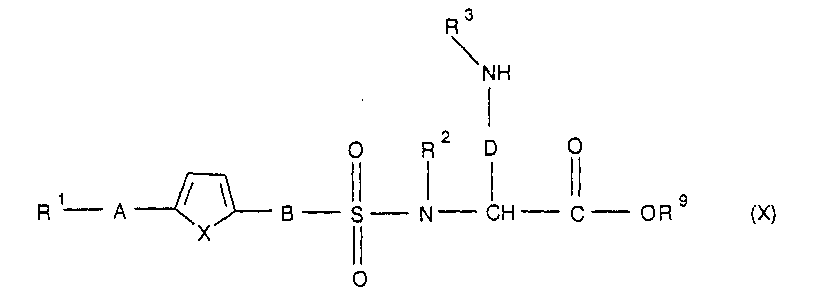 and/or a stereoisomeric form of the compound of the formula X and/or a physiologically tolerable salt of the compound of the formula X, where R¹, A, X, B, R², R³ and D have the meaning mentioned in the compound of the formula I as claimed in claim 1 and R⁹ is a hydrogen atom, (C₁-C₆)-alkyl, phenyl, succinimidyl, benzotriazolyl or benzyl.

4. A process for the preparation of the compound of the formula I as claimed in one or more of claims 1 to 3, which comprises
a) reacting a diaminocarboxylic acid of the formula III 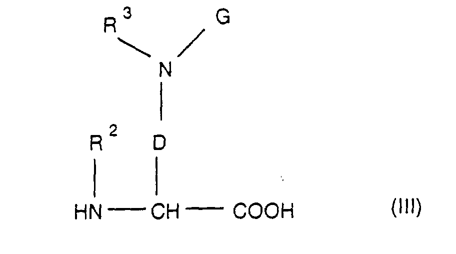 in which R², R³, D and G are as defined in formula I, with a sulfonic acid derivative of the formula IV 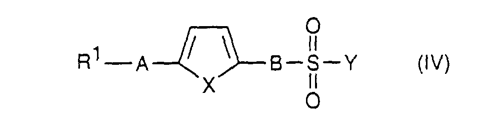 in which R¹, A and B are as defined in formula I and Y is a halogen atom, imidazolyl or -OR⁹, in which R⁹ is a hydrogen atom, (C₁-C₆)-alkyl, phenyl, succinimidyl, benzotriazolyl or benzyl, optionallly substituted, in the presence of a base or if appropriate of a dehydrating agent, to give a compound of the formula I, or
b) reacting a diaminocarboxylic acid ester of the formula V 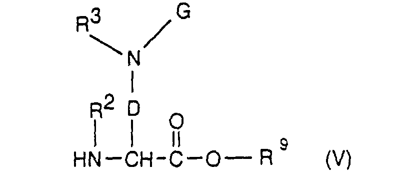 in which R², R³, D, G and R⁹ have the abovementioned meaning, with a sulfonic acid derivative of the formula IV under the abovementioned conditions to give a compound of the formula VI 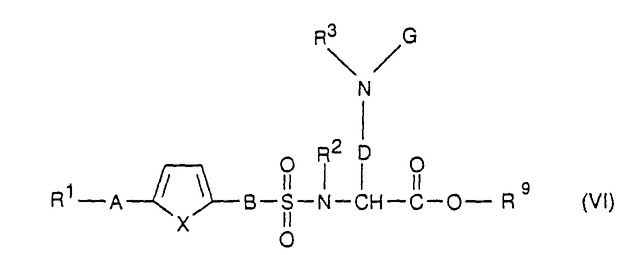 and converting the compound of the formula VI into a compound of the formula I with removal of the radical R⁹, preferably in the presence of a base or acid, or
c) reacting the protected diaminocarboxylic acids of the formula VII, 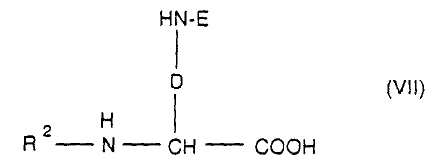 in which R² and D have the abovementioned meanings and E is a protective group of the amino function, with a sulfonic acid derivative of formula IV to give a compound of the formula VIII 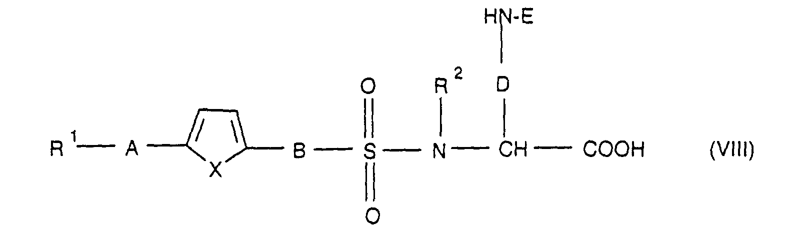 then converting the compound of the formula VIII, with removal of the protective group E with the aid of suitable cleavage agents, into a compound of the formula I, 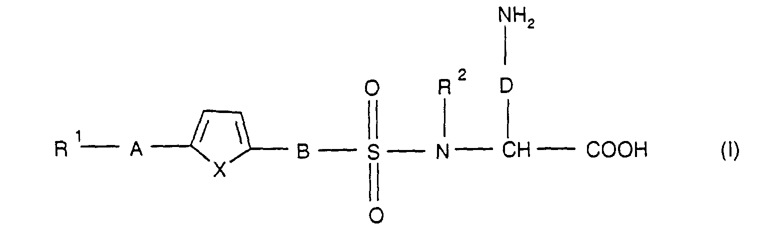 in which R¹, R², A, B, D and X have the abovementioned meaning and R³ and G are a hydrogen atom, and reacting this compound of the formula I if appropriate with the aid of R³-Y, in which R³ and Y have the meanings indicated above, in the presence of a base to give a compound of the formula I, 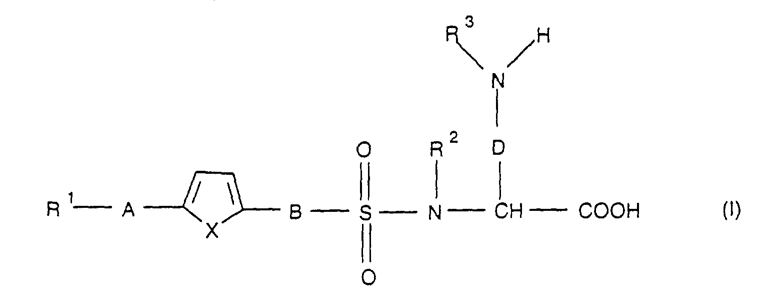 in which R¹, R², R³, A, B and X have the abovementioned meanings and G is a hydrogen atom,
d) as starting compounds, converting protected diamino acid esters of the formula IX, 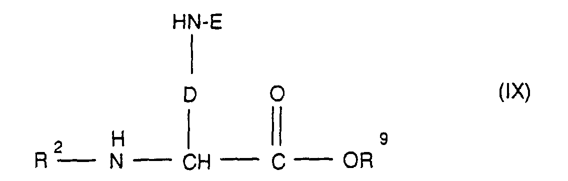 in which R², R⁹, D and E have the abovementioned meaning, in the same manner as described in process variant c), into the esters of the formula X, 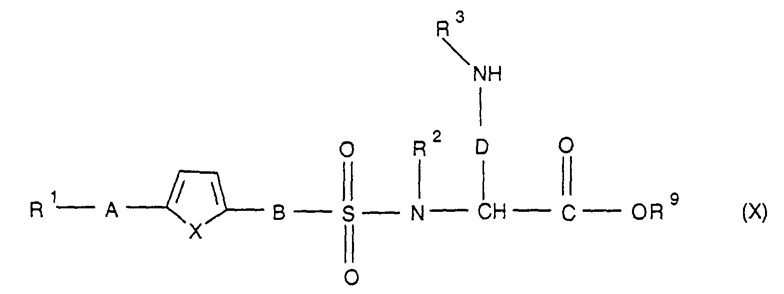 which are optionally converted into the corresponding compounds of the formula I according to process variant b), or
e) coupling a diaminocarboxylic acid of the formula XI, 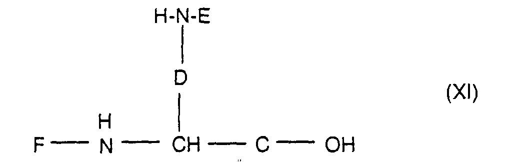 in which D is defined as in formula I and E and F are N-amino protective groups which are different from one another, by its carboxyl group via an intermediate chain L to a polymeric resin of the formula PS, a compound of the formula XII 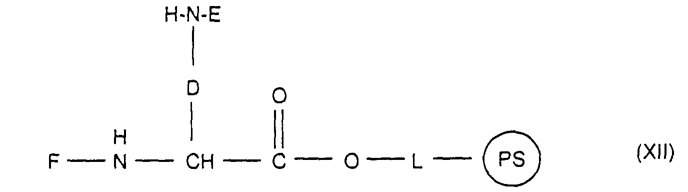 resulting, which, after selective removal of the protective group F, is reacted with a sulfonic acid derivative of the formula IV 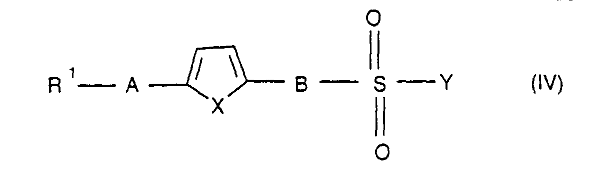 where R¹, A, B and Y have the abovementioned meanings, in the presence of a base or, if appropriate, of a dehydrating agent to give a compound of the formula XIII 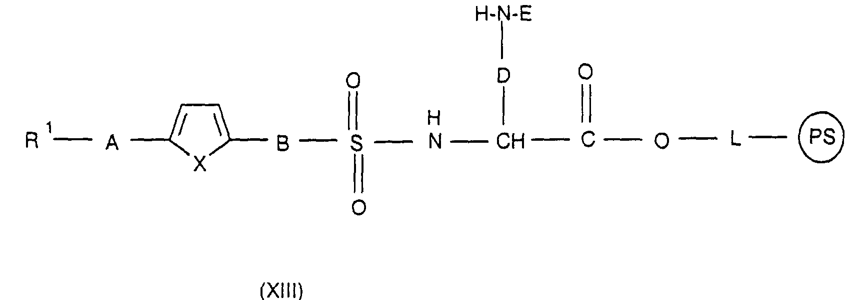 and reacting the compound of the formula XIII, after removal of the protective group E, with a carboxylic acid derivative of the formula XIV
R⁶-C(O)-Y (XIV)
in which R⁶ and Y have the abovementioned meaning, in the presence of a base or of a dehydrating agent, to give a compound of the formula XV 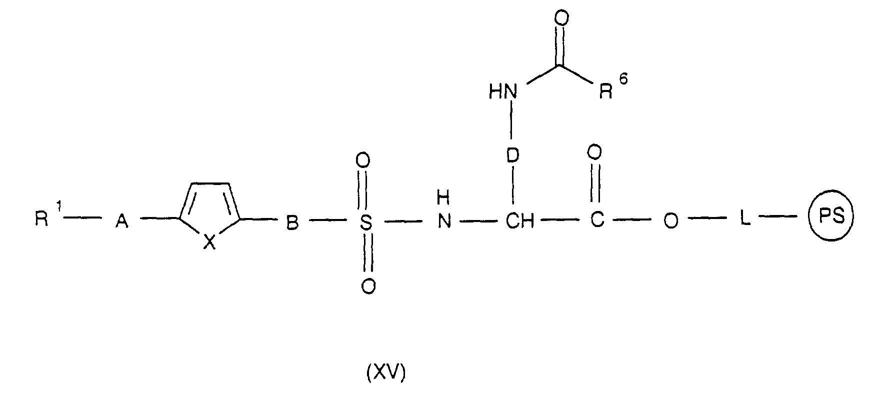 and converting this, after removal from the support material, into a compound of the formula I, 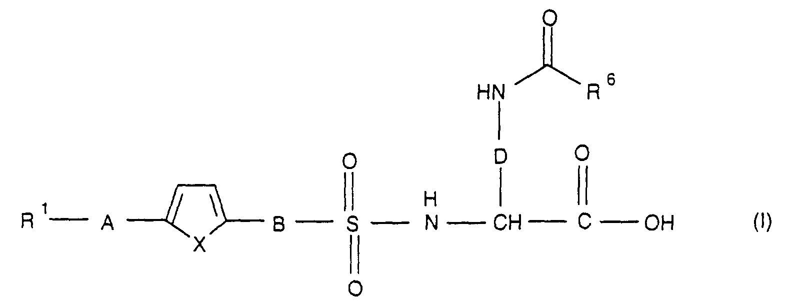 in which R¹, R⁶, A, B, D and X have the abovementioned meaning.

5. A pharmaceutical, which comprises an efficacious content of at least one compound of the formula I as claimed in one or more of claims 1 to 3, together with a pharmaceutically suitable and physiologically tolerable excipient, additive and/or other active compounds and auxiliaries.

6. The use of at least one compound of the formula I as claimed in one or more of claims 1 to 3 for the production of pharmaceuticals for the prophylaxis and therapy of disorders in the course of which an increased activity of matrix-degrading metalloproteinases is involved.

7. The use as claimed in claim 6, for the treatment of degenerative joint disorders such as osteoarthroses, spondyloses, chondrolysis after joint trauma or relatively long immobilization of the joint after meniscus or patella injuries or tears of the ligaments, disorders of the connective tissue such as collagenoses, periodontal disorders, wound healing disorders and chronic disorders of the locomotory apparatus such as inflammatory, immunologically or metabolically related acute and chronic athritides, arthropathies, myalgias and disorders of the bone metabolism, ulceration, atherosclerosis and stenoses, but also for the treatment of inflammation, carcinomatous disorders, formation of tumor metastases, cachexia, anorexia and septic shock.

8. A process for the production of a pharmaceutical which comprises bringing at least one compound of the formula I as claimed in one or more of claims 1 to 3 into a suitable administration form using a pharmaceutically suitable and physiologically tolerable excipient and, if appropriate, other suitable active compounds, additives or auxiliaries.

## Revendications

1. Composé de formule I 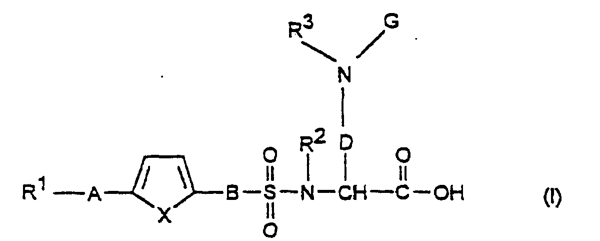 et/ou une forme stéréoisomère du composé de formule I et/ou un sel physiologiquement acceptable du composé de formule I dans lequel
R¹ représente
1. un groupe phényle,
2. phényle, qui est substitué une ou deux fois par
2.1 un groupe alkyle en C₁ à C₇, linéaire, cyclique ou ramifié,
2.2 -OH,
2.3 alkyl en C₁ à C₆-C(O)-O-,
2.4 alkyl en C₁ à C₆-O-,
2.5 alkyl en C₁ à C₆-O-alkyle en C₁ à C₄-O-,
2.6 un atome d'halogène,
2.7 -CF₃,
2.8 -CN,
2.9 -NO₂,
2.10 HO-C(O)-,
2.11 alkyl en C₁ à C₆-O-C(O)-,
2.12 méthylènedioxo,
2.13 R⁴- (R⁵) N-C (O) -,
2.14 R⁴-(R⁵)N-, ou
3. un composé hétéroaromatique choisi parmi les groupes suivants 3.1 à 3.16, qui est non substitué ou substitué comme décrit aux paragraphes 2.1 à 2.14,
3.1 pyrrole,
3.2 pyrazole,
3.3 imidazole,
3.4 triazole,
3.5 thiophène,
3.6 thiazole,
3.7 oxazole,
3.8 isoxazole,
3.9 pyridine,
3.10 pyrimidine,
3.11 indole,
3.12 benzothiophène,
3.13 benzimidazole,
3.14 benzoxazole,
3.15 benzothiazole ou
3.16 benzotriazole,
R², R⁴ et R⁵ sont identiques ou différents et représentent
1. un atome d'hydrogène,
2. un groupe alkyle en C₁ à C₆,
3. HO-C(O)-alkyle en C₁ à C₆,
4. phényl-(CH₂)ₒ-, dans lequel le phényle est non substitué ou une ou deux fois substitué comme décrit aux paragraphes 2.1 à 2.14 et o représente le nombre entier zéro, 1 ou 2, ou
5. picolyle ou
6. R⁴ et R⁵ forment ensemble avec le groupe amino situé dans le cycle, un cycle à 4 à 7 chaînons, dans lequel éventuellement l'un des atomes de carbone est remplacé par -O-, -S- ou -NH-,
R³ et G sont identiques ou différents et représentent
1. un atome d'hydrogène,
2. un groupe alkyle en C₁ à C₆, dans lequel le groupe alkyle est linéaire, ramifié ou cyclique,
3. alcényle en C₂ à C₆,
4. phényle-(CH₂)ₘ-, dans lequel le groupe phényle est non substitué ou une ou deux fois substitué comme décrit dans les paragraphes 2.1 à 2.14 et m est le nombre entier zéro, 1, 2 ou 3,
5. hétéroaryl- (CH₂)ₘ-, dans lequel le groupe hétéroaryle est défini comme aux paragraphes 3.1 à 3.16 et/ou est substitué comme décrit aux paragraphes 2.1 à 2.14 et m représente le nombre zéro, 1, 2 ou 3,
6. R⁶-C(O)-, dans lequel
R⁶ représente
6.1 un groupe alkyle en C₁ à C₆, dans lequel le groupe alkyle est non substitué ou substitué comme décrit aux paragraphes 2.1 à 2.14 ou est substitué par un groupe cycloalkyle en C₃ à C₆,
6.2 cycloalkyle en C₃ à C₆, dans lequel le groupe cycloalkyle est non substitué ou substitué comme décrit aux paragraphes 2.1 à 2.14,
6.3 alcényle en C₂ à C₆, dans lequel le groupe alcényle est non substitué ou une à trois fois substitué par
6.3.1 un groupe phényle, dans lequel le groupe phényle est non substitué ou substitué une à trois fois comme décrits aux paragraphes 2.1 à 2.14,
6.3.2 hétéroaryle, dans lequel le groupe hétéroaryle est défini comme aux paragraphes 3.1 à 3.16 et est non substitué ou une à trois fois substitué comme décrit aux paragraphes 2.1 à 2.14 ou
6.3.3 les groupes décrits aux paragraphes 2.1 à 2.14,
6.4 phényl-(CH₂)ₘ-, dans lequel le groupe phényle est non substitué ou une à trois fois substitué comme décrit aux paragraphes 2.1 à 2.14, par -O-CF₃, -SO₂-NH₂, -NH-C(O)-CF₃ ou par un groupe benzyle et éventuellement un atome d'hydrogène du groupe -(CH₂)- est substitué par le groupe -COOH et m représente le nombre entier zéro, 1, 2 ou 3,
6.5 naphtyle,
6.6 adamantyle ou
6.7 hétéroaryl-(CH₂)ₘ- dans lequel le groupe héréroaryle est défini comme dans les paragraphes 3.1 à 3.16 et/ou est substitué comme décrit aux paragraphes 2.1 à 2.14 et m représente le nombre entier zéro, 1, 2 ou 3,
7. R⁶-O-C(O)-, dans lequel R⁶ est défini comme indiqué ci-dessus,
8. R⁶-CH(NH₂)-C(O)-, dans lequel R⁶ est défini comme indiqué ci-dessus,
9. R⁸-N(R⁷)-C(O)-, dans lequel
R⁸ représente
9.1 un atome d'hydrogène
9.2 un groupe alkyle en C₁ à C₆,
9.3 phényl - (CH₂)ₘ-, dans lequel le groupe phényle est non substitué ou une à trois fois substitué comme décrit aux paragraphes 2.1 à 2.14 et m représente le nombre entier zéro, 1, 2 ou 3, ou
9.4 hétéroaryl- (CH₂)ₘ- dans lequel le groupe héréroaryle est défini comme dans les paragraphes 3.1 à 3.16 et/ou est substitué comme décrit aux paragraphes 2.1 à 2.14 et m représente le nombre entier zéro, 1, 2 ou 3, et dans lequel
R⁷ représente un atome d'hydrogène ou un groupe alkyle en C₁ à C₆ ou dans lequel
R⁷ et R⁸ forment ensemble avec l'atome d'azote auquel ils sont liés un cycle à 4 à 7 chaînons et le cycle est non substitué ou un atome de carbone dans le cycle est remplacé par -O-, -S- ou -NH-,
10. R⁶-SO₂-, dans lequel R⁶ est défini comme indiqué ci-dessus,
11. R⁶-SO₂-N(R⁷)-C(O)-, dans lequel R⁶ et R⁷ sont définis comme indiqué ci-dessus,
12. R⁶-NH-C(=NR⁷)-, dans lequel R⁶ et R⁷ sont définis comme indiqué ci-dessus ou
12.1 alkyl en C₁ à C₆-C(O)-,
12.2 -NO₂ ou
12.3 -SO₂-(CH₂)ₘ-phényle, dans lequel le groupe phényle est non substitué ou une ou deux fois substitué comme décrit aux paragraphes 2.1 à 2.14 et m représente le nombre zéro, 1, 2 ou 3,
13. 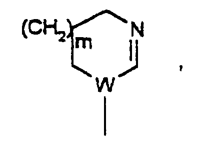 dans lequel le nombre entier m représente zéro, 1, 2 ou 3 et W représente un atome d'azote, d'oxygène ou de soufre, ou
R³ et G forment ensemble avec l'atome d'azote auquel ils sont liés un cycle de formule partielle IIa à IIp 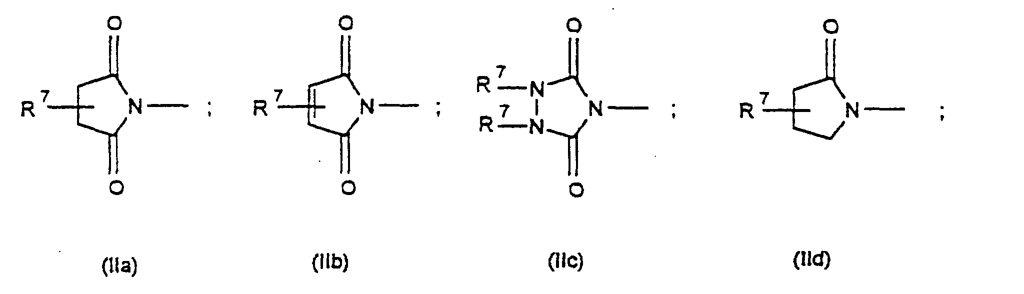 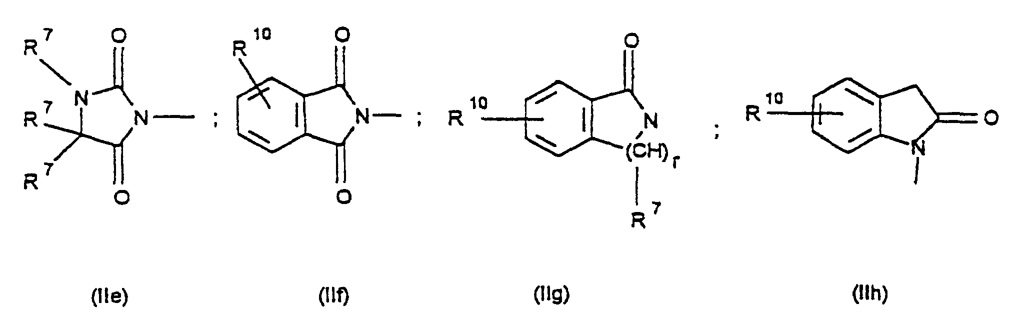 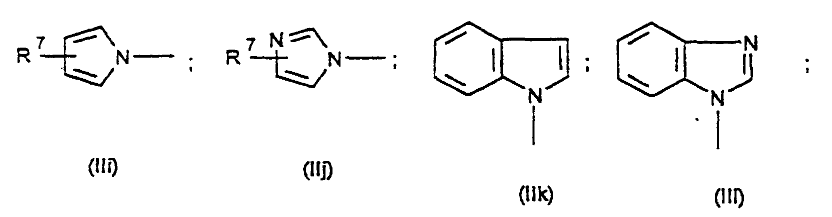 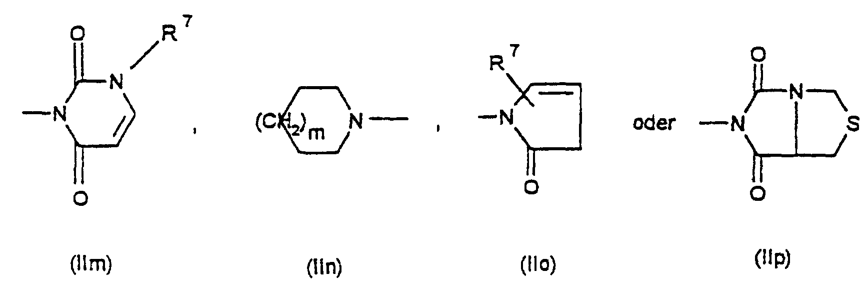 dans lesquelles r représente le nombre 1 ou 2, R¹⁰ représente un groupe comme décrit aux paragraphes 2.1 à 2.14, et R⁷ et m ont la signification indiquée ci-dessus et éventuellement dans la formule partielle IIg un atome de carbone dans le cycle est remplacé par un atome d'oxygène, de soufre, SO₂ ou est non substitué ou est remplacé par un atome d'azote substitué par R²,
A représente
a) une liaison covalente,
b) -O-,
c) -CH=CH- ou
d) -C≡C-,
B représente
a) -(CH₂)ₘ-, dans lequel m a la signification susmentionnée,
b) -O-(CH₂)_{q}, dans lequel q représente le nombre entier 1, 2, 3, 4 ou 5 ou
c) -CH=CH-,
D représente -(CH₂)ₛ-, dans lequel s représente le nombre entier 1, 2, 3, 4, 5 ou 6 et éventuellement un des atomes C de chaîne est remplacé par un groupe -NH- éventuellement substitué, un atome -O- ou-S-, et
X représente -CH=CH-, un atome d'oxygène ou un atome de soufre.

2. Composé de formule I selon la revendication 1, **caractérisé en ce que**,
R¹ représente
1. un groupe phényle ou
2. phényle, qui est substitué une ou deux fois par
2.1 un atome d'halogène, en particulier de chlore ou de fluor ou
2.2 R⁴-(R⁵)N-, dans lequel R⁴ et R⁵ sont identiques ou différents et représentent
2.2.1 un groupe alkyle en C₁ à C₃ ou
2.2.2 R⁴ et R⁵ forment ensemble avec le groupe amino situé dans le cycle, un cycle à 5 ou 6 chaînons, dans lequel éventuellement un des atomes de carbone est remplacé par -O- ou -NH-,
R² représente un atome d'hydrogène,
G et R³ sont différents, et
G représente un atome d'hydrogène ou un groupe alkyle en C₁ à C₄ et
R³ représente
1. un groupe phényl-(CH₂)ₘ, dans lequel le groupe phényle est non substitué ou une ou deux fois substitué comme décrit dans les paragraphes 2.1 à 2.14 et m est le nombre entier 1,
2. hétéroaryl-(CH₂)ₙ, dans lequel le groupe hétéroaryle est défini comme en 3.10 et est non substitué ou est substitué comme décrit dans les paragraphes 2.1 à 2.14 et n représente zéro,
3. R⁶-C(O)-, dans lequel
R⁶ représente
3.1 un groupe alkyle en C₁ à C₆, dans lequel le groupe alkyle est linéaire, ramifié ou cyclique,
3.2 phényl-(CH₂)ᵣ- dans lequel le phényle est non substitué ou une ou deux fois substitué comme décrit dans les paragraphes 2.1 à 2.14 et éventuellement un atome d'hydrogène du groupe -(CH₂)- est remplacé par le groupe -COOH et r représente zéro, 1, 2 ou 3, ou
3.3 hétéroaryl - (CH₂)ₒ-, dans lequel hétéroaryle est défini comme dans les paragraphes 3.1 à 3.15 et est non substitué ou substitué comme décrit dans les paragraphes 2.1 à 2.14 et o est zéro, 1, 2 ou 3, ou
4. représente R⁸-N(R⁷)-C(O)-, dans lequel
R⁸ et R⁷ forment avec l'atome d'azote auquel ils sont liés un cycle à 5 ou 6 chaînons et le cycle est non substitué ou un atome C de cycle est remplacé par un atome d'oxygène, ou
R³ et G forment ensemble avec l'atome d'azote auquel ils sont liés un cycle de formule partielle IIg, dans laquelle r est 1,
A représente une liaison covalente,
B représente -(CH₂)ₚ- et p représente zéro,
D représente -(CH₂)_{q}- et q représente un nombre entier 2, 3 ou 4, et
X représente -CH=CH-.

3. Composé de formule X 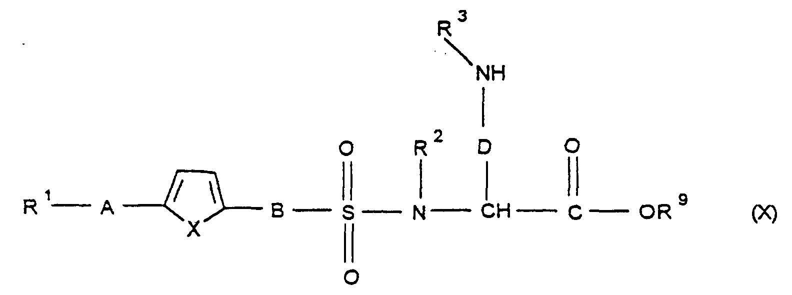 et/ou une forme stéréoisomère du composé de formule X et/ou un sel physiologiquement acceptable du composé de formule X, dans lequel R¹, A, X, B, R², R³ et D ont la signification indiquée dans le composé de formule I selon la revendication 1 et R⁹ représente un atome d'hydrogène, un groupe alkyle en C₁ à C₆, phényle, succinimidyle, benzotriazolyle ou benzyle.

4. Procédé pour la préparation du composé de formule I selon une ou plusieurs des revendications 1 à 3, **caractérisé en ce qu'**on met à réagir
a) un acide diaminocarboxylique de formule III 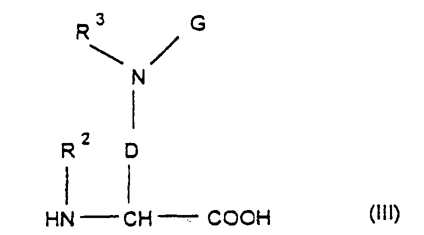 dans laquelle R², R³, D et G sont définis comme à la formule I, avec un dérivé d'acide sulfonique de formule IV 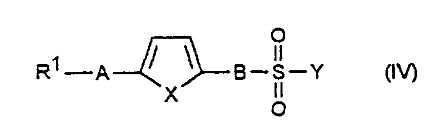 dans laquelle R¹, A et B sont comme définis à la formule I et Y représente un atome d'halogène, un groupe imidazolyle ou -OR⁹, dans lequel R⁹ représente un atome d'hydrogène, un groupe alkyle en C₁ à C₆, phényle, succinimyle, benzotriazolyle ou benzyle, éventuellement substitué,
en présence d'une base ou éventuellement d'un agent déshydratant pour obtenir un composé de formule I, ou
b) un ester d'acide diaminocarboxylique de formule V 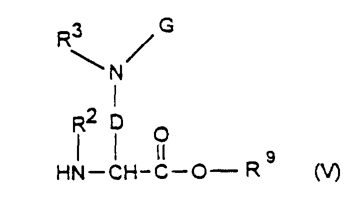 dans laquelle R², R³, D, G et R⁹ ont la signification susmentionnée,
avec un dérivé d'acide sulfonique de formule IV dans les conditions susmentionnées pour obtenir un composé de formule VI 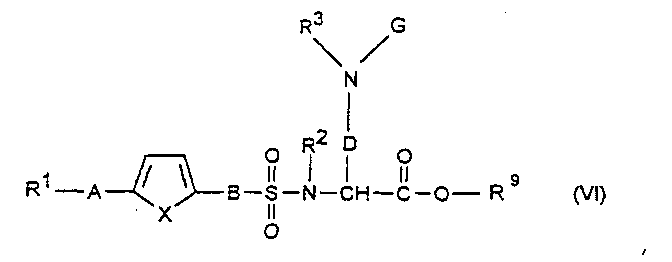 et on transforme le composé de formule VI par clivage du groupe R⁹, de préférence en présence d'une base ou d'un acide en un composé de formule I, ou
c) on met à réagir les acides diaminocarboxyliques protégés de formule VII, 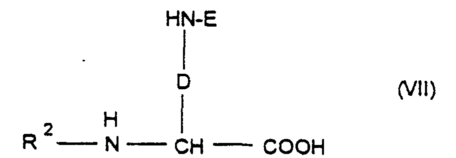 dans laquelle R² et D ont les significations susmentionnées et E représente un groupe de protection de la fonction amino, avec un dérivé d'acide sulfonique de formule IV pour obtenir un composé de formule VIII 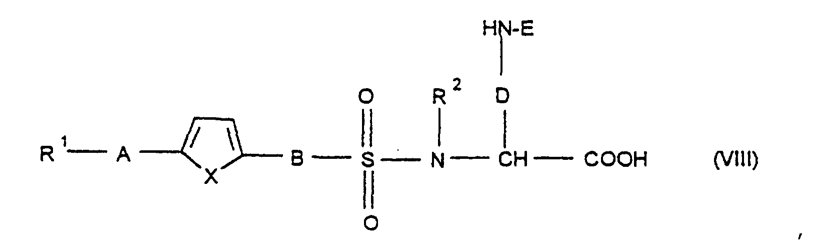 on transforme ensuite le composé de formule VIII par clivage du groupe de protection E au moyen de réactifs de coupure appropriés en un composé de formule I, 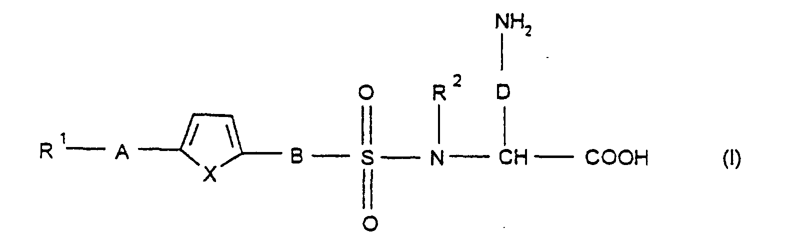 dans laquelle R¹, R², A, B, D et X ont la signification susmentionnée et R³ et G représentent un atome d'hydrogène, et
on met à réagir ce composé de formule I éventuellement au moyen de R³-Y, dans lequel R³ et Y ont les significations susmentionnées, en présence d'une base pour obtenir un composé de formule I, 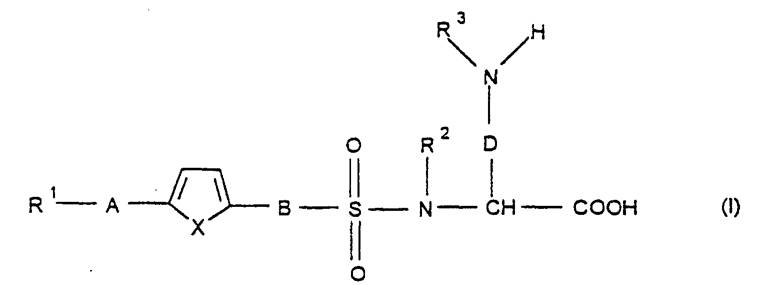 dans laquelle R¹, R², R³, A, B et X ont les significations susmentionnées et G représente un atome d'hydrogène, ou
d) dans le cas où l'on transforme comme composés de départ des esters de diaminoacide protégés de formule IX 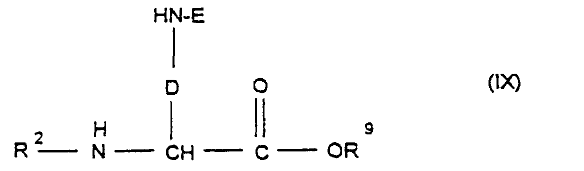 dans laquelle R², R⁹, D et E ont la signification susmentionnée, de la même manière que décrit dans la variante de procédé c) pour obtenir les esters de formule X, 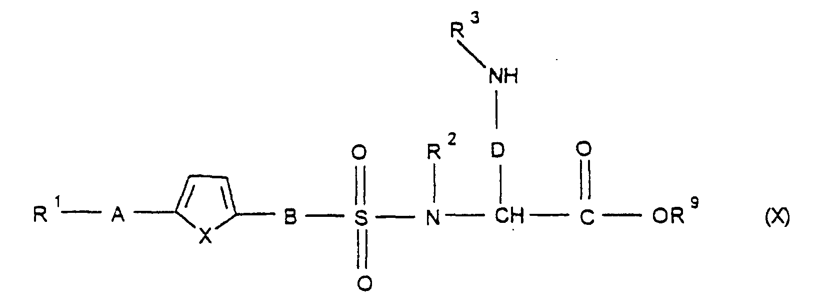 qui sont éventuellement transformés en composés correspondants de formule I selon la variante de procédé b), ou
e) on couple un acide diaminocarboxylique de formule XI, 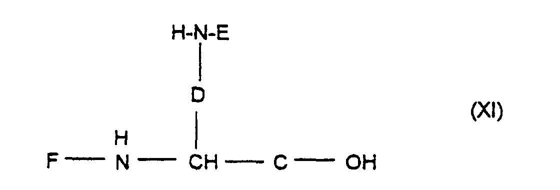
dans laquelle D est défini comme à la formule I et E et F représentent des groupes de protection N-amino différents entre eux, avec leur groupe carboxyle via une chaîne intermédiaire à une résine polymère de formule générale PS, ce par quoi se forme un composé de formule XII 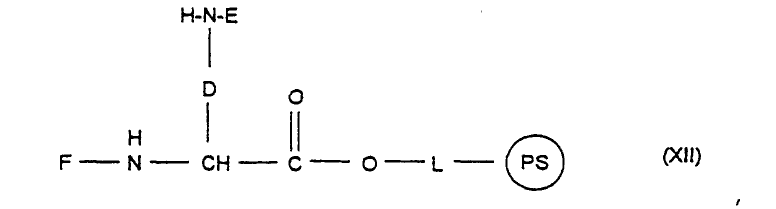 qui est mis à réagir après clivage sélectif du groupe de protection F avec un dérivé d'acide sulfonique de formule IV 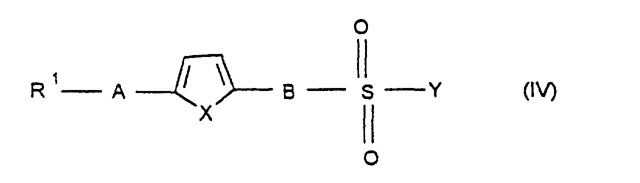 dans laquelle R¹, A, B et Y ont les significations indiquées ci-dessus, en présence d'une base ou éventuellement d'un agent déshydratant pour obtenir un composé de formule XIII 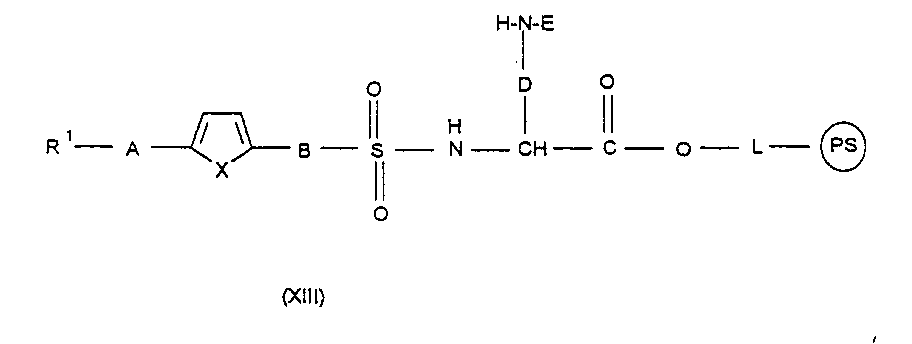 et on met à réagir le composé de formule XIII après clivage du groupe de protection E avec un dérivé d'acide carboxylique de formule XIV
R⁶-C(O)-Y (XIV)
dans laquelle R⁸ et Y ont la signification susmentionnée, en présence d'une base ou d'un agent déshydratant pour obtenir un composé de formule XV 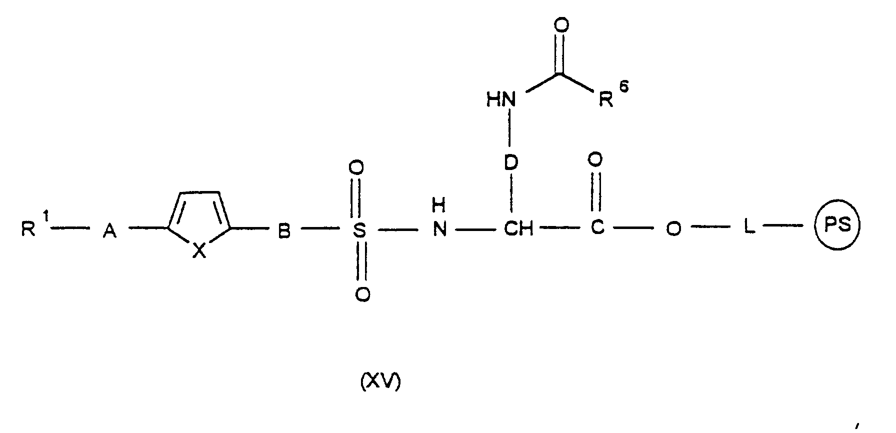 et on transforme celui-ci après clivage du matériau support en un composé de formule I, 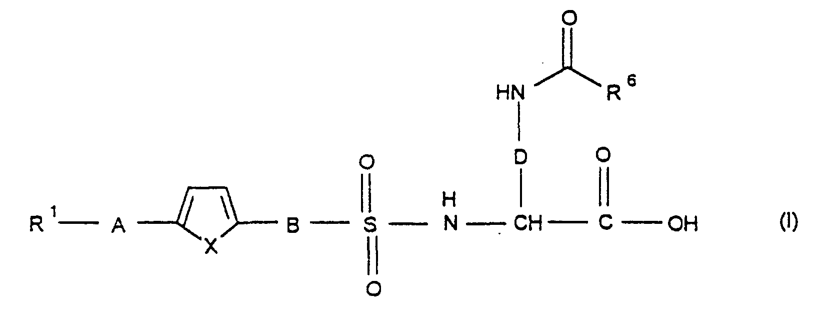 dans laquelle R¹, R⁶, A, B, D et X ont la signification susmentionnée.

5. Médicament, **caractérisé par** une concentration active d'au moins un composé de formule I selon une ou plusieurs des revendications 1 à 3, avec un véhicule, un additif et/ou d'autres substances actives ou auxiliaires physiologiquement acceptables et pharmaceutiquement appropriés.

6. Utilisation d'au moins un composé de formule I selon une ou plusieurs des revendications 1 à 3, pour la préparation de médicaments pour la prophylaxie et la thérapie de maladies, dont l'évolution est due à un renforcement de l'activité des métalloprotéinases décomposant la matrice.

7. Utilisation selon la revendication 6, pour le traitement de maladies dégénératives des articulations comme les ostéoarthroses, les spondyloses, un dépérissement du cartilage après traumatisme des articulations ou maintien au repos de longue durée des articulations après des blessures du ménisque ou de la rotule ou déchirures des ligaments, maladies du tissu conjonctif comme les collagénoses, les maladies périodontales, les troubles de cicatrisation des blessures et les maladies chroniques de l'appareil moteur comme les arthrites inflammatoires, immunologiques ou métaboliques aiguës et chroniques, les arthropathies, les myalgies et les troubles du métabolisme osseux, de l'ulcération, de l'athérosclérose et des sténoses, mais aussi pour le traitement d'inflammations, de maladies cancéreuses, de formation de métastases tumorales, de cachexie, d'anorexie et de choc septique.

8. Procédé pour la préparation d'un médicament, **caractérisé en ce qu'**on met sous une forme d'administration appropriée au moins un composé de formule I selon une ou plusieurs des revendications 1 à 3 avec un véhicule physiologiquement acceptable et pharmaceutiquement approprié et éventuellement d'autres substances actives, additifs ou substances auxiliaires.
